(19) Europäisches Patentamt
European Patent Office
Office européen des brevets

(11) EP 2 513 069 B1

(12) **EUROPEAN PATENT SPECIFICATION**

(45) Date of publication and mention of the grant of the patent:
**15.10.2014 Bulletin 2014/42**

(21) Application number: **10812837.2**

(22) Date of filing: **15.12.2010**

(51) Int Cl.:
***C07D 265/32*** *(2006.01)* ***C07D 413/12*** *(2006.01)*
***C07D 417/12*** *(2006.01)*

(86) International application number:
**PCT/BE2010/000083**

(87) International publication number:
**WO 2011/072345 (23.06.2011 Gazette 2011/25)**

(54) **ANTIFUNGAL COMPOUNDS**

ANTIMYKOTISCHE ZUSAMMENSETZUNGEN

COMPOSÉS ANTIFONGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2009 GB 0921871**

(43) Date of publication of application:
**24.10.2012 Bulletin 2012/43**

(73) Proprietor: **Katholieke Universiteit Leuven**
**3000 Leuven (BE)**

(72) Inventors:
- **BARDIOT, Dorothée**
  **B-3000 Leuven (BE)**
- **CAMMUE, Bruno**
  **B-1652 Alsemberg (BE)**
- **CHALTIN, Patrick**
  **B-1370 Zetrud-Lumay (BE)**
- **MARCHAND, Arnaud**
  **B-3360 Korbeek-Lo (BE)**
- **THEVISSEN, Karin**
  **3360 Bierbeek (BE)**

(74) Representative: **Bounaga, Sakina**
**De Clercq & Partners cvba**
**Edgard Gevaertdreef 10a**
**9830 Sint-Martens-Latem (BE)**

(56) References cited:
**WO-A1-2007/011292**
**WO-A1-2010/003197**
**US-A- 4 044 131**
**US-A- 4 879 291**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 513 069 B1

**Description**

## FIELD OF THE INVENTION

The present invention relates to a series of novel compounds which have been shown to possess antifungal activity, in particular against Candida, Aspergillus and Fusarium species. The invention therefore relates to the new compounds, methods for their preparation, pharmaceutical compositions comprising them and to the compounds for use as a medicament, more in particular as an antifungal medicament.

## BACKGROUND OF THE INVENTION

Occurring worldwide, most fungi are largely invisible to the naked eye, living for the most part in soil, dead matter, and as symbionts of plants, animals, or other fungi. However, several species of the fungi are significant pathogens of humans and other animals or crops. The most commonly known human pathogens are part of the Candida, Aspergillus and/or Fusarium species.
*Candida albicans* is among the gut flora, the many organisms which live in the human mouth and gastrointestinal tract. Under normal circumstances, *C. albicans* lives in 80% of the human population with no harmful effects, although overgrowth results in candidiasis. Candida albicans is a causal agent of opportunistic oral and genital infections in humans. Systemic fungal infections have emerged as important causes of morbidity and mortality in immunocompromised patients (e.g., AIDS, cancer chemotherapy, organ or bone marrow transplantation). In addition, hospital-related infections in patients not previously considered at risk (e.g. patients in an intensive care unit) have become a cause of major health concern.
Until recently, *C. glabrata* was thought to be a primarily non-pathogenic organism. However, with the ever increasing population of immunocompromised individuals, trends have shown *C. glabrata* to be a highly opportunistic pathogen of the urogenital tract, and of the bloodstream. It is especially prevalent in HIV positive people, and the elderly. A major phenotype and potential virulence factor that *C. glabrata* possesses is low-level intrinsic resistance to the azole drugs, which are the most commonly prescribed antifungal (antimycotic) drugs. *Candida krusei* falls under the same umbrella as C. glabrata in the meaning that this fungus has recently emerged as a human pathogen responsible for mortality in immunocompromised patients and is not sensitive to the azole antimycotics.
*Aspergillus* is a genus of around 200 moulds found throughout much of nature worldwide. Some *Aspergillus* species cause serious disease in humans and animals, and can be pathogenic. The most common species causing invasive disease are *Aspergillus fumigatus* and *Aspergillus flavus.* Aspergillus flavus produces aflatoxin which is both a toxin and a carcinogen, and which can potentially contaminate foods such as nuts. The most common species causing allergic disease are *Aspergillus fumigatus* and *Aspergillus clavatus.* Other species are important as agricultural pathogens. Aspergillosis is the group of diseases caused by *Aspergillus* and includes paranasal sinus infections (fever, cough, chest pain or breathlessness), allergic bronchopulmonary aspergillosis or ABPA, acute invasive aspergillosis and disseminated invasive aspergillosis.
Today, there are 4 classes of established antifungal drugs on the market: (1) the polyenes (e.g. amphotericin B, nystatin, natamycin), (2) the azoles (e.g. fluconazole, itraconazole, voriconazole), (3) allylamines (e.g. terbinafine), and (4) the newly introduced echinocandins (e.g. caspofungin). Of these classes, only the polyenes, azoles and echinocandins are used to treat systemic fungal infections, not the allylamines. All the currently marketed antifungal drugs have major drawbacks, including no broad-spectrum activity, no per oral absorption, side-effects, low antifungal activity, no fungicidal activity, drug-drug interactions and high costs.
Therefore, there is still a stringent need in the art for potent antifungals for topical or systemic infections, especially with a broad spectrum activity against multiple species, per oral absorption, lower amount of side-effects, fungicidal activity, no drug-drug interactions or lower costs or a combination of these. Therefore a goal of the present invention is to satisfy this urgent need by identifying efficient and non-harmful pharmaceutically active ingredients and combination of ingredients for the treatment of (systemic) fungal infections in animals and in humans.
The prior art describes different anti-fungal compound classes, including but not limited to the polyenes, the azoles, allylamines and echinocandins (e.g. caspofungin), but no antifungal compound class relates to the compounds of the invention.

## SUMMARY OF THE INVENTION

In the present invention, new antifungal compounds are provided. The compounds have a 6-substituted morpholin-2-one structure and it has been shown in the present invention that they possess antifungal (fungicidal) activity, more specifically against Candida, Aspergillus and Fusarium species. The present invention demonstrates that the compounds inhibit the replication of fungi. Therefore, these compounds constitute a new potent class of antifungal agents

that can be used in the treatment and prevention of fungal infections in animals, mammals and humans, more specifically for the treatment and prevention of (topical or systemic) infections with Candida, Aspergillus and/or Fusarium species.

The present invention provides novel compounds which have fungistatic or fungicidal properties. The invention also provides methods for preparation of all such compounds and provides pharmaceutical compositions comprising the compounds. The invention further relates to the novel compounds for use as a medicament and for the prevention and/or treatment of fungal infections in subjects (including animals, mammals and humans). The invention also relates to the use of the compounds in or for the manufacture of a medicament for the prevention or treatment of subjects suffering from a fungal infection, more in particular for treatment of subjects infected with Candida species (e.g. C. albicans, C. glabrata, C. krusei), Aspergillus species (e.g. A. flavus, A. fumigatus, A. clavatus), Fusarium species (e.g. F. oxysporum, F. culmorum) or other fungi. The invention also provides methods of treatment or prevention of a fungal infection in a subject, including animals, mammals and humans.

US 4,879,291 and US 4,044,130 disclose morpholino-derivatives for use in treating fungal infections

One aspect of the present invention is the provision of novel compounds which are 6-substituted 2-(2-oxo-morpholin-3-yl)-acetamides, in an embodiment of this invention having a structure according to the formula (A'):

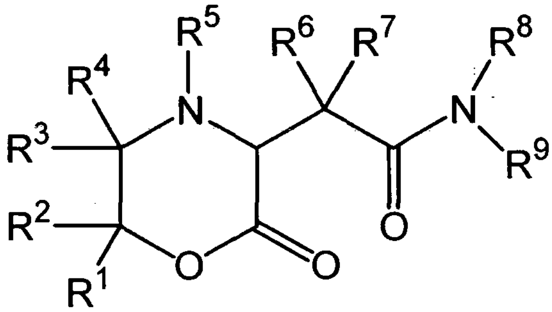

(A')

wherein,

- each $R^1$ and $R^2$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; and arylheteroalkynyl;
  provided that at least one of $R^1$ and $R^2$ is not hydrogen;
- each $R^3$ and $R^4$ is independently selected from hydrogen; alkyl; alkenyl; alkynyl; heteroalkyl; heteroalkenyl; and heteroalkynyl;
- $R^5$ is independently selected from hydrogen; alkyl; alkenyl; alkynyl; heteroalkyl; heteroalkenyl; heteroalkynyl; aryl; arylalkyl; arylalkenyl; arylalkynyl; -C(O)$R^{10}$; and-$S(O)_2R^{10}$;
  wherein each of said alkyl, alkenyl, alkynyl, heteroalkyl, heteroalkenyl, heteroalkynyl, aryl, arylalkyl, arylalkenyl, and arylalkynyl can be unsubstituted or substituted;
- each $R^6$ and $R^7$ is independently selected from hydrogen; alkyl; alkenyl; alkynyl; heteroalkyl; heteroalkenyl; and heteroalkynyl;
- each $R^8$ and $R^9$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocylce-alkynyl; heterocycle-heteroalkyl; heterocycle-heteroalkenyl; and heterocycle-heteroalkynyl; and wherein $R^8$ and $R^9$ can be taken together in order to form a (5-, 6-, or 7-membered) heterocycle;
  provided that at least one of $R^8$ and $R^9$ is not hydrogen; and wherein each of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocylce-alkynyl, heterocycle-heteroalkyl, heterocycle-heteroalkenyl, and heterocycle-heteroalkynyl, can be unsubstituted or substituted;
- each $R^{10}$ is independently selected from hydrogen; alkyl; alkenyl; alkynyl; heteroalkyl; heteroalkenyl; heteroalkynyl; aryl; arylalkyl; arylalkenyl; and arylalkynyl;
  wherein each of said alkyl, alkenyl, alkynyl, heteroalkyl, heteroalkenyl, heteroalkynyl, aryl, arylalkyl, arylalkenyl, and arylalkynyl can be unsubstituted or substituted.

and stereo-isomers, tautomers, solvates, or pharmaceutically acceptable salts thereof.

In a particular embodiment, the compounds have a structure according to formula (A),

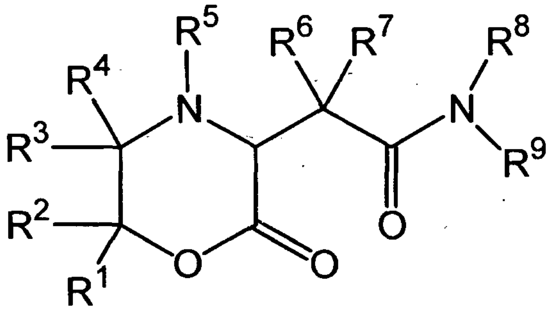

(A)

wherein,

- each $R^1$ and $R^2$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; and arylheteroalkynyl; provided that at least one of $R^1$ and $R^2$ is not hydrogen;
- each $R^3$ and $R^4$ is independently selected from hydrogen; alkyl; alkenyl; alkynyl; heteroalkyl; heteroalkenyl; and heteroalkynyl;
- $R^5$ is independently selected from hydrogen; alkyl; alkenyl; alkynyl; heteroalkyl; heteroalkenyl; heteroalkynyl; aryl; arylalkyl; arylalkenyl; arylalkynyl; $-C(O)R^{10}$; and $-S(O)_2R^{10}$;
wherein each of said alkyl, alkenyl, alkynyl, heteroalkyl, heteroalkenyl, heteroalkynyl, aryl, arylalkyl, arylalkenyl, and arylalkynyl can be unsubstituted or substituted with one or more substituents each independently selected from alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH, or $-NH_2$;
- each $R^6$ and $R^7$ is independently selected from hydrogen; alkyl; alkenyl; alkynyl; heteroalkyl; heteroalkenyl; and heteroalkynyl;
- each $R^8$ and $R^9$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocylce-alkynyl; heterocycle-heteroalkyl; heterocycle-heteroalkenyl; and heterocycle-heteroalkynyl; and wherein $R^8$ and $R^9$ can be taken together in order to form a (5-, 6-, or 7-membered) heterocycle;
provided that at least one of $R^8$ and $R^9$ is not hydrogen; and
wherein each of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocylce-alkynyl, heterocycle-heteroalkyl, heterocycle-heteroalkenyl, and heterocycle-heteroalkynyl, can be unsubstituted or substituted with one or more $R^{11}$;
- each $R^{10}$ is independently selected from hydrogen; alkyl; alkenyl; alkynyl; heteroalkyl; heteroalkenyl; heteroalkynyl; aryl; arylalkyl; arylalkenyl; and arylalkynyl;
wherein each of said alkyl, alkenyl, alkynyl, heteroalkyl, heteroalkenyl, heteroalkynyl, aryl, arylalkyl, arylalkenyl, and arylalkynyl can be unsubstituted or substituted with one or more substituents each independently selected from alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH, or $-NH_2$;
- each $R^{11}$ is independently selected from the group consisting of halogen; hydroxyl; sulfhydryl; $-OZ^2$; =O; $-SZ^2$; =S; $-S(O)Z^2$; $-S(O)_2Z^3$; $-S(O)_2NZ^4Z^5$; trifluoromethyl; nitro; $-NZ^4Z^5$; $-NZ^4S(O)_2Z^2$; $-NZ^4C(O)Z^2$; $-NZ^4C(O)NZ^4Z^5$; cyano; $-C(O)Z^3$; $-C(O)NZ^4Z^5$; -C(O)H; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl; or heterocycle-heteroalkynyl; and wherein said alkyl, cyclic alkyl, alkenyl, cyclic alkenyl, alkynyl, cyclic alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more substituents selected from the group of alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH, or $-NH_2$;
- each $Z^2$ is independently selected from alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl; heterocycle-heteroalkenyl; or heterocycle-heteroalkynyl;
wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with alkyl, alkenyl, alkynyl, heteroalkyl, heteroalkenyl, heteroalkynyl, hydroxyl, =O, halogen, -SH, =S, trifluoromethyl,-$OCF_3$, cyano, nitro, -C(O)OH, or -$NH_2$;

- each $Z^3$ is independently selected from hydroxyl; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl; heterocycle-heteroalkenyl; or heterocycle-heteroalkynyl;
wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more selected from the group of alkyl, alkenyl, alkynyl, heteroalkyl, heteroalkenyl, heteroalkynyl, hydroxyl, =O, halogen, -SH, =S, trifluoromethyl, -$OCF_3$, cyano, nitro, -C(O)OH, or -$NH_2$;
- each $Z^4$ and $Z^5$ is independently selected from hydrogen; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl; heterocycle-heteroalkenyl; or heterocycle-heteroalkynyl;
wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more selected from the group of alkyl, alkenyl, alkynyl, heteroalkyl, heteroalkenyl, heteroalkynyl, hydroxyl, =O, halogen, -SH, =S, trifluoromethyl, -$OCF_3$, cyano, nitro, -C(O)OH, or -$NH_2$; and wherein $Z^4$ and $Z^5$ can be taken together in order to form a (5-, 6-, or 7-membered) heterocycle which can be unsubstituted or substituted with alkyl, alkenyl, alkynyl, heteroalkyl, heteroalkenyl, heteroalkynyl, hydroxyl, =O, halogen, -SH, =S, trifluoromethyl, -$OCF_3$, cyano, nitro, -C(O)OH, or -$NH_2$;

and stereo-isomers, tautomers, solvates, or pharmaceutically acceptable salts thereof.

In another particular embodiment, the compounds of the invention have a structure according to formula (A') or (A) wherein,

- each $R^1$ and $R^2$ is independently selected from hydrogen; alkyl; and aryl; provided that at least one of $R^1$ and $R^2$ is not hydrogen;
- each $R^3$ and $R^4$ is independently selected from hydrogen; and alkyl;
- $R^5$ is independently selected from hydrogen; alkyl; aryl; arylalkyl; -C(O)$R^{10}$; and - $S(O)_2R^{10}$;
wherein each of said alkyl, aryl, and arylalkyl, can be unsubstituted or substituted with one or more halogens;
- each $R^6$ and $R^7$ is independently selected from hydrogen; and alkyl;
- each $R^8$ and $R^9$ is independently selected from hydrogen; alkyl; aryl; arylalkyl; heterocycle; and heterocycle-alkyl; and wherein $R^8$ and $R^9$ can be taken together in order to form a (5-, 6-, or 7-membered) heterocycle;
provided that at least one of $R^8$ and $R^9$ is not hydrogen; and
wherein each of said alkyl, aryl, arylalkyl, heterocycle, and heterocycle-alkyl, can be unsubstituted or substituted with one or more $R^{11}$;
- each $R^{10}$ is independently selected from alkyl;
- each $R^{11}$ is independently selected from the group consisting of halogen; trifluoromethyl; cyano; alkyl; heteroalkyl; aryl; arylalkyl; aryl-heteroalkyl (including -0-aryl); heterocylce; heterocycle-alkyl; heterocycle-heteroalkyl.

and stereo-isomers tautomers solvates, or pharmaceutically acceptable salts thereof.

In a particular embodiment, each of $R^1$ and $R^2$ is selected from hydrogen, alkyl and aryl. More in particular, each of $R^1$ and $R^2$ is alkyl, more in particular is $C_1$-$C_6$ alkyl, yet more particularly is methyl.
In another embodiment, each of $R^3$ and $R^4$ is selected from alkyl and hydrogen, yet more particularly is selected from $C_1$-$C_6$ alkyl and hydrogen. In a more particular embodiment, each of $R^3$ and $R^4$ is hydrogen.
In yet another particular embodiment, $R^5$ is alkyl, more preferable is $C_1$-$C_6$ alkyl, yet more preferable is ethyl.
In another particular embodiment, each of $R^6$ and $R^7$ is selected from alkyl and hydrogen, yet more particucularly is selected from $C_1$-$C_6$ alkyl and hydrogen. In a more particular embodiment, each of $R^6$ and $R^7$ is hydrogen.
In another particular embodiment, one of $R^8$ and $R^9$ is hydrogen and the other of $R^8$ and $R^9$ is selected from unsubstituted or substituted (particularly with one or more $R^{11}$) aryl, arylalkyl, arylheteroalkyl, heterocycle, heterocycle-alkyl or heterocycle-heteroalkyl. Particularly, said substituted aryl, arylalkyl, arylheteroalkyl, heterocycle, heterocycle-alkyl or heterocycle-heteroalkyl is selected from aryl, arylalkyl, heterocycle or heterocycle-alkyl substituted independently with one or more halogen, hydroxyl, thiol, $CF_3$, nitro, cyano, alkyl, heteroalkyl, alkenyl, heteroalkenyl, alkynyl and/or heteroalkynyl. Chemical moieties substituted with one or more $R^{11}$ can be substituted with 1, 2, 3, 4 or 5 independently selected $R^{11}$.

In another particular embodiment, the present invention relates to compounds with a structure according to formula (B), (C), (D) or (E),

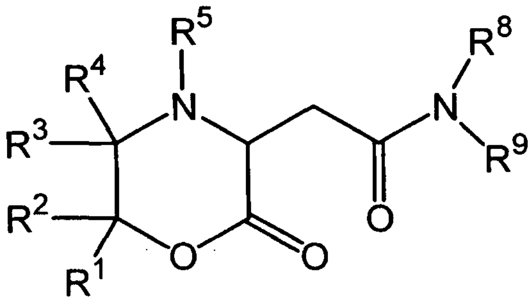

(B)

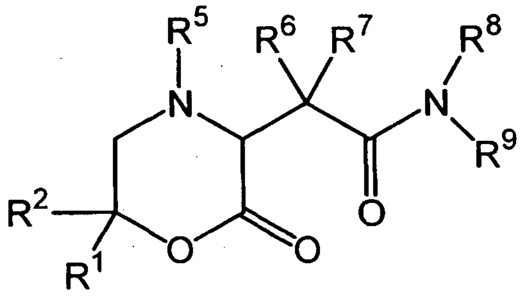

(C)

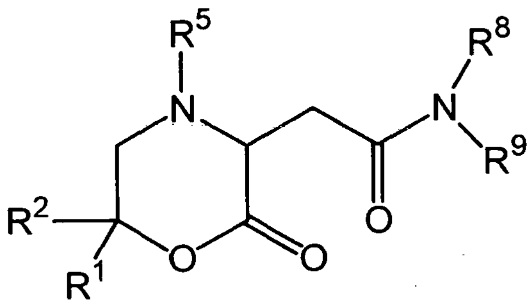

(D)

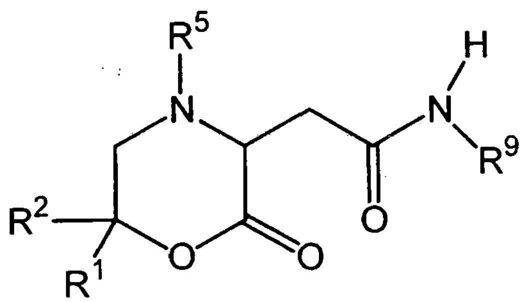

(E)

wherein each of $R^1$, $R^2$, $R^3$, $R^4$, $R^5$, $R^6$, $R^7$, $R^8$ and $R^9$ are as in formula (A') or (A) and the embodiments thereof.

In another particular embodiment, the present invention relates to compounds with a structure according to formula (Fa), (Fb), (Fc) or (Fd),

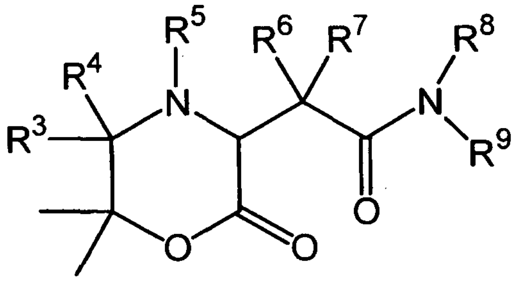

(Fa)

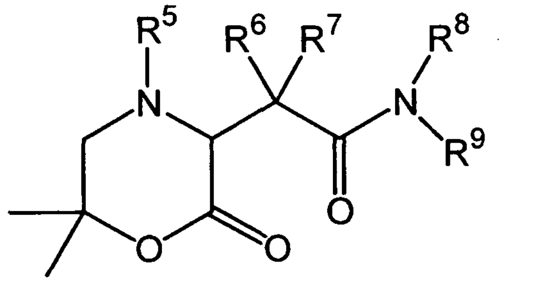

(Fb)

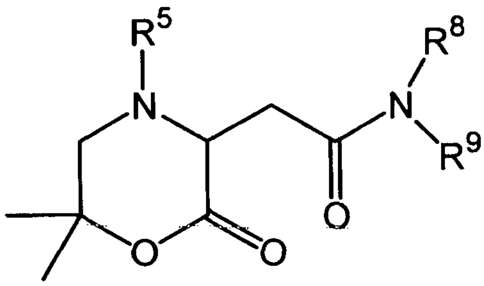

(Fc)

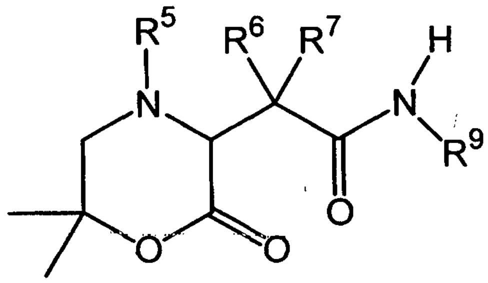

(Fd)

wherein each of $R^3$, $R^4$, $R^5$, $R^6$, $R^7$, $R^8$ and $R^9$ are as in formula (A') or (A) and the embodiments thereof.

In a particular embodiment, the compounds of the present invention are selected from the list of compounds in table 1.

Yet in a more particular embodiment, the compounds are selected from the list of:

2-((4-ethyl-6,6-dimethyl-2-oxomorpholin-3-yl)-N-(1-methyl-1H-indol-5-yl)acetamide;
N-(4-chlorobenzyl)-2-(4-ethyl-6,6-dimethyl-2-oxomorpholin-3-yl)acetamide;
N-(2,3-dihydro-1H-inden-1-yl)-2-(4-ethyl-6,6-dimethyl-2-oxomorpholin-3-yl)acetamide;
N-(benzo[d]thiazol-5-yl)-2-(4-ethyl-6,6-dimethyl-2-oxomorpholin-3-yl)acetamide;
2-((4-ethyl-6,6-dimethyl-2-oxomorpholin-3-yl)-N-(1,2,3,4-tetrahydronaphthalen-1-yl)acetamide;
N-benzyl-2-(4-ethyl-6,6-dimethyl-2-oxomorpholin-3-yl)acetamide;
2-((4-ethyl-6,6-dimethyl-2-oxomorpholin-3-yl)-N-(pyridin-3-yl)acetamide;
N-(1-benzylpiperidin-4-yl)-2-(4-ethyl-6,6-dimethyl-2-oxomorpholin-3-yl)acetamide;
2-((4-ethyl-6,6-dimethyl-2-oxomorpholin-3-yl)-N-(1-phenylethyl)acetamide;
3-((2-(4-benzylpiperidin-1-yl)-2-oxoethyl)-4-ethyl-6,6-dimethylmorpholin-2-one;
2-((4-ethyl-6,6-dimethyl-2-oxomorpholin-3-yl)-N-(pyridin-4-yl)acetamide;
2-((4-ethyl-6,6-dimethyl-2-oxomorpholin-3-yl)-N-(quinazolin-2-yl)acetamide;
2-((4-ethyl-6,6-dimethyl-2-oxomorpholin-3-yl)-N-phenethylacetamide;
4-ethyl-6,6-dimethyl-3-(2-oxo-2-(4-phenylpiperazin-1-yl)ethyl)morpholin-2-one;
2-((4-ethyl-6,6-dimethyl-2-oxomorpholin-3-yl)-N-(pyridin-2-yl)acetamide;
2-((4-ethyl-6,6-dimethyl-2-oxomorpholin-3-yl)-N-(thiophen-2-ylmethyl)acetamide;
2-((4-ethyl-6,6-dimethyl-2-oxomorpholin-3-yl)-N-(furan-2-ylmethyl)acetamide;
2-((4-ethyl-6,6-dimethyl-2-oxomorpholin-3-yl)-N-methyl-N-phenylacetamide;
2-((4-ethyl-6,6-dimethyl-2-oxomorpholin-3-yl)-N-(pyrimidin-4-yl)acetamide;
N-(3,5-dimethylisoxazol-4-yl)-2-(4-ethyl-6,6-dimethyl-2-oxomorpholin-3-yl)acetamide;
2-((4-ethyl-6,6-dimethyl-2-oxomorpholin-3-yl)-N-(1-methyl-1H-pyrazol-5-yl)acetamide;
2-((4-ethyl-6,6-dimethyl-2-oxomorpholin-3-yl)-N-(3-phenyl-1,2,4-thiadiazol-5-yl)acetamide;
2-((4-ethyl-6,6-dimethyl-2-oxomorpholin-3-yl)-N-(1-methyl-1H-pyrazol-3-yl)acetamide;
2-((4-ethyl-6,6-dimethyl-2-oxomorpholin-3-yl)-N-(5-(trifluoromethyl)-1,3,4-thiadiazol-2-yl)acetamide;
4-ethyl-6,6-dimethyl-3-(2-oxo-2-(1H-pyrazol-1-yl)ethyl)morpholin-2-one;
N-(4,5-dimethylthiazol-2-yl)-2-(4-ethyl-6,6-dimethyl-2-oxomorpholin-3-yl)acetamide;
N,N-diethyl-2-(4-ethyl-6,6-dimethyl-2-oxomorpholin-3-yl)acetamide;
2-((4-ethyl-6,6-dimethyl-2-oxomorpholin-3-yl)-N-isobutylacetamide;
N-cyclohexyl-2-(4-ethyl-6,6-dimethyl-2-oxomorpholin-3-yl)acetamide;
2-((4-ethyl-6,6-dimethyl-2-oxomorpholin-3-yl)-N-propylacetamide;
N-tert-butyl-2-(4-ethyl-6,6-dimethyl-2-oxomorpholin-3-yl)acetamide;
N-(3,3-dimethylbutyl)-2-(4-ethyl-6,6-dimethyl-2-oxomorpholin-3-yl)acetamide;
2-((4-ethyl-6,6-dimethyl-2-oxomorpholin-3-yl)-N-(2,2,2-trifluoroethyl)acetamide;
N-cyclopentyl-2-(4-ethyl-6,6-dimethyl-2-oxomorpholin-3-yl)acetamide;
4-ethyl-6,6-dimethyl-3-(2-oxo-2-(pyrrolidin-1-yl)ethyl)morpholin-2-one;
N-ethyl-2-(4-ethyl-6,6-dimethyl-2-oxomorpholin-3-yl)acetamide;
N-sec-butyl-2-(4-ethyl-6,6-dimethyl-2-oxomorpholin-3-yl)acetamide;
N-(cyclohexylmethyl)-2-(4-ethyl-6,6-dimethyl-2-oxomorpholin-3-yl)acetamide;
2-((4-ethyl-6,6-dimethyl-2-oxomorpholin-3-yl)-N,N-dimethylacetamide;
N-(3-chlorobenzyl)-2-(4-ethyl-6,6-dimethyl-2-oxomorpholin-3-yl)acetamide;
N-(3,4-dichlorobenzyl)-2-(4-ethyl-6,6-dimethyl-2-oxomorpholin-3-yl)acetamide;

N-(2,6-dichlorobenzyl)-2-(4-ethyl-6,6-dimethyl-2-oxomorpholin-3-yl)acetamide;
N-(2-chlorobenzyl)-2-(4-ethyl-6,6-dimethyl-2-oxomorpholin-3-yl)acetamide;
4-ethyl-6,6-dimethyl-3-(2-(4-methylpiperazin-1-yl)-2-oxoethyl)morpholin-2-one;
N-cyclopropyl-2-(4-ethyl-6,6-dimethyl-2-oxomorpholin-3-yl)acetamide;
2-((4-ethyl-6,6-dimethyl-2-oxomorpholin-3-yl)-N-(4-phenoxyphenyl)acetamide;
N-(4-tert-butylthiazol-2-yl)-2-(4-ethyl-6,6-dimethyl-2-oxomorpholin-3-yl)acetamide;
2-((4-ethyl-6,6-dimethyl-2-oxomorpholin-3-yl)-N-(2-methylbenzyl)acetamide;
2-((4-ethyl-6,6-dimethyl-2-oxomorpholin-3-yl)-N-(3-methylbenzyl)acetamide;
2-((4-ethyl-6,6-dimethyl-2-oxomorpholin-3-yl)-N-(4-methylbenzyl)acetamide;
2-((4-ethyl-6,6-dimethyl-2-oxomorpholin-3-yl)-N-(2-(trifluoromethyl)benzyl)acetamide;
2-((4-ethyl-6,6-dimethyl-2-oxomorpholin-3-yl)-N-(3-(trifluoromethyl)benzyl)acetamide;
2-((4-ethyl-6,6-dimethyl-2-oxomorpholin-3-yl)-N-(4-(trifluoromethyl)benzyl)acetamide;
2-((4-ethyl-6,6-dimethyl-2-oxomorpholin-3-yl)-N-(2-methoxybenzyl)acetamide;
2-((4-ethyl-6,6-dimethyl-2-oxomorpholin-3-yl)-N-(3-methoxybenzyl)acetamide;
2-((4-ethyl-6,6-dimethyl-2-oxomorpholin-3-yl)-N-(4-methoxybenzyl)acetamide;
N-(biphenyl-2-ylmethyl)-2-(4-ethyl-6,6-dimethyl-2-oxomorpholin-3-yl)acetamide;
N-(biphenyl-3-ylmethyl)-2-(4-ethyl-6,6-dimethyl-2-oxomorpholin-3-yl)acetamide;
N-(biphenyl-4-ylmethyl)-2-(4-ethyl-6,6-dimethyl-2-oxomorpholin-3-yl)acetamide;
2-((4-ethyl-2-oxo-6-phenylmorpholin-3-yl)-N-(4-isopropylphenyl)acetamide;
2-(((6S)-4-ethyl-2-oxo-6-phenylmorpholin-3-yl)-N-(4-isopropylphenyl)acetamide;
2-(((6R)-4-ethyl-2-oxo-6-phenylmorpholin-3-yl)-N-(4-isopropylphenyl)acetamide;
2-(((6S)-4-ethyl-6-methyl-2-oxomorpholin-3-yl)-N-(4-isopropylphenyl)acetamide;
2-(((6R)-4-ethyl-6-methyl-2-oxomorpholin-3-yl)-N-(4-isopropylphenyl)acetamide;
2-((4-ethyl-6-methyl-2-oxo-6-phenylmorpholin-3-yl)-N-(4-isopropylphenyl)acetamide;
2-((4-ethyl-6-methyl-2-oxo-6-phenylmorpholin-3-yl)-N-(4-isopropylphenyl)acetamide;
2-((4-ethyl-5,5,6,6-tetramethyl-2-oxomorpholin-3-yl)-N-(4-isopropylphenyl)acetamide;
2-((4-ethyl-5,6,6-trimethyl-2-oxomorpholin-3-yl)-N-(4-isopropylphenyl)acetamide;
N-(4-isopropylphenyl)-2-(4,6,6-triethyl-2-oxomorpholin-3-yl)acetamide;
2-((4-ethyl-6,6-dimethyl-2-oxomorpholin-3-yl)-N-(4-isopropylphenyl)-2-methylpropanamide;
2-((4-benzyl-6,6-dimethyl-2-oxomorpholin-3-yl)-N-(4-isopropylphenyl)acetamide;
2-((6,6-dimethyl-2-oxomorpholin-3-yl)-N-(4-isopropylphenyl)acetamide;
N-(4-isopropylphenyl)-2-(4,6,6-trimethyl-2-oxomorpholin-3-yl)acetamide;
2-((6,6-dimethyl-2-oxo-4-propylmorpholin-3-yl)-N-(4-isopropylphenyl)acetamide;
2-((4-cyclopentyl-6,6-dimethyl-2-oxomorpholin-3-yl)-N-(4-isopropylphenyl)acetamide;
2-((4-isopropyl-6,6-dimethyl-2-oxomorpholin-3-yl)-N-(4-isopropylphenyl)acetamide;
2-((4-cyclopropyl-6,6-dimethyl-2-oxomorpholin-3-yl)-N-(4-isopropylphenyl)acetamide;
2-((6,6-dimethyl-2-oxo-4-(2,2,2-trifluoroethyl)morpholin-3-yl)-N-(4-isopropylphenyl)acetamide;
2-((4-(2-fluoroethyl)-6,6-dimethyl-2-oxomorpholin-3-yl)-N-(4-isopropylphenyl)acetamide;
2-((4-acetyl-6,6-dimethyl-2-oxomorpholin-3-yl)-N-(4-isopropylphenyl)acetamide;
2-((6,6-dimethyl-4-(methylsulfonyl)-2-oxomorpholin-3-yl)-N-(4-isopropylphenyl)acetamide;
2-((6,6-dimethyl-2-oxo-4-phenylmorpholin-3-yl)-N-(4-isopropylphenyl)acetamide;
2-((4-ethyl-6,6-dimethyl-2-oxomorpholin-3-yl)-N-(4-isopropylphenyl)acetamide;
4-ethyl-6,6-dimethyl-3-(2-morpholino-2-oxoethyl)morpholin-2-one.

According to a second aspect, the invention relates to the compounds as described herein, more specifically the compounds according to the formulae described herein (A' and A to Fd) and embodiments thereof, and according to the claims, for use as a medicament, more in particular for use as an antifungal medicament and for the use in the prevention or treatment of a fungal infection in or on a subject (animal, mammal or human).
The present invention also relates to the use of the compounds of the formulae described herein and embodiments thereof as antifungal compounds, more particularly as compounds active against Candida species (e.g. C. albicans, C. glabrata, C. krusei), Aspergillus species (e.g. A. flavus, A. fumigatus, A. clavatus), Fusarium species (e.g. F. oxysporum, F. culmorum) or other fungi. The invention also relates to the use of compounds of the formulae, embodiments and claims herein for the manufacture of a medicament or as a pharmaceutically active ingredient, especially as a fungal replication inhibitor or fungicidal compound, for instance for the manufacture of a medicament or pharmaceutical composition having antifungal activity for the prevention and/or treatment of fungal infections in humans, mammals and animals in general. The present invention further relates to a method of prevention or treatment of a fungal infection, preferably a Candida species, Aspergillus species or Fusarium species infection in a subject, an animal, including mammals, including a human, comprising administering to the subject in need of such treatment a therapeutically effective

amount of a compound of the formulae, embodiments and claims herein as an active ingredient, preferably in admixture with at least a pharmaceutically acceptable carrier.

According to another aspect, the invention relates to the compounds as described herein, more specifically the compounds according to the formulae described herein and embodiments thereof, for use as plant antifungals, more in particular for use as an antifungal in the prevention, reduction or eradication of a fungal infection in or on plants (e.g. in agriculture).

According to another aspect, the invention relates to methods to prevent, reduce or eradicate fungal infection in or on plants (e.g. in agriculture), or on materials for industrial or medical use, for example by inhibiting biofilm formation such as fungal biofilm formation.

Another aspect of the invention further relates to methods for the preparation of compounds of formulae and claims herein, more specifically the compounds according to the formulae described herein and embodiments thereof. Also the intermediates used in the preparation methods described herein are aspects of the present invention.

In a particular embodiment of this invention, the method of preparation of the compounds of the invention comprises the following steps (or a selection of a number of these steps):

- preparing a substituted morpholin-2-one derivative by condensation of a suitable amino-alcohol derivative with glyoxal or with a suitable acetate derivative of formula LG-$CH_2$-COOR (R being preferably an alkyl or a benzyl radical);
- reacting this morpholin-2-one derivative with a suitable acetate derivative of formula LG-$CH_2$-COOR (R being preferably an alkyl or a benzyl radical) in order to obtain a 2-(substituted-2-oxomorpholin-3-yl)acetate derivative;
- converting the obtained ester in a carboxylic acid derivative via a saponification reaction;
- converting the acid derivative in a desired amide derivative using standard peptide coupling reagents;
- preparing a 2-(4-benzyl-2-oxomorpholin-3-yl)-N-substituted acetamide derivative using the previous detailed step;
- removing the N-benzyl protecting group from the morpholin-2-one derivative in order to obtain a 2-(2-oxomorpholin-3-yl)-N-substituted acetamide derivative;
- condensing the 2-(2-oxomorpholin-3-yl)-N-substituted acetamide derivative with a suitable reagent in order to substitute the free nitrogen atom from the morpholin-2-one ring and obtain the desired compounds.

Yet another aspect of the present invention relates to a pharmaceutical composition comprising the compounds of the invention according to formulae, embodiments and claims herein, in admixture with at least a pharmaceutically acceptable carrier, the active ingredient preferably being in a concentration range of about 0.1 to 100% by weight, and to these compositions for use as a medicament, more specifically for the treatment or prevention of subjects suffering from a fungal infection, in particular a Candida species, Aspergillus species or Fusarium species infection.

The invention further relates to the use of a composition comprising (a) one or more derivatives of formulae and embodiments herein, and (b) one or more fungistatic or fungicidal compounds as biologically active agents in respective proportions such as to provide a synergistic effect against a fungal infection in a mammal, for instance in the form of a combined preparation for simultaneous, separate or sequential use in fungal infection therapy. Within the framework of this embodiment of the invention, the inhibitors used as a therapeutically active ingredients (b) may belong to categories already known in the art.

More generally, the invention relates to the compounds of formulae and embodiments thereof, being useful as agents having biological activity or as diagnostic agents. The invention also relates to the use of these compounds of formulae and embodiments herein, as agents having biological activity or as diagnostic agents.

Any of the uses mentioned with respect to the present invention may be restricted to a non-medical use, a non-therapeutic use, a non-diagnostic use, or exclusively an in vitro use, or a use related to cells remote from an animal. For example, as described herein, the compounds of present invention can be used to eradicate or reduce or prevent fungal infections on plants or materials (e.g. biofilm formation).

## DETAILED DESCRIPTION OF THE FIGURES

Figure 1: Results of a *C. elegans in vivo* efficacy study of CPD-06. The figure shows the % survival of *C. albicans* infected *C. elegans* treated with different concentrations of CPD-06.

Figure 2: Results of a *mouse in vivo* efficacy study of CPD-06. The figure shows the colony forming units (CFU) from kidneys of *C. albicans* infected mice treated with CPD-06. The graphs represent the CFU from kidneys (k) of BALB/c mice infected with $2x10^3$ cells of *C. albicans* isolated SC5314. Control mice were infected and received the vehicle alone (5%DMSO and Methyl cellulose 0.5%, referred to as "Control"). The first group of mice was treated with fluconazole (10mg/kg) (referred to as "Fluconazole"). A second group of mice received Cpd06 once a day (referred to as "1 Dose"). The third group received twice a day Cpd06 (referred to as "2 Dose"). Bars represent the CFU means and standard deviations from organs of five animals in each group. * Significant ($p < 0.02$) difference

in relation to the control group.

## DETAILED DESCRIPTION OF THE INVENTION

The present invention will be described with respect to particular embodiments but the invention is not limited thereto.

The term "treat" or "treating" as used herein is intended to refer to administration of a compound or composition of the invention to a subject for the purpose of effecting a therapeutic or prophylactic benefit through inhibition of fungal growth. Treating includes reversing, ameliorating, alleviating, inhibiting the progress of, lessening the severity of, or preventing a disease, disorder, or condition, or one or more symptoms of such disease, disorder or condition. The term "subject" refers to an animal or mammalian patient in need of such treatment, such as a human.

It is to be noticed that the term "comprising", used in the claims, should not be interpreted as being restricted to the means listed thereafter; it does not exclude other elements or steps.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments. Where an indefinite or definite article is used when referring to a singular noun e.g. "a" or "an", "the", this includes a plural of that noun unless something else is specifically stated.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects.

In each of the following definitions, the number of carbon atoms represents the maximum number of carbon atoms generally optimally present in the substituent or linker; it is understood that where otherwise indicated in the present application, the number of carbon atoms represents the optimal maximum number of carbon atoms for that particular substituent or linker.

The term "leaving group" or "LG" as used herein means a chemical group which is susceptible to be displaced by a nucleophile or cleaved off or hydrolyzed in basic or acidic conditions. In a particular embodiment, a leaving group is selected from a halogen atom (e.g., Cl, Br, I) or a sulfonate (e.g., mesylate, tosylate, triflate).

The term "protecting group" refers to a moiety of a compound that masks or alters the properties of a functional group or the properties of the compound as a whole. The chemical substructure of a protecting group varies widely. One function of a protecting group is to serve as intermediates in the synthesis of the parental drug substance. Chemical protecting groups and strategies for protection/deprotection are well known in the art. See: "Protective Groups in Organic Chemistry", Theodora W. Greene (John Wiley & Sons, Inc., New York, 1991. Protecting groups are often utilized to mask the reactivity of certain functional groups, to assist in the efficiency of desired chemical reactions, e.g. making and breaking chemical bonds in an ordered and planned fashion. Protection of functional groups of a compound alters other physical properties besides the reactivity of the protected functional group, such as the polarity, lipophilicity (hydrophobicity), and other properties which can be measured by common analytical tools. Chemically protected intermediates may themselves be biologically active or inactive.

Protected compounds may also exhibit altered, and in some cases, optimized properties in vitro and in vivo, such as passage through cellular membranes and resistance to enzymatic degradation or sequestration. In this role, protected compounds with intended therapeutic effects may be referred to as prodrugs. Another function of a protecting group is to convert the parental drug into a prodrug, whereby the parental drug is released upon conversion of the prodrug in vivo. Because active prodrugs may be absorbed more effectively than the parental drug, prodrugs may possess greater potency in vivo than the parental drug. Protecting groups are removed either in vitro, in the instance of chemical intermediates, or in vivo, in the case of prodrugs. With chemical intermediates, it is not particularly important that the resulting products after deprotection, e.g. alcohols, be physiologically acceptable, although in general it is more desirable if the products are pharmacologically innocuous.

The term "hydrocarbyl", "$C_{1-18}$ hydrocarbyl", "hydrocarbyl group" or "$C_{1-18}$ hydrocarbyl group" as used herein refers to $C_1$-$C_{18}$ normal, secondary, tertiary, unsaturated or saturated, non-aromatic, acyclic or cyclic, hydrocarbons and combinations thereof. This term therefore comprises alkyl, alkenyl, alkynyl, cycloalkyl, cycloalkenyl and cycloalkynyl.

The terminology "heterohydrocarbyl", "hetero $C_{1-18}$ hydrocarbyl", "heterohydrocarbyl group", "hetero $C_{1-18}$ hydrocarbyl group" or "hydrocarbyl group which optionally includes one or more heteroatoms, said heteroatoms being selected from the atoms consisting of O, S, and N" as used herein, refers to a hydrocarbyl group where one or more carbon atoms are replaced by an oxygen, nitrogen or sulphur atom(s) and thus includes heteroalkyl, heteroalkenyl, heteroalkynyl and non-aromatic heterocycle. This term therefore comprises as an example alkoxy, alkenyloxy, $C_w$alkyl-O-$C_{18-w}$alkyl,

$C_w$alkenyl-O-alkyl, $C_w$alkyl-NH-$C_{18-w}$alkenyl, among others, wherein w is selected from any number between 1 and 18.

The term "alkyl" or "$C_{1-18}$ alkyl" as used herein means $C_1$-$C_{18}$ normal, secondary, or tertiary, linear, branched or straight hydrocarbon with no site of unsaturation. Examples are methyl, ethyl, 1-propyl (n-propyl), 2-propyl (iPr), 1-butyl, 2-methyl-1-propyl(i-Bu), 2-butyl (s-Bu), 2-dimethyl-2-propyl (t-Bu), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, cyclopropylethylene, methylcyclopropylene, 2,3-dimethyl-2-butyl, 3,3-dimethyl-2-butyl, *n*-heptyl, *n*-octyl, *n*-nonyl, *n*-decyl, *n*-undecyl, *n*-dodecyl, *n*-tridecyl, *n*-tetradecyl, *n*-pentadecyl, *n*-hexadecyl, *n*-heptadecyl, *n*-octadecyl, *n*-nonadecyl, *n*-icosyl. In a particular embodiment, the term alkyl refers to $C_{1-12}$ hydrocarbons, yet more in particular to $C_{1-6}$ hydrocarbons as further defined herein above.

The term "acyclic alkyl" as used herein means $C_1$-$C_{18}$ normal, secondary, or tertiary, linear, branched or straight, hydrocarbon with no site of unsaturation. Examples are methyl, ethyl, 1-propyl, 2-propyl (iPr), 1-butyl, 2-methyl-1-propyl(i-Bu), 2-butyl (s-Bu), 2-methyl-2-propyl (t-Bu), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl *n*-heptyl, *n*-octyl, *n*-nonyl, *n*-decyl, *n*-undecyl, *n*-dodecyl, *n*-tridecyl, *n*-tetradecyl, *n*-pentadecyl, *n*-hexadecyl, *n*-heptadecyl, *n*-octadecyl, *n*-nonadecyl and *n*-icosyl.

The term "cycloalkyl" or "$C_{3-18}$ cycloalkyl" as used herein and unless otherwise stated means a saturated hydrocarbon monovalent radical having from 3 to 18 carbon atoms consisting of or comprising a $C_{3-10}$ monocyclic or $C_{7-18}$ polycyclic saturated hydrocarbon, such as for instance cyclopropyl, cyclobutyl, cyclopentyl, cyclopropylethylene, methylcyclopropylene, cyclohexyl, cycloheptyl, cyclooctyl, cyclooctylmethylene, norbornyl, fenchyl, trimethyltricycloheptyl, decalinyl, adamantyl and the like.

The term "alkenyl" or "$C_{2-18}$alkenyl" as used herein is $C_2$-$C_{18}$ normal, secondary or tertiary, linear, branched or straight hydrocarbon with at least one site (usually 1 to 3, preferably 1) of unsaturation, namely a carbon-carbon, sp2 double bond. Examples include, but are not limited to: ethylene or vinyl ($-CH=CH_2$), allyl ($-CH_2CH=CH_2$), and 5-hexenyl ($-CH_2CH_2CH_2CH_2CH=CH_2$). The double bond may be in the cis or trans configuration. In a particular embodiment, the term alkenyl refers to $C_{1-12}$ hydrocarbons, yet more in particular to $C_{1-6}$ hydrocarbons as further defined herein above.

The term "acyclic alkenyl" as used herein refers to $C_2$-$C_{18}$ normal, secondary or tertiary, linear, branched or straight hydrocarbon with at least one site (usually 1 to 3, preferably 1) of unsaturation, namely a carbon-carbon, sp2 double bond. Examples include, but are not limited to: ethylene or vinyl ($-CH=CH_2$), allyl ($-CH_2CH=CH_2$) and 5-hexenyl ($-CH_2CH_2CH_2CH_2CH=CH_2$). The double bond may be in the cis or trans configuration.

The term "cycloalkenyl" as used herein refers to a non-aromatic hydrocarbon radical having from 4 to 18 carbon atoms with at least one site (usually 1 to 3, preferably 1) of unsaturation, namely a carbon-carbon, sp2 double bond and consisting of or comprising a $C_{4-10}$ monocyclic or $C_{7-18}$ polycyclic hydrocarbon. Examples include, but are not limited to: cyclopentenyl ($-C_5H_7$), cyclopentenylpropylene, methylcyclohexenylene and cyclohexenyl ($-C_6H_9$). The double bond may be in the cis or trans configuration. In a particular embodiment, The term "cycloalkenyl" as used herein refers to a non-aromatic hydrocarbon radical having from 3 to 18 carbon atoms with at least one site (usually 1 to 3, preferably 1) of unsaturation, namely a carbon-carbon, sp2 double bond and consisting of or comprising a $C_{3-10}$ monocyclic or $C_{7-18}$ polycyclic hydrocarbon. Examples include, but are not limited to: cyclopentenyl ($-C_5H_7$), cyclopentenylpropylene, methylcyclohexenylene and cyclohexenyl ($-C_6H_9$). The double bond may be in the cis or trans configuration.

The term "alkynyl" or "$C_{2-18}$alkynyl" as used herein refers to $C_2$-$C_{18}$ normal, secondary, tertiary, linear, branched or straight hydrocarbon with at least one site (usually 1 to 3, preferably 1) of unsaturation, namely a carbon-carbon, sp triple bond. Examples include, but are not limited to: ethynyl ($-C\equiv CH$), 3-ethyl-cyclohept-1-ynylene, 4-cyclohept-1-yn-methylene and 1-propynyl (propargyl, $-CH_2C\equiv CH$). In a particular embodiment, the term alkenyl refers to $C_{1-12}$ hydrocarbons, yet more in particular to $C_{1-6}$ hydrocarbons as further defined herein above.

The term "acyclic alkynyl" as used herein refers to $C_2$-$C_{18}$ normal, secondary, tertiary, linear, branched or straight hydrocarbon with at least one site (usually 1 to 3, preferably 1) of unsaturation, namely a carbon-carbon, sp triple bond. Examples include, but are not limited to: ethynyl ($-C\equiv CH$) and 1-propynyl (propargyl, $-CH_2C\equiv CH$).

The term "cycloalkynyl" as used herein refers to a non-aromatic hydrocarbon radical having from 5 to 18 carbon atoms with at least one site (usually 1 to 3, preferably 1) of unsaturation, namely a carbon-carbon, sp triple bond and consisting of or comprising a $C_{5-10}$ monocyclic or $C_{7-18}$ polycyclic hydrocarbon. Examples include, but are not limited to: cyclohept-1-yne, 3-ethyl-cyclohept-1-ynylene, 4-cyclohept-1-yn-methylene and ethylene-cyclohept-1-yne. In a particular embodiment, the term "cycloalkynyl" as used herein refers to a non-aromatic hydrocarbon radical having from 3 to 18 carbon atoms with at least one site (usually 1 to 3, preferably 1) of unsaturation, namely a carbon-carbon, sp triple bond and consisting of or comprising a $C_{3-10}$ monocyclic or $C_{7-18}$ polycyclic hydrocarbon. Examples include, but are not limited to: cyclohept-1-yne, 3-ethyl-cyclohept-1-ynylene, 4-cyclohept-1-yn-methylene and ethylene-cyclohept-1-yne.

The term "alkylene" as used herein each refer to a saturated, branched or straight chain hydrocarbon radical of 1-18 carbon atoms (more in particular $C_{1-12}$ or $C_{1-6}$ carbon atoms), and having two monovalent radical centers derived by the removal of two hydrogen atoms from the same or two different carbon atoms of a parent alkane. Typical alkylene

radicals include, but are not limited to: methylene ($-CH_2-$), 1,2-ethyl ($-CH_2CH_2-$), 1,3-propyl ($-CH_2CH_2CH_2-$), 1,4-butyl ($-CH_2CH_2CH_2CH_2-$), and the like.

The term "alkenylene" as used herein each refer to a branched or straight chain hydrocarbon radical of 2-18 carbon atoms (more in particular $C_{2-12}$ or $C_{2-6}$ carbon atoms) with at least one site (usually 1 to 3, preferably 1) of unsaturation, namely a carbon-carbon, sp2 double bond, and having two monovalent radical centers derived by the removal of two hydrogen atoms from the same or two different carbon atoms of a parent alkene.

The term "alkynylene" as used herein each refer to a branched or straight chain hydrocarbon radical of 2-18 carbon atoms (more in particular $C_{2-12}$ or $C_{2-6}$ carbon atoms) with at least one site (usually 1 to 3, preferably 1) of unsaturation, namely a carbon-carbon, sp triple bond, and having two monovalent radical centers derived by the removal of two hydrogen atoms from the same or two different carbon atoms of a parent alkyne.

The term "heteroalkyl" as used herein refers to an acyclic alkyl wherein one or more carbon atoms are replaced by an oxygen, nitrogen or sulphur atom. The term heteroalkyl thus comprises -O-alkyl, -NH-alkyl, $-N(alkyl)_2$, and -S-alkyl.

The term "heteroalkenyl" as used herein refers to an acyclic alkenyl wherein one or more carbon atoms are replaced by an oxygen, nitrogen or sulphur atom. The term heteroalkenyl thus comprises -O-alkenyl, -NH-alkenyl, $-N(alkenyl)_2$, -N(alkyl)(alkenyl), and -S-alkenyl.

The term "heteroalkynyl" as used herein refers to an acyclic alkynyl wherein one or more carbon atoms are replaced by an oxygen, nitrogen or sulphur atom. The term heteroalkynyl thus comprises -O-alkynyl, -NH-alkynyl, $-N(alkynyl)_2$, -N(alkyl)(alkynyl), - N(alkenyl)(alkynyl), and -S-alkynyl.

The term "heteroalkylene" as used herein refers to an alkylene wherein one or more carbon atoms are replaced by an oxygen, nitrogen or sulphur atom.

The term "heteroalkenylene" as used herein refers to an alkenylene wherein one or more carbon atoms are replaced by an oxygen, nitrogen or sulphur atom.

The term "heteroalkynylene" as used herein refers to an alkynylene wherein one or more carbon atoms are replaced by an oxygen, nitrogen or sulphur atom.

The term "aryl" as used herein means an aromatic hydrocarbon radical of 6-20 carbon atoms derived by the removal of hydrogen from a carbon atom of a parent aromatic ring system. A "parent aromatic ring system" means a monocyclic aromatic ring system or a bi- or tricyclic ring system of which at least one ring is aromatic. Typical aryl groups include, but are not limited to 1 ring, or 2 or 3 rings fused together and includes radicals derived from benzene, naphthalene, anthracene, biphenyl, 2,3-dihydro-1H-indene, and the like.

The term "arylalkyl" or "arylalkyl-" as used herein refers to an acyclic alkyl radical in which one of the hydrogen atoms bonded to a carbon atom, typically a terminal or sp3 carbon atom, is replaced with an aryl radical. Typical arylalkyl groups include, but are not limited to, benzyl, 2-phenylethan-1-yl, 2-phenylethen-1-yl, naphthylmethyl, 2-naphthylethyl, and the like. The arylalkyl group comprises 6 to 20 carbon atoms, e.g. the alkyl moiety of the arylalkyl group is 1 to 6 carbon atoms and the aryl moiety is 6 to 14 carbon atoms.

The term "arylalkenyl" or "arylalkenyl-" as used herein refers to an acyclic alkenyl radical in which one of the hydrogen atoms bonded to a carbon atom, is replaced with an aryl radical. The arylalkenyl group comprises 6 to 20 carbon atoms, e.g. the alkenyl moiety of the arylalkenyl group is 1 to 6 carbon atoms and the aryl moiety is 6 to 14 carbon atoms.

The term "arylalkynyl" or "arylalkynyl-" as used herein refers to an acyclic alkynyl radical in which one of the hydrogen atoms bonded to a carbon atom, is replaced with an aryl radical. The arylalkynyl group comprises 6 to 20 carbon atoms, e.g. the alkynyl moiety of the arylalkynyl group is 1 to 6 carbon atoms and the aryl moiety is 6 to 14 carbon atoms.

The term "arylheteroalkyl" or "arylheteroalkyl-" as used herein refers to a heteroalkyl radical in which one of the hydrogen atoms bonded to a carbon atom, typically a terminal or sp3 carbon atom, is replaced with an aryl radical. The arylheteroalkyl group comprises 6 to 20 carbon atoms, e.g. the heteroalkyl moiety of the arylheteroalkyl group is 1 to 6 carbon atoms and the aryl moiety is 6 to 14 carbon atoms.

The term "arylheteroalkenyl" or "arylheteroalkenyl-" as used herein refers to a heteroalkenyl radical in which one of the hydrogen atoms bonded to a carbon atom, is replaced with an aryl radical. The arylheteroalkenyl group comprises 6 to 20 carbon atoms, e.g. the heteroalkenyl moiety of the arylheteroalkenyl group is 1 to 6 carbon atoms and the aryl moiety is 6 to 14 carbon atoms.

The term "arylheteroalkynyl" or "arylheteroalkynyl-" as used herein refers to a heteroalkynyl radical in which one of the hydrogen atoms bonded to a carbon atom, is replaced with an aryl radical. The arylheteroalkynyl group comprises 6 to 20 carbon atoms, e.g. the heteroalkynyl moiety of the arylheteroalkynyl group is 1 to 6 carbon atoms and the aryl moiety is 6 to 14 carbon atoms.

The term "heterocycle" as used herein means a saturated, unsaturated or aromatic ring system of 3 to 18 atoms including at least one N, O, S, or P. Heterocycle thus include heteroaryl groups. Heterocycle as used herein includes by way of example and not limitation these heterocycles described in Paquette, Leo A. "Principles of Modern Heterocyclic Chemistry" (W.A. Benjamin, New York, 1968), particularly Chapters 1, 3, 4, 6, 7, and 9; "The Chemistry of Heterocyclic Compounds, A series of Monographs" (John Wiley & Sons, New York, 1950 to present), in particular Volumes 13, 14, 16, 19, and 28; Katritzky, Alan R., Rees, C.W. and Scriven, E. "Comprehensive Heterocyclic Chemistry" (Pergamon

Press, 1996); and J. Am. Chem. Soc. (1960) 82:5566. In a particular embodiment, the term means pyridyl, dihydroypyridyl, tetrahydropyridyl (piperidyl), thiazolyl, tetrahydrothiophenyl, sulfur oxidized tetrahydrothiophenyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, piperidinyl, 4-piperidonyl, pyrrolidinyl, 2-pyrrolidonyl, pyrrolinyl, tetrahydrofuranyl, bis-tetrahydrofuranyl, tetrahydropyranyl, bis-tetrahydropyranyl, tetrahydroquinolinyl, tetrahydroisoquinolinyl, decahydroquinolinyl, octahydroisoquinolinyl, azocinyl, triazinyl, 6H-1,2,5-thiadiazinyl, 2H,6H-1,5,2-dithiazinyl, thianthrenyl, pyranyl, isobenzofuranyl, chromenyl, xanthenyl, phenoxathinyl, 2H-pyrrolyl, isothiazolyl, isoxazolyl, pyrazinyl, pyridazinyl, indolizinyl, isoindolyl, 3H-indolyl, 1H-indazoly, purinyl, 4H-quinolizinyl, phthalazinyl, naphthyridinyl, quinoxalinyl, quinazolinyl, cinnolinyl, pteridinyl, 4aH-carbazolyl, carbazolyl, ß-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, piperazinyl, indolinyl, isoindolinyl, quinuclidinyl, morpholinyl, oxazolidinyl, benzotriazolyl, benzisoxazolyl, oxindolyl, benzoxazolinyl, benzothienyl, benzothiazolyl and isatinoyl.

The term "heteroaryl" means an aromatic ring system of 5 to 18 atoms including at least one N, O, S, or P and thus refers to aromatic heterocycles. Examples of heteroaryl include but are not limited to pyridyl, dihydropyridyl, pyridazinyl, pyrimidinyl, pyrazinyl, s-triazinyl, oxazolyl, imidazolyl, thiazolyl, isoxazolyl, pyrazolyl, isothiazolyl, furyl, thienyl, and pyrrolyl.

The term "non-aromatic heterocycle" as used herein means a saturated or unsaturated non-aromatic ring system of 3 to 18 atoms including at least one N, O, S, or P.

The term "heterocycle-alkyl" or "heterocycle-alkyl-" as used herein refers to an acyclic alkyl radical in which one of the hydrogen atoms bonded to a carbon atom, typically a terminal or sp3 carbon atom, is replaced with a heterocyle radical. An example of a heterocycle-alkyl group is 2-pyridyl-methylene. The heterocycle-alkyl group comprises 6 to 20 atoms, e.g. the alkyl moiety of the heterocycle-alkyl group is 1 to 6 carbon atoms and the heterocycle moiety is 3 to 14 atoms.

The term "heterocycle-alkenyl" or "heterocycle-alkenyl-" as used herein refers to an acyclic alkenyl radical in which one of the hydrogen atoms bonded to a carbon atom, is replaced with an heterocycle radical. The heterocycle-alkenyl group comprises 6 to 20 atoms, e.g. the alkenyl moiety of the heterocycle-alkenyl group is 1 to 6 carbon atoms and the heterocycle moiety is 3 to 14 atoms.

The term "heterocycle-alkynyl" or "heterocycle-alkynyl-" as used herein refers to an acyclic alkynyl radical in which one of the hydrogen atoms bonded to a carbon atom, is replaced with a heterocycle radical. The heterocycle-alkynyl group comprises 6 to 20 atoms, e.g. the alkynyl moiety of the heterocycle-alkynyl group is 1 to 6 carbon atoms and the heterocycle moiety is 3 to 14 atoms.

The term "heterocycle-heteroalkyl" or "heterocycle-heteroalkyl-" as used herein refers to a heteroalkyl radical in which one of the hydrogen atoms bonded to a carbon atom, typically a terminal or sp3 carbon atom, is replaced with a heterocyle radical. The heterocycle-heteroalkyl group comprises 6 to 20 atoms, e.g. the heteroalkyl moiety of the heterocycle-heteroalkyl group is 1 to 6 carbon atoms and the heterocycle moiety is 3 to 14 atoms.

The term "heterocycle-heteroalkenyl" or "heterocycle-heteroalkenyl-" as used herein refers to a heteroalkenyl radical in which one of the hydrogen atoms bonded to a carbon atom, is replaced with an heterocycle radical. The heterocycle-heteroalkenyl group comprises 6 to 20 atoms, e.g. the heteroalkenyl moiety of the heterocycle-heteroalkenyl group is 1 to 6 carbon atoms and the heterocycle moiety is 3 to 14 atoms.

The term "heterocycle-heteroalkynyl" or "heterocycle-heteroalkynyl-" as used herein refers to a heteroalkynyl radical in which one of the hydrogen atoms bonded to a carbon atom, is replaced with a heterocycle radical. The heterocycle-heteroalkynyl group comprises 6 to 20 atoms, e.g. the heteroalkynyl moiety of the heterocycle-heteroalkynyl group is 1 to 6 carbon atoms and the heterocycle moiety is 3 to 14 atoms.

The term "heteroaryl-alkyl" or "heteroaryl-alkyl-" as used herein refers to an acyclic alkyl radical in which one of the hydrogen atoms bonded to a carbon atom, typically a terminal or sp3 carbon atom, is replaced with a heteraryl radical. An example of a heteroaryl-alkyl group is 2-pyridyl-methylene. The heteroaryl-alkyl group comprises 6 to 20 atoms, e.g. the alkyl moiety of the heteroaryl-alkyl group is 1 to 6 carbon atoms and the heteroaryl moiety is 5 to 14 atoms.

The term "heteroaryl-alkenyl" or "heteroaryl-alkenyl-" as used herein refers to an acyclic alkenyl radical in which one of the hydrogen atoms bonded to a carbon atom, is replaced with an heteroaryl radical. The heteroaryl-alkenyl group comprises 6 to 20 atoms, e.g. the alkenyl moiety of the heteroaryl-alkenyl group is 1 to 6 carbon atoms and the heteroaryl moiety is 5 to 14 atoms.

The term "heteroaryl-alkynyl" or "heteroaryl-alkynyl-" as used herein refers to an acyclic alkynyl radical in which one of the hydrogen atoms bonded to a carbon atom, is replaced with a heteroaryl radical. The heteroaryl-alkynyl group comprises 6 to 20 atoms, e.g. the alkynyl moiety of the heteroaryl-alkynyl group is 1 to 6 carbon atoms and the heteroaryl moiety is 5 to 14 atoms.

The term "heteroaryl-heteroalkyl" or "heteroaryl-heteroalkyl-" as used herein refers to a heteroalkyl radical in which one of the hydrogen atoms bonded to a carbon atom, typically a terminal or sp3 carbon atom, is replaced with a heterocyle radical. The heteroaryl-heteroalkyl group comprises 6 to 20 atoms, e.g. the heteroalkyl moiety of the heteroaryl-heteroalkyl group is 1 to 6 carbon atoms and the heteroaryl moiety is 5 to 14 atoms.

The term "heteroaryl-heteroalkenyl" or "heteroaryl-heteroalkenyl-" as used herein refers to a heteroalkenyl radical in which one of the hydrogen atoms bonded to a carbon atom, is replaced with an heteroaryl radical. The heteroaryl-heteroalkenyl group comprises 6 to 20 atoms, e.g. the heteroalkenyl moiety of the heteroaryl-heteroalkenyl group is 1 to 6 carbon atoms and the heteroaryl moiety is 5 to 14 atoms.

The term "heteroaryl-heteroalkynyl" or "heteroaryl-heteroalkynyl-" as used herein refers to a heteroalkynyl radical in which one of the hydrogen atoms bonded to a carbon atom, is replaced with a heteroaryl radical. The heteroaryl-heteroalkynyl group comprises 6 to 20 atoms, e.g. the heteroalkynyl moiety of the heteroaryl-heteroalkynyl group is 1 to 6 carbon atoms and the heteroaryl moiety is 5 to 14 atoms.

The term "non-aromatic heterocycle-alkyl" or "non-aromatic heterocycle-alkyl-" as used herein refers to an acyclic alkyl radical in which one of the hydrogen atoms bonded to a carbon atom, typically a terminal or sp3 carbon atom, is replaced with a non-aromatic heterocycle radical. The non-aromatic heterocycle-alkyl group comprises 6 to 20 atoms, e.g. the alkyl moiety of the non-aromatic heterocycle-alkyl group is 1 to 6 carbon atoms and the non-aromatic heterocycle moiety is 3 to 14 atoms.

The term "non-aromatic heterocycle-alkenyl" or "non-aromatic heterocycle-alkenyl-" as used herein refers to an acyclic alkenyl radical in which one of the hydrogen atoms bonded to a carbon atom, is replaced with an non-aromatic heterocycle radical. The non-aromatic heterocycle-alkenyl group comprises 6 to 20 atoms, e.g. the alkenyl moiety of the non-aromatic heterocycle-alkenyl group is 1 to 6 carbon atoms and the non-aromatic heterocycle moiety is 3 to 14 atoms.

The term "non-aromatic heterocycle-alkynyl" or "non-aromatic heterocycle-alkynyl-" as used herein refers to an acyclic alkynyl radical in which one of the hydrogen atoms bonded to a carbon atom, is replaced with a non-aromatic heterocycle radical. The non-aromatic heterocycle-alkynyl group comprises 6 to 20 atoms, e.g. the alkynyl moiety of the non-aromatic heterocycle-alkynyl group is 1 to 6 carbon atoms and the non-aromatic heterocycle moiety is 3 to 14 atoms.

The term "non-aromatic heterocycle-heteroalkyl" or "non-aromatic heterocycle-heteroalkyl-" as used herein refers to a heteroalkyl radical in which one of the hydrogen atoms bonded to a carbon atom, typically a terminal or sp3 carbon atom, is replaced with a heterocyle radical. The non-aromatic heterocycle-heteroalkyl group comprises 6 to 20 atoms, e.g. the heteroalkyl moiety of the non-aromatic heterocycle-heteroalkyl group is 1 to 6 carbon atoms and the non-aromatic heterocycle moiety is 3 to 14 atoms.

The term "non-aromatic heterocycle-heteroalkenyl" or "non-aromatic heterocycle-heteroalkenyl-" as used herein refers to a heteroalkenyl radical in which one of the hydrogen atoms bonded to a carbon atom, is replaced with an non-aromatic heterocycle radical. The non-aromatic heterocycle-heteroalkenyl group comprises 6 to 20 atoms, e.g. the heteroalkenyl moiety of the non-aromatic heterocycle-heteroalkenyl group is 1 to 6 carbon atoms and the non-aromatic heterocycle moiety is 3 to 14 atoms.

The term "non-aromatic heterocycle-heteroalkynyl" or "non-aromatic heterocycle-heteroalkynyl-" as used herein refers to a heteroalkynyl radical in which one of the hydrogen atoms bonded to a carbon atom, is replaced with a non-aromatic heterocycle radical. The non-aromatic heterocycle-heteroalkynyl group comprises 6 to 20 atoms, e.g. the heteroalkynyl moiety of the non-aromatic heterocycle-heteroalkynyl group is 1 to 6 carbon atoms and the non-aromatic heterocycle moiety is 3 to 14 atoms.

By way of example, carbon bonded heterocyclic rings are bonded at position 2, 3, 4, 5, or 6 of a pyridine, position 3, 4, 5, or 6 of a pyridazine, position 2, 4, 5, or 6 of a pyrimidine, position 2, 3, 5, or 6 of a pyrazine, position 2, 3, 4, or 5 of a furan, tetrahydrofuran, thiophene, pyrrole or tetrahydropyrrole, position 2, 4, or 5 of an oxazole, imidazole or thiazole, position 3, 4, or 5 of an isoxazole, pyrazole, or isothiazole, position 2 or 3 of an aziridine, position 2, 3, or 4 of an azetidine, position 2, 3, 4, 5, 6, 7, or 8 of a quinoline or position 1, 3, 4, 5, 6, 7, or 8 of an isoquinoline. Still more typically, carbon bonded heterocycles include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5-pyrimidinyl, 6-pyrimidinyl, 2-pyrazinyl, 3-pyrazinyl, 5-pyrazinyl, 6-pyrazinyl, 2-thiazolyl, 4-thiazolyl, or 5-thiazolyl. By way of example, nitrogen bonded heterocyclic rings are bonded at position 1 of an aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, 1H-indazole, position 2 of a isoindole, or isoindoline, position 4 of a morpholine, and position 9 of a carbazole, or ß-carboline. Still more typically, nitrogen bonded heterocycles include 1-aziridyl, 1-azetedyl, 1-pyrrolyl, 1-imidazolyl, 1-pyrazolyl, and 1-piperidinyl.

As used herein and unless otherwise stated, the terms "alkoxy", "cyclo-alkoxy", "aryloxy", "arylalkyloxy", "heterocycleoxy", "alkylthio", "cycloalkylthio", "arylthio", "arylalkylthio" and "heterocyclethio" refer to substituents wherein an alkyl group, respectively a cycloalkyl, aryl, arylalkyl or heterocycle (each of them such as defined herein), are attached to an oxygen atom or a sulfur atom through a single bond, such as but not limited to methoxy, ethoxy, propoxy, butoxy, thioethyl, thiomethyl, phenyloxy, benzyloxy, mercaptobenzyl and the like. The same definitions will apply for alkenyl and alkynyl radicals in stead of alkyl.

As used herein and unless otherwise stated, the term halogen means any atom selected from the group consisting of fluorine (F), chlorine (Cl), bromine (Br) and iodine (I).

The terminology regarding a chemical group "which optionally includes one or more heteroatoms, said heteroatoms being selected from the atoms consisting of O, S, and N" as used herein, refers to.a group where one or more carbon atoms are replaced by an oxygen, nitrogen or sulphur atom and thus includes, depending on the group to which is referred, heteroalkyl, heteroalkenyl, heteroalkynyl, heteroalkyl, heteroalkenyl, heteroalkynyl, cycloheteroalkyl, cycloheteroalkenyl, cycloheteroalkynyl, heteroaryl, arylheteroalkyl, heteroarylalkyl, heteroarylheteroalkyl, arylheteroalkenyl, heteroarylalkenyl, heteroarylheteroalkenyl, heteroarylheteroalkenyl, arylheteroalkynyl, heteroarylalkynyl, heteroarylheteroalkynyl, among others. This term therefore comprises, depending on the group to which is referred, as an example alkoxy, alkenyloxy, alkynyloxy, alkyl-O-alkylene, alkenyl-O-alkylene, arylalkoxy, benzyloxy, heterocycle-heteroalkyl, heterocycle-alkoxy, among others. As an example, the terminology "alkyl which optionally includes one or more heteroatoms, said heteroatoms being selected from the atoms consisting of O, S, and N" therefore refers to heteroalkyl, meaning an alkyl which comprises one or more heteroatoms in the hydrocarbon chain, whereas the heteroatoms may be positioned at the beginning of the hydrocarbon chain, in the hydrocarbon chain or at the end of the hydrocarbon chain. Examples of heteroalkyl include methoxy, methylthio, ethoxy, propoxy, $CH_3$-O-$CH_2$-, $CH_3$-S-$CH_2$-, $CH_3$-$CH_2$-O-$CH_2$-, $CH_3$-NH-, $(CH_3)_2$-N-, $(CH_3)_2$-$CH_2$-NH-$CH_2$-$CH_2$-, among many other examples. As an example, the terminology "arylalkylene which optionally includes one or more heteroatoms in the alkylene chain, said heteroatoms being selected from the atoms consisting of O, S, and N" therefore refers to arylheteroalkylene, meaning an arylalkylene which comprises one or more heteroatoms in the hydrocarbon chain, whereas the heteroatoms may be positioned at the beginning of the hydrocarbon chain, in the hydrocarbon chain or at the end of the hydrocarbon chain. "Arylheteroalkylene" thus includes aryloxy, arylalkoxy, aryl-alkyl-NH- and the like and examples are phenyloxy, benzyloxy, aryl-$CH_2$-S-$CH_2$-, aryl-$CH_2$-O-$CH_2$-, aryl-NH-$CH_2$- among many other examples. The same counts for "heteroalkenylene", "heteroalkynylene", and other terms used herein when referred to "which optionally includes one or more heteroatoms, said heteroatoms being selected from the atoms consisting of O, S, and N".

The terminology regarding a chemical group "wherein optionally two or more hydrogen atoms on a carbon atom or heteroatom of said group can be taken together to form a =O or =S" as used herein, refers to a group where two or more hydrogen atoms on a carbon atom or heteroatom of said group are taken together to form =O or =S. As an example, the terminology refers to "an alkyl wherein optionally two or more hydrogen atoms on a carbon atom or heteroatom of said alkyl can be taken together to form a =O or =S", includes among other examples $CH_3$-C(O)-$CH_2$-, $CH_3$-C(O)-, $CH_3$-C(S)-$CH_2$-, $CH_3$-$S(O)_2$-$CH_2$- and $(CH_3)_2$-$CH_2$-C(O)-$CH_2$-$CH_2$-.

The combination for a group "which optionally includes one or more heteroatoms, said heteroatoms being selected from the atoms consisting of O, S, and N" and "wherein optionally two or more hydrogen atoms on a carbon atom or heteroatom of said group can be taken together to form a =O or =S" can combine the two aspects described herein above and includes, if the group referred to is alkyl, among other examples $CH_3$-C(O)O-, $CH_3$-C(O)O-$CH_2$-, $CH_3$-NH-C(O)-, $CH_3$-C(O)-NH- $CH_3$-NH-C(O)-$CH_2$-, $CH_3$-NH-C(S)-$CH_2$-, $CH_3$-NH-C(S)-NH-$CH_2$-, $CH_3$-NH-$S(O)_2$- and $CH_3$-NH-$S(O)_2$-NH-$CH_2$-.

As used herein with respect to a substituting group, and unless otherwise stated, the terms "substituted" such as in "substituted alkyl", "substituted alkenyl", substituted alkynyl", "substituted aryl", "substituted heterocycle", "substituted arylalkyl", "substituted heterocycle-alkyl" and the like refer to the chemical structures defined herein, and wherein the said hydrocarbyl, heterohydrocarbyl group and/or the said aryl or heterocycle may be optionally substituted with one or more substituents (preferable 1, 2, 3, 4, 5 or 6), meaning that one or more hydrogen atoms are each independently replaced with a substituent. Typical substituents include, but are not limited to and in a particular embodiment said substituents are being independently selected from the group consisting of halogen, amino, hydroxyl, sulfhydryl, alkyl, alkoxy, alkenyl, alkenyloxy, alkynyl, alkynyloxy, cycloalkyl, cycloalkenyl, cycloalkynyl, heteroalkyl, heteroalkenyl, heteroalkynyl, aryl, heterocycle, arylalkyl, arylalkenyl, arylalkynyl, heterocycle-alkyl, heterocycle-alkenyl and heterocycle-alkynyl, -X, -Z, $-O^-$, -OZ, =O, -SZ, $-S^-$, =S, $-NZ_2$, $-N^+Z_3$, =NZ, =N-OZ, $-CX_3$ (e.g. trifluoromethyl), -CN, -OCN, -SCN, - N=C=O, -N=C=S, -NO, $-NO_2$, $=N_2$, $-N_3$, -NZC(O)Z, -NZC(S)Z, $-NZC(O)O^-$, -NZC(O)OZ, - NZC(S)OZ, -NZC(O)NZZ, NZC(NZ)Z, NZC(NZ)NZZ, -C(O)NZZ, -C(NZ)Z, $-S(O)_2O^-$, - $S(O)_2OZ$, $-S(O)_2Z$, $-OS(O)_2OZ$, $-OS(O)_2Z$, $-OS(O)_2O^-$, $-S(O)_2NZ$, -S(O)Z, $-OP(O)(OZ)_2$, - $P(O)(OZ)_2$, $-P(O)(O^-)_2$, $-P(O)(OZ)(O^-)$, $-P(O)(OH)_2$, -C(O)Z, -C(O)X, -C(S)Z, -C(O)OZ, - $C(O)O^-$, -C(S)OZ, -C(O)SZ, -C(S)SZ, -C(O)NZZ, -C(S)NZZ, -C(NZ)NZZ, -OC(O)Z, - OC(S)Z, $-OC(O)O^-$, -OC(O)OZ, -OC(S)OZ, wherein each X is independently a halogen selected from F, Cl, Br, or I; and each Z is independently -H, alkyl, alkenyl, alkynyl, heteroalkyl, heteroalkenyl, heteroalkynyl, aryl, heterocycle, protecting group or prodrug moiety, while two Z bonded to a nitrogen atom can be taken together with the nitrogen atom to which they are bonded to form a heterocycle. Alkyl(ene), alkenyl(ene), and alkynyl(ene) groups may also be similarly substituted. Any substituent designation that is found in more than one site in a compound of this invention shall be independently selected.

Substituents optionally are designated with or without bonds. Regardless of bond indications, if a substituent is polyvalent (based on its position in the structure referred to), then any and all possible orientations of the substituent are intended.

As used herein and unless otherwise stated, the term "solvate" includes any combination which may be formed by a derivative of this invention with a suitable inorganic solvent (e.g. hydrates) or organic solvent, such as but not limited to alcohols, ketones, esters, ethers, nitriles and the like.

## Detailed description

The present invention relates to a series of novei compounds which have been shown to possess antifungal activity, in particular against fungi of the family of the Candida, Aspergillus and/or Fusarium species. The invention therefore relates to the new compounds, methods for their preparation, pharmaceutical compositions comprising them, the use of the compounds for the preparation of a medicament, and to the compounds for use as a medicament, more in particular as antifungal medicament. The present invention also relates to methods to treat or to prevent a fungal infection.

The present invention provides novel compounds which are unsubstituted or substituted 6-substituted 2-(2-oxo-morpholin-3-yl)-acetamides and which are in particular embodiments described herein.
In a yet more particular embodiment, the present invention provides novel 6-substituted 2-(2-oxo-morpholin-3-yl)-acetamide compounds having a structure according to the formula (A'):

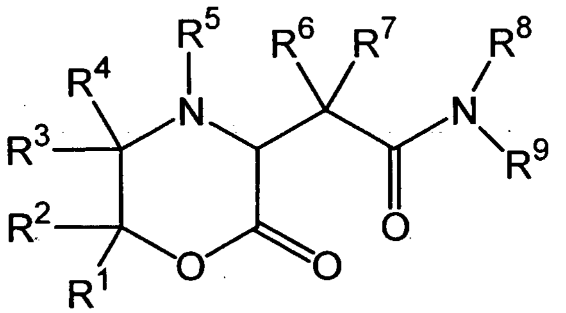

(A')

wherein,

- each $R^1$ and $R^2$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; and arylheteroalkynyl;
provided that at least one of $R^1$ and $R^2$ is not hydrogen;
- each $R^3$ and $R^4$ is independently selected from hydrogen; alkyl; alkenyl; alkynyl; heteroalkyl; heteroalkenyl; and heteroalkynyl;
- $R^5$ is independently selected from hydrogen; alkyl; alkenyl; alkynyl; heteroalkyl; heteroalkenyl; heteroalkynyl; aryl; arylalkyl; arylalkenyl; arylalkynyl; $-C(O)R^{10}$; and $-S(O)_2R^{10}$;
wherein each of said alkyl, alkenyl, alkynyl, heteroalkyl, heteroalkenyl, heteroalkynyl, aryl, arylalkyl, arylalkenyl, and arylalkynyl can be unsubstituted or substituted;
- each $R^6$ and $R^7$ is independently selected from hydrogen; alkyl; alkenyl; alkynyl; heteroalkyl; heteroalkenyl; and heteroalkynyl;
- each $R^8$ and $R^9$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocylce-alkynyl; heterocycle-heteroalkyl; heterocycle-heteroalkenyl; and heterocycle-heteroalkynyl; and wherein $R^8$ and $R^9$ can be taken together in order to form a (5-, 6-, or 7-membered) heterocycle;
provided that at least one of $R^8$ and $R^9$ is not hydrogen; and
wherein each of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocylce-alkynyl, heterocycle-heteroalkyl, heterocycle-heteroalkenyl, and heterocycle-heteroalkynyl, can be unsubstituted or substituted;
- each $R^{10}$ is independently selected from hydrogen; alkyl; alkenyl; alkynyl; heteroalkyl; heteroalkenyl; heteroalkynyl; aryl; arylalkyl; arylalkenyl; and arylalkynyl;
wherein each of said alkyl, alkenyl, alkynyl, heteroalkyl, heteroalkenyl, heteroalkynyl, aryl, arylalkyl, arylalkenyl, and arylalkynyl can be unsubstituted or substituted.

and stereo-isomers, tautomers, solvates, or pharmaceutically acceptable salts thereof.

The present invention also relates to the different embodiments described herein of this aspect and to other aspects such as the compounds for use as a medicament, a method for the preparation of the compounds, a pharmaceutical composition of the compounds and methods of treatment or prevention of a disease by using the compounds.

The compounds of the invention are employed for the treatment or prophylaxis of fungal infections, more particularly Candida, Aspergillus or Fusarium species infections.
When using one or more compounds according to the formulae and embodiments of the application as defined herein:

- the active ingredients of the compound(s) may be administered to the mammal (including a human) to be treated by any means well known in the art, i.e. orally, intranasally, subcutaneously, intramuscularly, intradermally, intravenously, intra-arterially, parenterally or by catheterization.
- the therapeutically effective amount of the preparation of the compound(s), especially for the treatment of fungal infections in humans and other mammals, corresponds to an amount which ensures a plasma level of between 1μg/ml and 100 mg/ml, optionally of 10 mg/ml. Depending upon the pathologic condition to be treated and the patient's condition, the effective amount may be divided into several sub-units per day or may be administered at more than one day intervals.

The present invention further relates to compounds for preventing or treating a fungal infection in a subject or patient by administering to the patient in need thereof a therapeutically effective amount of the compounds of the present invention. The therapeutically effective amount of the preparation of the compound(s), especially for the treatment of fungal infections in humans and other mammals, preferably is a fungicidal amount. More preferably, it is a fungal replication inhibiting amount of the compounds of the formulae as defined herein. Depending upon the pathologic condition to be treated and the patient's condition, the effective amount may be divided into several sub-units per day or may be administered at more than one day intervals.
As is conventional in the art, the evaluation of a synergistic effect in a drug combination may be made by analyzing the quantification of the interactions between individual drugs, using the median effect principle described by Chou et al. in Adv. Enzyme Reg. (1984) 22:27. Briefly, this principle states that interactions (synergism, additivity, antagonism) between two drugs can be quantified using the combination index (hereinafter referred as CI) defined by the following equation:

$$CI_x = \frac{ED_x^{1c}}{ED_x^{1a}} + \frac{ED_x^{2c}}{ED_x^{2a}}$$

wherein $ED_x$ is the dose of the first or respectively second drug used alone (1a, 2a), or in combination with the second or respectively first drug (1c, 2c), which is needed to produce a given effect. The said first and second drug have synergistic or additive or antagonistic effects depending upon CI < 1, CI = 1, or CI > 1, respectively. Synergistic activity of the pharmaceutical compositions or combined preparations of this invention against fungal infection may also be readily determined by means of one or more tests such as, but not limited to, the isobologram method, as previously described by Elion et al. in J. Biol. Chem. (1954) 208:477-488 and by Baba et al. in Antimicrob. Agents Chemother. (1984) 25:515-517, using $EC_{50}$ for calculating the fractional inhibitory concentration (hereinafter referred as FIC). When the minimum FIC index corresponding to the FIC of combined compounds (e.g., $FIC_x + FIC_y$) is equal to 1.0, the combination is said to be additive; when it is between 1.0 and 0.5, the combination is defined as subsynergistic, and when it is lower than 0.5, the combination is defined as synergistic. When the minimum FIC index is between 1.0 and 2.0, the combination is defined as subantagonistic and, when it is higher than 2.0, the combination is defined as antagonistic.
This principle may be applied to a combination of different antifungal drugs of the invention or to a combination of the antifungal drugs of the invention with other drugs that exhibit antifungal activity, or with drugs that exhibit other medicinal or health promoting activity.
The invention thus relates to a pharmaceutical composition or combined preparation having synergistic effects against a fungal infection and containing:

either (a) a combination of two or more of the compounds of the present invention, and optionally one or more pharmaceutical excipients or pharmaceutically acceptable carriers, for simultaneous, separate or sequential use in the treatment or prevention of a fungal infection;
or (b) one or more antifungal agents, and at least one of the compounds of the present invention, and optionally one or more pharmaceutical excipients or pharmaceutically acceptable carriers, for simultaneous, separate or sequential use in the treatment or prevention of a fungal infection.

The pharmaceutical composition or combined preparation with synergistic activity against fungal infection according to

this invention may contain compounds of the present invention, compounds according to the formulae of the application, over a broad content range depending on the contemplated use and the expected effect of the preparation. Generally, the content of the compounds of the present invention of the combined preparation is within the range of 0.1 to 99.9% by weight, preferably from 1 to 99% by weight, more preferably from 5 to 95% by weight.

In a particular embodiment, the compounds of the invention can be used for the treatment or prevention of infection by Candida species (e.g. C. albicans, C. glabrata, C. krusei), Aspergillus species (e.g. A. flavus, A. fumigatus, A. clavatus), Fusarium species (e.g. F. oxysporum, F. culmorum) or other fungi. More in particular, the compounds of present invention can be used as a medicament for treating or preventing fungal infections in a subject in need thereof.

The present invention further provides veterinary compositions comprising at least one active ingredient as above defined together with a veterinary carrier therefore. Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

The present invention further provides agricultural compositions comprising at least one active ingredient as above defined together with a suitable carrier therefore.

More generally, the invention relates to the compounds according to the formulae of the application being useful as agents having biological activity (particularly antifungal activity) or as diagnostic agents. Any of the uses mentioned with respect to the present invention may be restricted to a non-medical use, a non-therapeutic use, a non-diagnostic use, or exclusively an in vitro use, or a use related to cells remote from an animal. For example, as described herein, the compounds of present invention can be used to eradicate or reduce or prevent fungal infections on plants or materials, or can be used to eradicate or reduce or prevent biofilm formation, e.g. a biofilm wherein fungi are attached or prevail or contribute to the biofilm formation.

The compounds of the invention optionally are bonded covalently to an insoluble matrix and used for affinity chromatography (separations, depending on the nature of the groups of the compounds, for example compounds with aryl are useful in hydrophobic affinity separations.

Those of skill in the art will also recognize that the compounds of the invention may exist in many different protonation states, depending on, among other things, the pH of their environment. While the structural formulae provided herein depict the compounds in only one of several possible protonation states, it will be understood that these structures are illustrative only, and that the invention is not limited to any particular protonation state, any and all protonated forms of the compounds are intended to fall within the scope of the invention.

The term "pharmaceutically acceptable salts" as used herein means the therapeutically active non-toxic salt forms which the compounds according to the formulae of the application are able to form. Therefore, the compounds of this invention optionally comprise salts of the compounds herein, especially pharmaceutically acceptable non-toxic salts containing, for example, $Na^+$, $Li^+$, $K^+$, $Ca^{+2}$ and $Mg^{+2}$. Such salts may include those derived by combination of appropriate cations such as alkali and alkaline earth metal ions or ammonium and quaternary amino ions with an acid anion moiety, typically a carboxylic acid. The compounds of the invention may bear multiple positive or negative charges. The net charge of the compounds of the invention may be either positive or negative. Any associated counterions are typically dictated by the synthesis and/or isolation methods by which the compounds are obtained. Typical counterions include, but are not limited to ammonium, sodium, potassium, lithium, halides, acetate, trifluoroacetate, etc., and mixtures thereof. It will be understood that the identity of any associated counter ion is not a critical feature of the invention, and that the invention encompasses the compounds in association with any type of counter ion. Moreover, as the compounds can exist in a variety of different forms, the invention is intended to encompass not only forms of the compounds that are in association with counterions (e.g., dry salts), but also forms that are not in association with counterions (e.g., aqueous or organic solutions). Metal salts typically are prepared by reacting the metal hydroxide with a compound of this invention. Examples of metal salts which are prepared in this way are salts containing $Li^+$, $Na^+$, and $K^+$. A less soluble metal salt can be precipitated from the solution of a more soluble salt by addition of the suitable metal compound. In addition, salts may be formed from acid addition of certain organic and inorganic acids to basic centers, typically amines, or to acidic groups. Examples of such appropriate acids include, for instance, inorganic acids such as hydrohalic acids, e.g. hydrochloric or hydrobromic acid, sulfuric acid, nitric acid, phosphoric acid and the like; or organic acids such as, for example, acetic, propanoic, hydroxylacetic, 2-hydroxylpropanoic, 2-oxopropanoic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclohexanesulfamic, salicylic (i.e. 2-hydroxylbenzoic), p-aminosalicylic and the like. Furthermore, this term also includes the solvates which the compounds according to the formulae of the application as well as their salts are able to form, such as for example hydrates, alcoholates and the like. Finally, it is to be understood that the compositions herein comprise compounds of the invention in their unionized, as well as zwitterionic form, and combinations with stoichiometric amounts of water as in hydrates.

Also included within the scope of this invention are the salts of the parental compounds with one or more amino acids, especially the naturally-occurring amino acids found as protein components. The amino acid typically is one

bearing a side chain with a basic or acidic group, e.g., lysine, arginine or glutamic acid, or a neutral group such as glycine, serine, threonine, alanine, isoleucine, or leucine.

The compounds of the invention also include physiologically acceptable salts thereof. Examples of physiologically acceptable salts of the compounds of the invention include salts derived from an appropriate base, such as an alkali metal (for example, sodium), an alkaline earth (for example, magnesium), ammonium and $NX_4^+$ (wherein X is $C_1$-$C_4$ alkyl). Physiologically acceptable salts of an hydrogen atom or an amino group include salts of organic carboxylic acids such as acetic, benzoic, lactic, fumaric, tartaric, maleic, malonic, malic, isethionic, lactobionic and succinic acids; organic sulfonic acids, such as methanesulfonic, ethanesulfonic, benzenesulfonic and p-toluenesulfonic acids; and inorganic acids, such as hydrochloric, sulfuric, phosphoric and sulfamic acids. Physiologically acceptable salts of a compound containing a hydroxyl group include the anion of said compound in combination with a suitable cation such as $Na^+$ and $NX_4^+$ (wherein X typically is independently selected from H or a $C_1$-$C_4$ alkyl group). However, salts of acids or bases which are not physiologically acceptable may also find use, for example, in the preparation or purification of a physiologically acceptable compound. All salts, whether or not derived form a physiologically acceptable acid or base, are within the scope of the present invention.

The compounds of the present invention can have different isomeric forms or can be a mixture of isomers.

As used herein and unless otherwise stated, the term "enantiomer" means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

The term "isomers" as used herein means all possible isomeric forms, including tautomeric and sterochemical forms ("stereo-isomers") and including positional isomers, which the compounds according to the formulae of the application may possess. In a particular embodiment, the term "isomers" excludes positional isomers. Typically, the structures shown herein exemplify only one tautomeric or resonance form of the compounds, but the corresponding alternative configurations are contemplated as well. Unless otherwise stated, the chemical designation of compounds denotes the mixture of all possible stereochemically isomeric forms, including stereoisomers and tautomers, said mixtures containing all diastereomers and enantiorners (since the compounds according to the formulae of the application may have at least one chiral center) of the basic molecular structure, as well as the stereochemically pure or enriched compounds. More particularly, stereogenic centers may have either the R- or S-configuration, and multiple bonds may have either cis- or trans-configuration.

Pure isomeric forms of the said compounds are defined as isomers substantially free of other enantiomeric or diastereomeric forms of the same basic molecular structure. In particular, the term "stereoisomerically pure" or "chirally pure" relates to compounds having a stereoisomeric excess of at least about 80% (i.e. at least 90% of one isomer and at most 10% of the other possible isomers), preferably at least 90%, more preferably at least 94% and most preferably at least 97%. The terms "enantiomerically pure" and "diastereomerically pure" should be understood in a similar way, having regard to the enantiomeric excess, respectively the diastereomeric excess, of the mixture in question.

Separation of stereoisomers is accomplished by standard methods known to those skilled in the art. One enantiomer of a compound of the invention can be separated substantially free of its opposing enantiomer by a method such as formation of diastereomers using optically active resolving agents ("Stereochemistry of Carbon Compounds," (1962) by E. L. Eliel, McGraw Hill; Lochmuller, C. H., (1975) J. Chromatogr., 113:(3) 283-302). Separation of isomers in a mixture can be accomplished by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure enantiomers, or (3) enantiomers can be separated directly under chiral conditions. Under method (1), diastereomeric salts can be formed by reaction of enantiomerically pure chiral bases such as brucine, quinine, ephedrine, strychnine, a-methyl-b-phenylethylamine (amphetamine), and the like with asymmetric compounds bearing acidic functionality, such as carboxylic acid and sulfonic acid. The diastereomeric salts may be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts. Alternatively, by method (2), the substrate to be resolved may be reacted with one enantiomer of a chiral compound to form a diastereomeric pair (Eliel, E. and Wilen, S. (1994) Stereochemistry of Organic Compounds, John Wiley & Sons, Inc., p. 322). Diastereomeric compounds can be formed by reacting asymmetric compounds with enantiomerically pure chiral derivatizing reagents, such as menthyl derivatives, followed by separation of the diastereomers and hydrolysis to yield the free, enantiomerically enriched compounds of the invention. A method of determining optical purity involves making chiral esters, such as a menthyl ester or Mosher ester, a-methoxy-a-(trifluoromethyl)phenyl acetate (Jacob III. (1982) J. Org. Chem. 47:4165), of the racemic mixture, and analyzing the NMR spectrum for the presence of the two atropisomeric diastereomers. Stable diastereomers can be separated and isolated by normal- and reverse-phase chromatography following methods for separation of atropisomeric naphthyl-isoquinolines (Hoye, T., WO 96/15111).Under method (3), a racemic mixture of two asymmetric enantiomers is separated by chromatography using a chiral stationary phase.

Suitable chiral stationary phases are, for example, polysaccharides, in particular cellulose or amylose derivatives. Commercially available polysaccharide based chiral stationary phases are ChiralCelTM CA, OA, OB5, OC5, OD, OF, OG, OJ and OK, and ChiralpakTM AD, AS, OP(+) and OT(+). Appropriate eluents or mobile phases for use in combination with said polysaccharide chiral stationary phases are hexane and the like, modified with an alcohol such as ethanol, isopropanol and the like. ("Chiral Liquid Chromatography" (1989) W. J. Lough, Ed. Chapman and Hall, New York; Okamoto, (1990) "Optical resolution of dihydropyridine enantiomers by High-performance liquid chromatography using phenylcarbamates of polysaccharides as a chiral stationary phase", J. of Chromatogr. 513:375-378).
The terms cis and trans are used herein in accordance with Chemical Abstracts nomenclature and include reference to the position of the substituents on a ring moiety. The absolute stereochemical configuration of the compounds according to the formulae of the application may easily be determined by those skilled in the art while using well-known methods such as, for example, X-ray diffraction or NMR.
Tautomers are organic compounds that are interconvertible by a chemical reaction called tautomerization. As most commonly encountered, this reaction results in the formal migration of a hydrogen atom or proton, accompanied by a switch of a single bond and adjacent double bond. In solutions where tautomerization is possible, a chemical equilibrium of the tautomers will be reached. The exact ratio of the tautomers depends on everal factors, including temperature, solvent, and pH.

The compounds of the invention may be formulated with conventional carriers and excipients, which will be selected in accordance with ordinary practice. Tablets will contain excipients, glidants, fillers, binders and the like. Aqueous formulations are prepared in sterile form, and when intended for delivery by other than oral administration generally will be isotonic. Formulations optionally contain excipients such as those set forth in the "Handbook of Pharmaceutical Excipients" (1986) and include ascorbic acid and other antioxidants, chelating agents such as EDTA, carbohydrates such as dextrin, hydroxylalkylcellulose, hydroxylalkylmethylcellulose, stearic acid and the like.
Subsequently, the term "pharmaceutically acceptable carrier" as used herein means any material or substance with which the active ingredient is formulated in order to facilitate its application or dissemination to the locus to be treated, for instance by dissolving, dispersing or diffusing the said composition, and/or to facilitate its storage, transport or handling without impairing its effectiveness. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, suspensions, ointments, creams, tablets, pellets or powders. Suitable pharmaceutical carriers for use in the said pharmaceutical compositions and their formulation are well known to those skilled in the art, and there is no particular restriction to their selection within the present invention. They may also include additives such as wetting agents, dispersing agents, stickers, adhesives, emulsifying agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, coating and/or grinding the active ingredients, in a one-step or multi-steps procedure, with the selected carrier material and, where appropriate, the other additives such as surface-active agents may also be prepared by inicronisation, for instance in view to obtain them in the form of microspheres usually having a diameter of about 1 to 10 gm, namely for the manufacture of microcapsules for controlled or sustained release of the active ingredients.
Suitable surface-active agents, also known as emulgent or emulsifier, to be used in the pharmaceutical compositions of the present invention are non-ionic, cationic and/or anionic materials having good emulsifying, dispersing and/or wetting properties. Suitable anionic surfactants include both water-soluble soaps and water-soluble synthetic surface-active agents. Suitable soaps are alkaline or alkaline-earth metal salts, unsubstituted or substituted ammonium salts of higher fatty acids ($C_{10}$-$C_{22}$), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanylphosphatidyl-choline, dipalmitoylphoshatidyl -choline and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups. Such compounds usually contain from 1 to 5 ethyleneglycol units per propyleneglycol unit. Representative examples of non-ionic surfactants are nonylphenol -polyethoxyethanol, castor oil polyglycolic ethers, polypropylene/polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, particularly halides, having 4 hydrocarbon radicals optionally substituted with halo, phenyl, substituted phenyl or hydroxyl; for instance quaternary ammonium salts containing as N-substituent at least one $C_8$-$C_{22}$ alkyl radical (e.g. cetyl, lauryl, palmityl, myristyl, oleyl and the like) and, as further substituents, unsubstituted or halogenated lower alkyl, benzyl and/or hydroxyl-lower alkyl radicals.

A more detailed description of surface-active agents suitable for this purpose may be found for instance in "McCutcheon's Detergents and Emulsifiers Annual" (MC Publishing Crop., Ridgewood, New Jersey, 1981), "Tensid-Taschenbucw', 2 d ed. (Hanser Verlag, Vienna, 1981) and "Encyclopaedia of Surfactants, (Chemical Publishing Co., New York, 1981).

Compounds of the invention and their physiologically acceptable salts (hereafter collectively referred to as the active ingredients) and pharmaceutical compositions thereof may be administered by any route appropriate to the condition to be treated, suitable routes including oral, rectal, nasal, topical (including ocular, buccal and sublingual), vaginal and parenteral (including subcutaneous, intramuscular, intravenous, intradermal, intrathecal and epidural). The preferred route of administration may vary with for example the condition of the recipient.

While it is possible for the active ingredients to be administered alone it is preferable to present them as pharmaceutical formulations or compositions. The formulations, both for veterinary and for human use, of the present invention comprise at least one active ingredient, as above described, together with one or more pharmaceutically acceptable carriers therefore and optionally other therapeutic ingredients. The carrier(s) optimally are "acceptable" in the sense of being compatible with the other ingredients of the formulation and not deleterious to the recipient thereof. The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered compound moistened with an inert liquid diluent. The tablets may optionally be coated or scored and may be formulated so as to provide slow or controlled release of the active ingredient therein. For infections of the eye or other external tissues e.g. mouth and skin, the formulations are optionally applied as a topical ointment or cream containing the active ingredient(s) in an amount of, for example, 0.075 to 20% w/w (including active ingredient(s) in a range between 0.1% and 20% in increments of 0.1% w/w such as 0.6% w/w, 0.7% w/w, etc), preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base. If desired, the aqueous phase of the cream base may include, for example, at least 30% w/w of a polyhydric alcohol, i.e. an alcohol having two or more hydroxyll groups such as propylene glycol, butane 1,3-diol, mannitol, sorbitol, glycerol and polyethylene glycol (including PEG400) and mixtures thereof. The topical formulations may desirably include a compound which enhances absorption or penetration of the active ingredient through the skin or other affected areas. Examples of such dermal penetration enhancers include dimethylsulfoxide and related analogs.

The oily phase of the emulsions of this invention may be constituted from known ingredients in a known manner. While the phase may comprise merely an emulsifier (otherwise known as an emulgent), it desirably comprises a mixture of at

least one emulsifier with a fat or an oil or with both a fat and an oil. Optionally, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations. The choice of suitable oils or fats for the formulation is based on achieving the desired cosmetic properties, since the solubility of the active compound in most oils likely to be used in pharmaceutical emulsion formulations is very low. Thus the cream should optionally be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used. Formulations suitable for topical administration to the eye also include eye drops wherein the active ingredient is dissolved or suspended in a suitable carrier, especially an aqueous solvent for the active ingredient. The active ingredient is optionally present in such formulations in a concentration of 0.5 to 20%, advantageously 0.5 to 10% particularly about 1.5% w/w. Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate. Formulations suitable for nasal administration wherein the carrier is a solid include a coarse powder having a particle size for example in the range 20 to 500 microns (including particle sizes in a range between 20 and 500 microns in increments of 5 microns such as 30 microns, 35 microns, etc), which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient. Formulations suitable for aerosol administration may be prepared according to conventional methods and may be delivered with other therapeutic agents.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavoring agents.

Compounds of the invention can be used to provide controlled release pharmaceutical formulations containing as active ingredient one or more compounds of the invention ("controlled release formulations") in which the release of the active ingredient can be controlled and regulated to allow less frequency dosing or to improve the pharmacokinetic or toxicity profile of a given invention compound. Controlled release formulations adapted for oral administration in which discrete units comprising one or more compounds of the invention can be prepared according to conventional methods. Additional ingredients may be included in order to control the duration of action of the active ingredient in the composition. Control release compositions may thus be achieved by selecting appropriate polymer carriers such as for example polyesters, polyamino acids, polyvinyl pyrrolidone, ethylene-vinyl acetate copolymers, methyl cellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxylmethylcellulose, polyethyl methacrylate and the other above-described polymers. Such methods include colloid drug delivery systems like liposomes, microspheres, microemulsions, nanoparticles, nanocapsules and so on. Depending on the route of administration, the pharmaceutical composition may require protective coatings. Pharmaceutical forms suitable for injectionable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol and the like and mixtures thereof.

In view of the fact that, when several active ingredients are used in combination, they do not necessarily bring

out their joint therapeutic effect directly at the same time in the mammal to be treated, the corresponding composition may also be in the form of a medical kit or package containing the two ingredients in separate but adjacent repositories or compartments. In the latter context, each active ingredient may therefore be formulated in a way suitable for an administration route different from that of the other ingredient, e.g. one of them may be in the form of an oral or parenteral formulation whereas the other is in the form of an ampoule for intravenous injection or an aerosol.

The compounds of the invention according to the formulae of the application can be prepared while using a series of chemical reactions known to those skilled in the art, altogether making up the process for preparing said compounds and exemplified further. The processes described further are only meant as examples and by no means are meant to limit the scope of the present invention.

The compounds of the present invention can be prepared according to the following general procedures depicted hereunder:

Scheme 1:

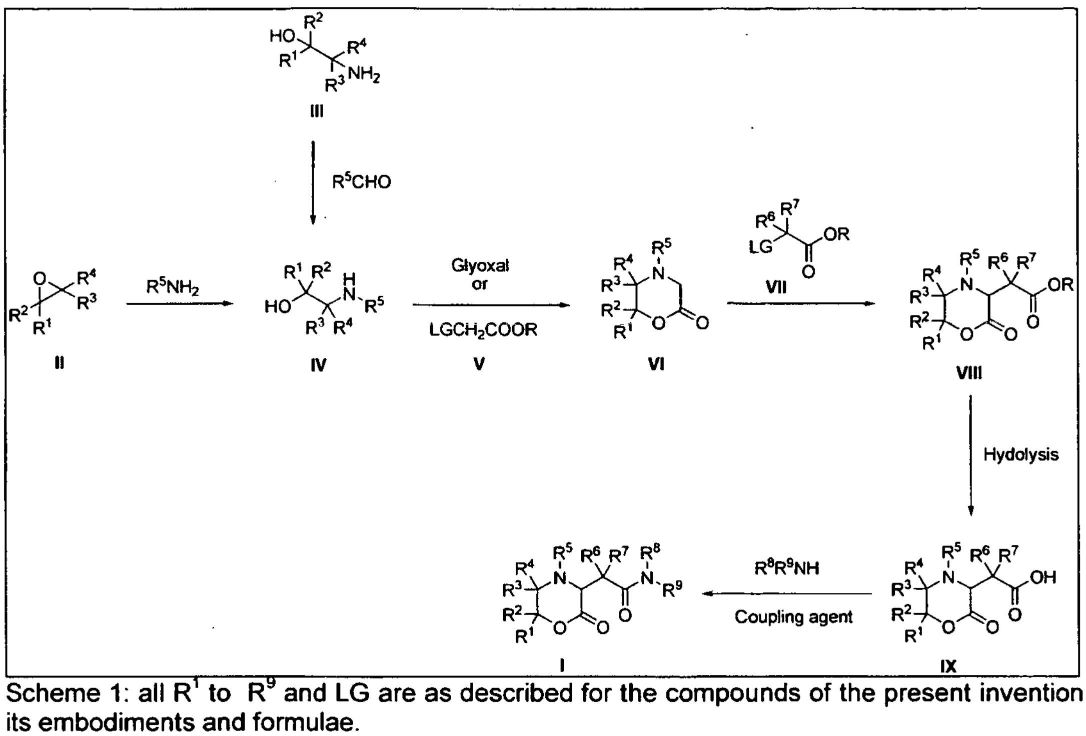


**Scheme 1: all $R^1$ to $R^9$ and LG are as described for the compounds of the present invention and its embodiments and formulae.**

It should be noted that this scheme 1 is preferably used to prepare compounds wherein $R^5NH_2$ is a primary amine selected from alkyl-$NH_2$, alkenyl-$NH_2$, alkynyl-$NH_2$, aryl-$NH_2$, arylalkyl-$NH_2$, arylalkenyl-$NH_2$, arylalkynyl-$NH_2$, heteroalkyl-$NH_2$, heteroalkenyl-$NH_2$, heteroalkynyl-$NH_2$.

Condensation of epoxides of general formula II (commercially available or synthesized by procedures known to the skilled in the art or as set forth in the examples below) with primary amines using standard procedures known to the skilled in the art furnishes the amino-alcohol intermediates of formula IV. Alternatively, intermediates IV may also be obtained from intermediates of general formula III (commercially available or synthesized by procedures known to the skilled in the art or as set forth in the examples below) following standard reductive amination reactions. Condensation of Intermediates IV with glyoxal or a reagent of formula V, wherein R is an ester protecting group (e.g., methyl, ethyl, tert-butyl, benzyl and the like) and LG is a leaving group, following procedures known to the skilled in the art or as set forth in the examples below provides intermediates of formula VI. The intermediates VI can then be alkylated with intermediates of formula VII, wherein R is an ester protecting group (e.g., methyl, ethyl, tert-butyl, benzyl and the like) and LG is a leaving group, in the presence of a strong base (e.g., LiHMDS, KHMDS and the like) in a polar aprotic solvent (e.g., THF, DMF and the like) at a temperature raising from -80°C to 0°C, to provide the desired intermediates of formula VIII. The ester protecting group is then hydrolyzed following standard procedures known to the skilled in the art or as set forth in the examples below to furnish the free carboxylic acid intermediates of formula IX which are converted into the desired compounds of formula I using standard amidation reactions known to the skilled in the art or as set forth in the examples below.

Alternatively, compounds of the formulae herein can also be prepared as outlined in Scheme 2 below.

Scheme 2:

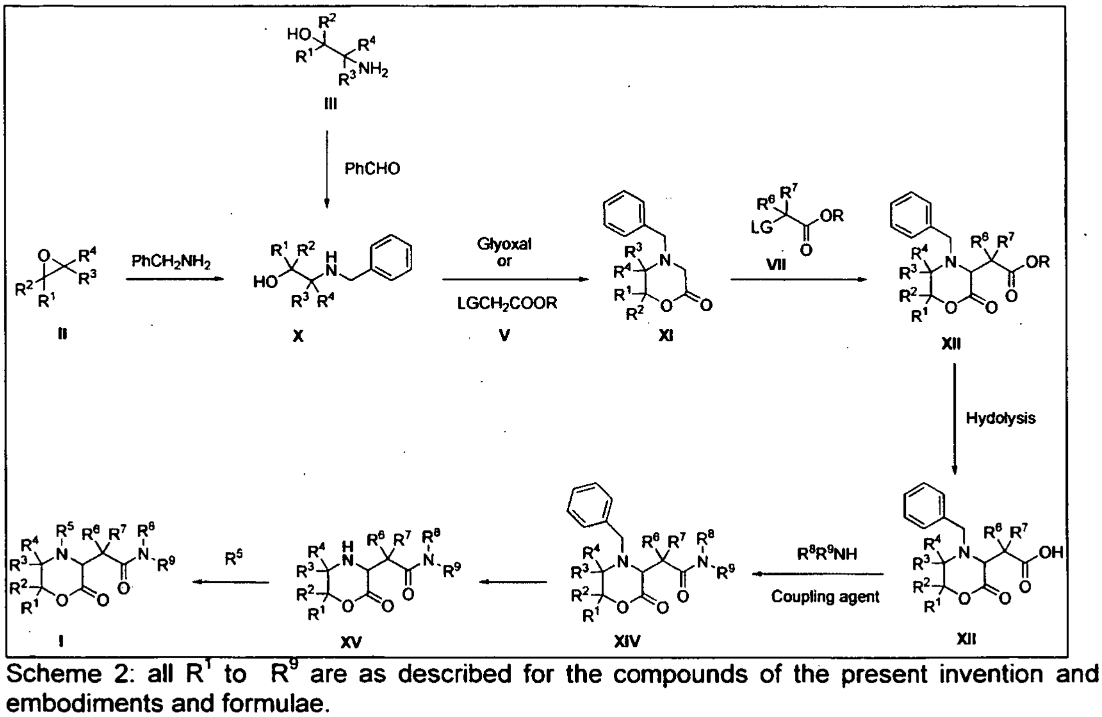


Scheme 2: all $R^1$ to $R^9$ are as described for the compounds of the present invention and its embodiments and formulae.

Condensation of epoxides of general formula II (commercially available or synthesized by procedures known to the skilled in the art or as set forth in the examples below) with benzylamine using standard procedures known to the skilled in the art furnishes the amino-alcohol intermediates of formula X. Alternatively, intermediates X may also be obtained by a condensation of intermediates of general formula III (commercially available or synthesized by procedures known to the skilled in the art or as set forth in the examples below) with benzaldehyde following a standard reductive amination reaction. Condensation of Intermediates X with glyoxal or a reagent of formula V, wherein R is an ester protecting group (e.g., methyl, ethyl, tert-butyl, benzyl and the like) and LG is a leaving group, following procedures known to the skilled in the art or as set forth in the examples below provides intermediates of formula XI. The intermediates XI can then be alkylated with intermediates of formula VII, wherein R is an ester protecting group (e.g., methyl, ethyl, tert-butyl, benzyl and the like) and LG is a leaving group, in the presence of a strong base (e.g., LiHMDS, KHMDS and the like) in a polar aprotic solvent (e.g., THF, DMF and the like) at a temperature raising from -80°C to 0°C, to provide the desired intermediates of formula XII. The ester protecting group is then hydrolyzed following standard procedures known to the skilled in the art or as set forth in the examples below to furnish the free carboxylic acid intermediates of formula XIII which are converted into the desired intermediates of formula XIV using standard amidation reactions known to the skilled in the art or as set forth in the examples below. The benzyl protecting group is then removed using an hydrogenation reaction to provide the key intermediates of formula XV which can be reacted with a suitable $R^5$ precursor (e.g., alkyl halide, acyl halide, ketone, aldehyde, sulfonyl chlorides among others) by procedures known to the skilled in the art or as set forth in examples below, to provide the desired compounds of formula I.
All other compounds not explicitly mentioned in this general description can be prepared by using the methods as described herein and further combined with the knowledge of a person skilled in the art.

**Examples**

The following examples are provided for the purpose of illustrating the present invention and should in no way be interpreted as limiting the scope thereof.

Table 1: Structures of example compounds of the invention and their respective codes.

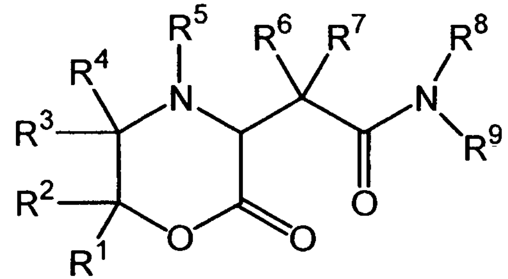

| STRUCTURE | Name | Code | $R^1$ | $R^2$ | $R^3$ | $R^4$ | $R^5$ | $R^6$ | $R^7$ | $R^8$ | $R^9$ |
|---|---|---|---|---|---|---|---|---|---|---|---|
| 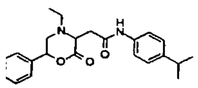 | 2-(4-ethyt-2-oxo-6-phenylmorpholin-3-yl)-N-(4-isopropylphenyl)acetamide | CPD-01 | Ph | H | H | H | Et | H | H | H | 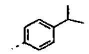 |
| 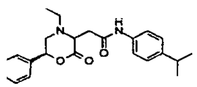 | 2-((6S)-4-ethyl-2-oxo-6-phenylmorpholin-3-yl)-N-(4-isopropylphenyl)acetamide | CPD-02 | (S)-Ph | H | H | H | Et | H | H | H | 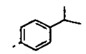 |
| 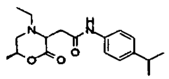 | 2-((6S)-4-ethyl-6-methyl-2-oxomorpholin-3-yl)-N-(4-isopropylphenyl)acetamide | CPD-03 | (S)-Me | H | H | H | Et | H | H | H | 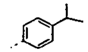 |
| 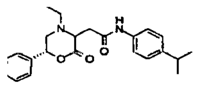 | 2-((6R)-4-ethyl-2-oxo-6-phenylmorpholin-3-yl)-N-(4-isopropylphenyl)acetamide | CPD-04 | (R)-Ph | H | H | H | Et | H | H | H | 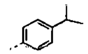 |
| 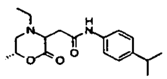 | 2-((6R)-4-ethyl-6-methyl-2-oxomorpholin-3-yl)-N-(4-isopropylphenyl)acetamide | CPD-05 | (R)-Me | H | H | H | Et | H | H | H | 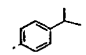 |
| 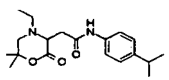 | 2-(4-ethyl-6,6-dimethyl-2-oxomorpholin-3-yl)-N-(4-isopropylphenyl)acetamide | CPD-06 | Me | Me | H | H | Et | H | H | H | 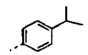 |
| 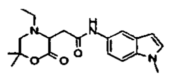 | 2-(4-ethyl-6,6-dimethyl-2-oxomorpholin-3-yl)-N-(1-methyl-1H-indol-5-yl)acetamide | CPD-07 | Me | Me | H | H | Et | H | H | H | 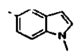 |
| 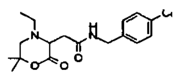 | N-(4-1chlorobenzyl)-2-(4-ethyl-6,8-dimethyl-2-oxomorpholin-3-yl)acetamide | CPD-08 | Me | Me | H | H | Et | H | H | H | 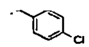 |

(continued)

| STRUCTURE | Name | Code | $R^1$ | $R^2$ | $R^3$ | $R^4$ | $R^5$ | $R^6$ | $R^7$ | $R^8$ | $R^9$ |
|---|---|---|---|---|---|---|---|---|---|---|---|
| 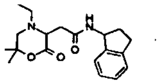 | N-(2,3-dihydro-1H-inden-1-yl)-2-(4-ethyl-6,6-dimethyl-2-oxomorpholin-3-yl)acetamide | CPD-09 | Me | Me | H | H | Et | H | H | H | 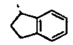 |
| 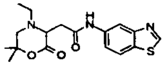 | N-(benzo[d]thiazol-5-yl)-2-(4-ethyl-6,6-dimethyl-2-oxomorpholin-3-yl)acetamide | CPD-10 | Me | Me | H | H | Et | H | H | H | 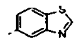 |
| 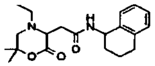 | 2-(4-ethyl-6,6-dimethyl-2-oxomorpholin-3-yl)-N-(1,2,3,4-tetrahydronaphthalen-1-yl)acetamide | CPD-11 | Me | Me | H | H | Et | H | H | H | 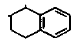 |
| 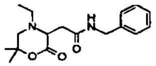 | N-benzyl-2-(4-ethyl-6,6-dimethyl-2-oxomorpholin-3-yl)acetamide | CPD-12 | Me | Me | H | H | Et | H | H | H | 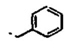 |
| 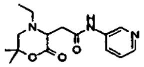 | 2-(4-ethyl-6,6-dimethyl-2-oxomorpholin-3-yl)-N-(pyridin-3-yl)acetamide | CPD-13 | Me | Me | H | H | Et | H | H | H | 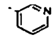 |
| 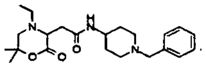 | N-(1-benzylpiperidin-4-yl)-2-(4-ethyl-6,6-dimethyl-2-oxomorpholin-3-yl)acetamide | CPD-14 | Me | Me | H | H | Et | H | H | H | 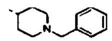 |
| 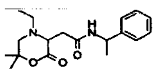 | 2-(4-ethyl-6,6-dimethyl-2-oxomorpholin-3-yl)-N-(1-phenylethyl)acetamide | CPD-15 | Me | Me | H | H | Et | H | H | H | 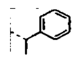 |
| 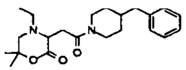 | 3-(2-(4-benzylpiperidin-1-yl)-2-oxoethyl)-4-ethyl-6,6-dimethylmorpholin-2-one | CPD-16 | Me | Me | H | H | Et | H | H | 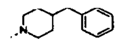 | |
| 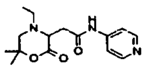 | 2-(4-ethyl-6,6-dimethyl-2-oxomorpholin-3-yl)-N-(pyridin-4-yl)acetamide | CPD-17 | Me | Me | H | H | Et | H | H | H | 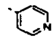 |
| 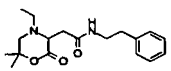 | 2-(4-ethyl-6,6-dimethyl-2-oxomorpholin-3-yl)-N-phenethylacetamide | CPD-18 | Me | Me | H | H | Et | H | H | H | 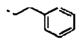 |
| 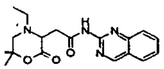 | 2-(4-ethyl-6,6-dimethyl-2-oxomorpholin-3-yl)-N-(quinazolin-2-yl)acetamide | CPD-19 | Me | Me | H | H | Et | H | H | H | 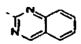 |
| 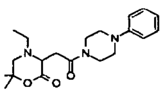 | 4-ethyl-6,6-dimethyl-3-(2-ozo-2-(4-phenylpiperazin-1-yl)ethyl)morpholin-2-one | CPD-20 | Me | Me | H | H | Et | H | H | 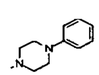 | |

(continued)

| STRUCTURE | Name | Code | $R^1$ | $R^2$ | $R^3$ | $R^4$ | $R^5$ | $R^6$ | $R^7$ | $R^8$ | $R^9$ |
|---|---|---|---|---|---|---|---|---|---|---|---|
| 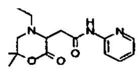 | 2-(4-ethyl-6,6-dimethyl-2-oxomorpholin-3-yl-N-(pyridin-2-yl)acetamide | CPD-21 | Me | Me | H | H | Et | H | H | H | 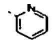 |
| 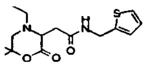 | 2-(4-ethyl-6,6-dimethyl-2-oxomorpholin-3-yl)-N-(thiophen-2-ylmethyl)acetamide | CPD-22 | Me | Me | H | H | Et | H | H | H | 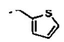 |
| 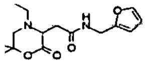 | 2-(4-ethyl-6,6-dimethyl-2-oxomorpholin-3-yl)-N-(furan-2-ylmethyl)acetamide | CPD-23 | Me | Me | H | H | Et | H | H | H | 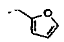 |
| 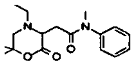 | 2-(4-ethyl-6,6-dimethyl-2-oxomorpholin-3-yl)-N-methyl-N-phenylacetamide | CPD-24 | Me | Me | H | H | Et | H | H | Me | 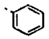 |
| 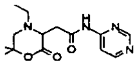 | 2-(4-ethyl-6,6-dimethyl-2-oxomorpholin-3-yl)-N-(pyrimidin 4-yl)acetamide | CPD-25 | Me | Me | H | H | Et | H | H | H | 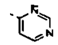 |
| 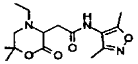 | N-(3,5-dimethylisoxazol-4-yl)-2-(4-ethyl-6,6-dimethyl-2-oxomorpholin-3-yl)acetamide | CPD-26 | Me | Me | H | H | Et | H | H | H | 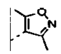 |
| 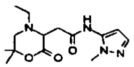 | 2-(4-ethyl-6,6-dimethyl-2-oxomorpholin-3-yl)-N-(1-methyl-1H-pyrazol-5-yl)acetamide | CPD-27 | Me | Me | H | H | Et | H | H | H | 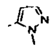 |
| 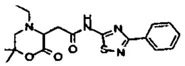 | 2-(4-ethyl-6,6-dimethyl-2-oxomorpholin-3-yl)-N-(3-(phenyl-1,2,4-thiadiazol-5-yl)acetamide | CPD-28 | Me | Me | H | H | Et | H | H | H | 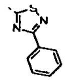 |
| 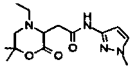 | 2-(4-ethyl-6,6-dimethyl-2-oxomorpholin-3-yl)-N-(1-methyl-1H-pyrazol-3-yl)acetamide | CPD-29 | Me | Me | H | H | Et | H | H | H | 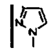 |
| 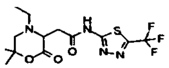 | 2-(4-ethyl-6,6-dimethyl-2-oxomorpholin-3-yl)-N-(5-(trifluoromethyl)-1,3,4-thiadiazol 2-yl)acetamide | CPD-30 | Me | Me | H | H | Et | H | H | H | 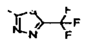 |
| 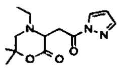 | 4-ethyl-6,6-dimethyl-3-(2-oxo-2-(1H-pyrazol-1-yl)ethyl)morpholin-2-one | CPD-31 | Me | Me | H | H | Et | H | H | 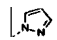 | |
| 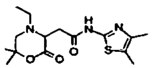 | N-(4,5-dimethylthiazol-2-yl)-2-4-ethyl-6,6-dimethyl-2-oxomorpholin-3-yl)acetamide | CPD-32 | Me | Me | H | H | Et | H | H | H | 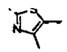 |

(continued)

| STRUCTURE | Name | Code | $R^1$ | $R^2$ | $R^3$ | $R^4$ | $R^5$ | $R^6$ | $R^7$ | $R^8$ | $R^9$ |
|---|---|---|---|---|---|---|---|---|---|---|---|
| 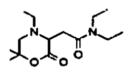 | N,N-diethyl-2-(4-(ethyl-6,6-dimethyl-2-oxomorpholin-3-yl)acetamide | CPD-33 | Me | Me | H | H | Et | H | H | Et | Et |
| 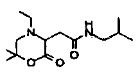 | 2-(4-ethyl-6,6-dimethyl-2-oxomorpholin-3-yl)-N-isobutylacetamide | CPD-34 | Me | Me | H | H | Et | H | H | H | 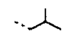 |
| 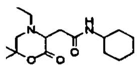 | N-cyclohexyl-2-(4-ethyl-6,6-dimethyl-2-oxomorpholin-3-yl)acetamide | CPD-35 | Me | Me | H | H | Et | H | H | H | 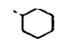 |
| 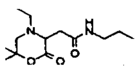 | 2-(4-ethyl-6,6-dimethyl-2-oxomorpholin-3-yl)-N-propylacetamide | CPD-36 | Me | Me | H | H | Et | H | H | H | Pr |
| 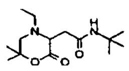 | N-tert-butyl-2-(4-ethyl-6,6-dimethyl-2-oxomorpholin-3-yl)acetamide | CPD-37 | Me | Me | H | H | Et | H | H | H | tBu |
| 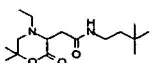 | N-(3,3-dimethylbutyl)-2-(4-ethyl-6,6-dimethyl-2-oxomorpholin-3-yl)acetamide | CPD-38 | Me | Me | H | H | Et | H | H | H | 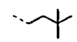 |
| 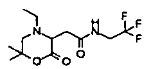 | 2-(4-ethyl-6,6-dimethyl-2-oxomorpholin-3-yl)-N-(2,2,2-trifluoroethyl)acetamide | CPD-39 | Me | Me | H | H | Et | H | H | H | 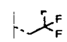 |
| 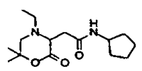 | N-cyclopentyl-2-(4-ethyl-6,6-dimethyl-2-oxomorpholin-3-yl)acetamide | CPD-40 | Me | Me | H | H | Et | H | H | H | 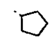 |
| 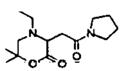 | 4-ethyl-6,6-dimethyl-3-(2-oxo-2-(pyrrolidin-1-yl)ethyl)morpholin-2-one | CPD-41 | Me | Me | H | H | Et | H | H | 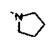 | |
| 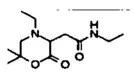 | N-ethyl-2-(4-ethyl-6,6-dimethyl-2-oxomorpholin-3-yl)acetamide | CPD-42 | Me | Me | H | H | Et | H | H | H | Et |
| 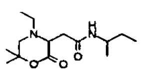 | N-sec-butyl-2-(4-ethyl-6,6-dimethyl-2-oxomorpholin-3-yl)acetamide | CPD-43 | Me | Me | H | H | Et | H | H | H | 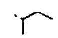 |
| 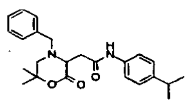 | 2-(4-benzyl-6,6-dimethyl-2-oxomorpholin-3-yl)-N-(4-isopropylphenyl)acetamide | CPD-44 | Me | Me | H | H | Bn | H | H | H | 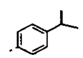 |

(continued)

| STRUCTURE | Name | Code | $R^1$ | $R^2$ | $R^3$ | $R^4$ | $R^5$ | $R^6$ | $R^7$ | $R^8$ | $R^9$ |
|---|---|---|---|---|---|---|---|---|---|---|---|
| 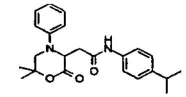 | 2-(6,6-dimethyl-2-oxo-4-phenylmorpholin-3-yl)-N-(4-isopropylphenyl)acetamide | CPD-45 | Me | Me | H | H | Ph | H | H | H | 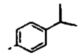 |
| 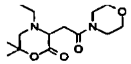 | 4-ethyl-6,6-dimethyl-3-(2-morpholino-2-oxoethyl)morpholin-2-one | CPD-46 | Me | Me | H | H | Et | H | H | 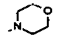 | |
| 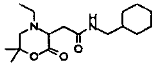 | N-(cyclohexylmethyl)-2-(4-ethyl-6,6-dimethyl-2-oxomorpholin-3-yl)acetamide | CPD-47 | Me | Me | H | H | Et | H | H | H | 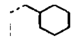 |
| 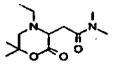 | 2-(4-ethyl-6,6-dimethyl-2-oxomorpholin-3-yl)-N,N-dimethylacetamide | CPD-48 | Me | Me | H | H | Et | H | H | Me | Me |
| 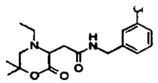 | N-(3-chlorobenzyl)-2-(4-ethyl-6,6-dimethyl-2-oxomorpholin-3-yl)acetamide | CPD-49 | Me | Me | H | H | Et | H | H | H | 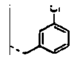 |
| 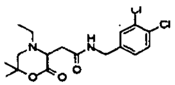 | N-(3,4-dichlorobenzyl)-2-(4-ethyl-6,6-dimethyl-2-oxomorpholin-3-yl)acetamide | CPD-50 | Me | Me | H | H | Et | H | H | H | 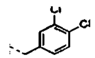 |
| 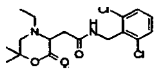 | N-(2,6-dichlorobenzyl)-2-(4-ethyl-6,6-dimethyl-2-oxomorpholin-3-yl)acetamide | CPD-51 | Me | Me | H | H | Et | H | H | H | 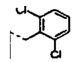 |
| 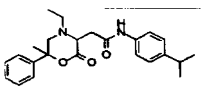 | 2-(4-ethyl-6-methyl-2-oxo-6-phenylmorpholin-3-yl)-N-(4-isopropylphenyl)acetamide | CPD-52 | Ph | Me | H | H | Et | H | H | H | 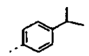 |
| 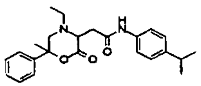 | 2-(4-ethyl-6-methyl-2-oxo-6-phenylmorpholin-3-yl)-N-(4-isopropylphenyl)acetamide | CPD-53 | Me | Ph | H | H | Et | H | H | H | 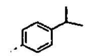 |
| 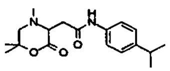 | N-(4-isopropylphenyl)-2-(4,6,6-trimethyl-2-oxomorpholin-3-yl)acetamide | CPD-54 | Me | Me | H | H | Me | H | H | H | 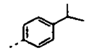 |
| 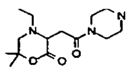 | 4-ethyl-6,6-dimethyl-3-(2-(4-methylpiperazin-1-yl)-2-oxoethyl)morpholin-2-one | CPD-55 | Me | Me | H | H | Et | H | H | 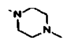 | |

(continued)

| STRUCTURE | Name | Code | $R^1$ | $R^2$ | $R^3$ | $R^4$ | $R^5$ | $R^6$ | $R^7$ | $R^8$ | $R^9$ |
|---|---|---|---|---|---|---|---|---|---|---|---|
| 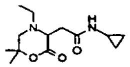 | N-cyclopropyl-2-(4-ethyl-6,6-dimethyl-2-oxomorpholin-3-yl)acetamide | CPD-56 | Me | Me | H | H | Et | H | H | H | 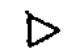 |
| 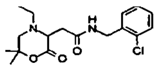 | N-(2-chlorobenzyl)-2-(4-ethyl-6,6-dimethyl-2-oxomorpholin-3-yl)acetamide | CPD-57 | Me | Me | H | H | Et | H | H | H | 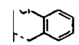 |
| 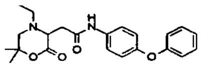 | 2-(4-ethyl-6,6-dimethyl-2-oxomorpholin-3-yl)-N-(4-phenoxyphenyl)acetamide | CPD-58 | Me | Me | H | H | Et | H | H | H | 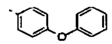 |
| 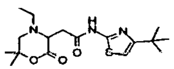 | N-(4-tert-butylthiazol-2-yl)-2-(4-ethyl-6,6-dimethyl-2-oxomorpholin-3-yl)acetamide | CPD-59 | Me | Me | H | H | Et | H | H | H | 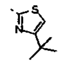 |
| 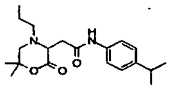 | 2-(6,6-dimethyl-2-oxo-4-propylmorpholin-3-yl)-N-(4-isopropylphenyl)acetamide | CPD-60 | Me | Me | H | H | Pr | H | H | H | 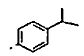 |
| 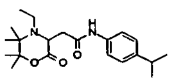 | 2-(4-ethyl-5,5,6,6-tetramethyl-2-oxomorpholin-3-yl)-N-(4-isopropylphenyl)acetamide | CPD-61 | Me | Me | Me | Me | Et | H | H | H | 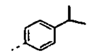 |
| 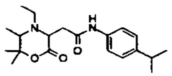 | 2-(4-ethyl-5,6,6-trimethyl-2-oxomorpholin-3-yl)-N-(4-isopropylphenyl)acetamide | CPD-62 | Me | Me | Me | H | Et | H | H | H | 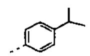 |
| 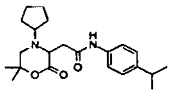 | 2-(4-cyclopentyl-6,6-dimethyl-2-oxomorpholin-3-yl)-N-(4-isopropylphenyl)acetamide | CPD-63 | Me | Me | H- | H | 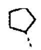 | H | H | H | 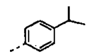 |
| 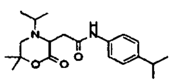 | 2-(4-isopropyl-6,6-dimethyl-2-oxomorpholin-3-yl)-N-(4-isopropylphenyl)acetamide | CPD-64 | Me | Me | H | H | 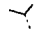 | H | H | H | 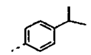 |
| 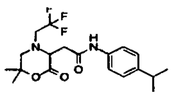 | 2-(6,6-dimethyl-2-oxo-4-(2,2,2-trifluoroethyl)morpholin-3-yl)-N-(4-isopropylphenyl)acetamide | CPD-65 | Me | Me | H | H | 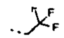 | H | H | H | 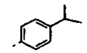 |
| 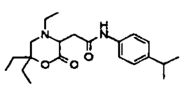 | N-(4-isopropylphenyl)-2-(4,6,6-triethyl-2-oxomorpholin-3-yl)acetamide | CPD-66 | Et | Et | H | H | Et | H | H | H | 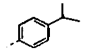 |

(continued)

| STRUCTURE | Name | Code | $R^1$ | $R^2$ | $R^3$ | $R^4$ | $R^5$ | $R^6$ | $R^7$ | $R^8$ | $R^9$ |
|---|---|---|---|---|---|---|---|---|---|---|---|
| 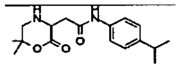 | 2-(6,6-dimethyl-2-oxomorpholin-3-yl)-N-(4-isopropylphenyl)acetamide | CPD-67 | Me | Me | H | H | H | H | H | H | 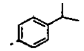 |
| 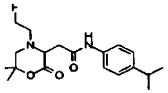 | 2-(4-(2-fluoroethyl)-6,6-dimetyl 2-oxomorpholin-3-yl)-N-(4-isopropylphenyl)acetamide | CPD-68 | Me | Me | H | H | 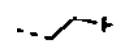 | H | H | H | 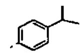 |
| 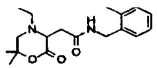 | 2-(4-ethyl-6,6-dimethyl-2-oxomorpholin-3-yl)-N-(2-methylbenzyl)acetamide | CPD-69 | Me | Me | H | H | Et | H | H | H | 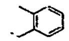 |
| 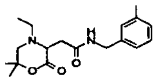 | 2-(4-ethyl-6,6-dimethyl-2-oxomorpholin-3-yl)-N-(3-methylbenzyl)acetamide | CPD-70 | Me | Me | H | H | Et | H | H | H | 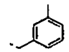 |
| 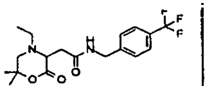 | 2-(4-ethyl-6,6-dimethyl-2-oxomorpholin-3-yl)-N-(4-(trifluoromethyl)benzyl)acetami de | CPD-71 | Me | Me | H | H | Et | H | H | H | 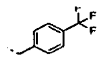 |
| 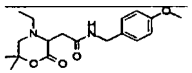 | 2-(4-ethyl-6,6-dimethyl-2-oxomorpholin-3-yl)-N-(4-methoxybenzyl)acetamide | CPD-72 | Me | Me | H | H | Et | H | H | H | 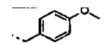 |
| 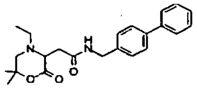 | -(biphenyl-4-ylmethyl)-2-(4-ethyl-6,6-dimethyl-2-oxomorpholin-3-yl)acetamide | CPD-73 | Me | Me | H | H | Et | H | H | H | 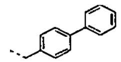 |
| 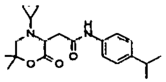 | 2-(4-cyclopropyl-6,6-dimethyl-2-oxomorpholin-3-yl)-N-(4-isopropylphenyl)acetamide | CPD-74 | Me | Me | H | H | 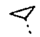 | H | H | H | 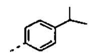 |
| 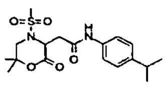 | 2-(6,6-dimethyl-4-(methylsulfonyl)-2-oxomorpholin-3-yl)-N-(4-isopropylphenyl)acetamide | CPD-75 | Me | Me | H | H | 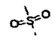 | H | H | H | 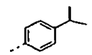 |
| 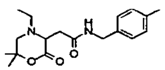 | 2-(4-ethyl-6,6-dimethyl-2-oxomorpholin-3-yl)-N-(4-methylbenzyl)acetamide | CPD-76 | Me | Me | H | H | Et | H | H | H | 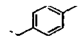 |
| 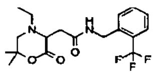 | 2-(4-ethyl-6,6-dimethyl-2-oxomorpholin-3-yl)-N-(2-(trifluoromethyl)benzyl)acetami de | CPD-77 | Me | Me | H | H | Et | H | H | H | 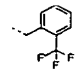 |

(continued)

| STRUCTURE | Name | Code | $R^1$ | $R^2$ | $R^3$ | $R^4$ | $R^5$ | $R^6$ | $R^7$ | $R^8$ | $R^9$ |
|---|---|---|---|---|---|---|---|---|---|---|---|
| 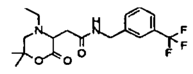 | 2-(4-ethyl-6-dimethyl-2-oxomorpholin-3-yl)-N-(3-(trifluoromethyl)benzyl)acetami de | CPD-78 | Me | Me | H | H | Et | H | H | H | 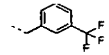 |
| 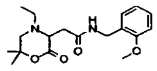 | 2-(4-ethyl-6,6-dimethyl-2-oxomorpholin-3-yl)-N-(2-methoxybenzyl)acetamide | CPD-79 | Me | Me | H | H | Et | H | H | H | 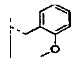 |
| 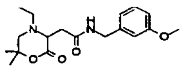 | 2-(4-ethyl-6,6-dimethyl-2-oxomorpholin-3-yl)-N-(3-methoxybenzyl)acetamide | CPD-80 | Me | Me | H | H | Et | H | H | H | 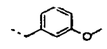 |
| 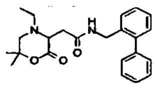 | N-(biphenyl-2-ylmethyl)-2-(4-ethyl-6,6-dimethyl-2-oxomorpholin-3-yl)acetamide | CPD-81 | Me | Me | H | H | Et | H | H | H | 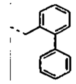 |
| 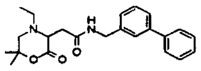 | N-(biphenyl-3-ylmethyl-2-(4-ethyl-6,6-dimethyl-2-oxomorpholin-3-yl)acetamide | CPD-82 | Me | Me | H | H | Et | H | H | H | 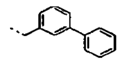 |
| 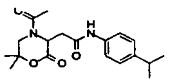 | 2-(4-acetyl-6,6-dimethyl-2-oxomorpholin-3-yl)-N-(4-isopropylphenyl)acetamide | CPD-83 | Me | Me | H | H | 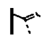 | H | H | H | 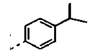 |
| 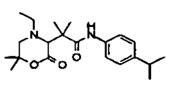 | 2-(4-ethyl-6,6-dimethyl-2-oxomorpholin-3-yl)-N-(4-isopropylphenyl)-2-methylpropanamide | CPD-84 | Me | Me | H | H | Et | Me | Me | H | 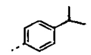 |

EXAMPLE 1: ANTIFUNGAL ACTIVITY

**Candida species (e.g. C. albicans, C. glabrata, C. krusei) antifungal activity:**

YPD agar (1% yeast extract, 2% peptone, 2% glucose, 1,5% agar) was inoculated with 1/500 of an overnight culture in YPD of either *Candida albicans* or *Candida glabrata,* and poored in a square petridish (12 cm x 12 cm). Five μl of the compound of the invention, dissolved at 2 mg/ml DMSO, were spotted on the agar. After 24 h incubation at 37°C, compounds were assessed for their growth-inhibitory potential by measurement of the inhibitory halo of the compounds. The majority of the compounds showed a growth-inhibitory halo of > 6 mm for Candida albicans.

**Determination of the Minimal Fungicidal Concentration (MFC) against *Candida* spp. and of the Minimal Inhibitory concentration (MIC) and MFC against filamentous fungi (*Aspergillus flavus, Fusarium* spp.)**

Two-fold dilution series of the compounds were prepared in 50% DMSO. 5 μl of these series were added to 95 μl PBS buffer inoculated with 1/500 cells of an overnight culture of either C. *albicans, C. glabrata* or *C. krusei* (final DMSO conc 2%). 2% DMSO served as negative control. After 24h incubation at 37°C of these cultures with the antifungal compounds in PBS, the cell cultures were plated on YPD agar and colony forming units (CFUs) were determined after incubation of the plates for 2 days. MFC was determined as the minimal concentration of a compound resulting in less than 1% survival of the yeast culture relative to the DMSO control.
To determine the MIC and MFC of a compound against *Aspergillus flavus, Fusarium oxysporum* or *F. culmorum,* a 2-fold dilution series of the compounds of the invention was incubated with the corresponding spore suspension in ½ PDB (potato dextrose broth, Difco) ($2x10^4$ spores/ml). After 24h incubation at 25°C, the minimal inhibitory concentration (MIC) was determined as the minimal concentration that inhibits growth of the fungus. Subsequently, the content of all the wells which showed no fungal growth was plated on PDB agar and colony forming units (CFUs) were determined after incubation of the plates for 2 days. MFC was determined as the minimal concentration of a compound resulting in less than 1% survival of the fungal culture relative to the DMSO control.
The majority of the compounds of the invention showed an antifungal activity against Candida, Aspergillus as well as against Fusarium species, while some compounds were less broad spectrum and showed activity against only one or two of Candida, Aspergillus or Fusarium species.

Table 2 shows an overview of the activity of examples of the compounds of the invention against C. albicans (MFC), C. glabrata (MFC), A. fumigatus (MIC), A. flavus (MIC) and F. oxysproum (MIC).

Table 2: Overview of the antifungal activity of compounds of the invention.

| Code | MFC C. albicans SC5314 (μg/ml) | MFC C. glabrata (μg/ml) | MIC A. fumigatus (μg/ml) | MIC A. flavus (μg/ml) | MIC F. oxysporum (μg/ml) |
|---|---|---|---|---|---|
| CPD-01 | 12,5 | 12,5 | 0,8 | 12,5 | ND |
| CPD-02 | 12,5 | 12,5 | 0,8 | 6,25 | ND |
| CPD-03 | 12,5 | 6,25 | 0,8 | 6,25 | ND |
| CPD-04 | 12,5 | 6,25 | 0,8 | 12,5 | ND |
| CPD-05 | 12,5 | 6,25 | 0,8 | 6,25 | ND |
| CPD-06 | 12,5 | 25 | 1,6 | 12,5 | 50 |
| CPD-07 | 12,5 | 50 | 3,125 | 25 | 50 |
| CPD-08 | 12,5 | 12,5 | 6,25 | 25 | 50 |
| CPD-09 | 25 | 50 | 50 | >50 | >50 |
| CPD-10 | >50 | >50 | 12,5 | >50 | >50 |
| CPD-12 | 12,5 | 25 | 12,5 | 50 | >50 |
| CPD-14 | 25 | >50 | >50 | >50 | >50 |
| CPD-18 | 25 | 50 | 6,25 | 25 | >50 |
| CPD-22 | 25 | 50 | 25 | 50 | 50 |

(continued)

| Code | MFC C. albicans SC5314 (μg/ml) | MFC C. glabrata (μg/ml) | MIC A. fumigatus (μg/ml) | MIC A. flavus (μg/ml) | MIC F. oxysporum (μg/ml) |
|---|---|---|---|---|---|
| CPD-23 | 50 | >50 | 25 | 50 | 50 |
| CPD-36 | 25 | >50 | 50 | >50 | >50 |
| CPD-38 | 25 | 12,5 | 50 | 50 | 50 |
| CPD-39 | 50 | >50 | 25 | >50 | >50 |
| CPD-47 | 25 | 6,25 | 25 | 50 | >50 |
| CPD-49 | 6,25 | 6,25 | 3,125 | 50 | 50 |
| CPD-50 | 12,5 | 3,12 | 1,6 | 6,25 | 25 |
| CPD-51 | 12,5 | 25 | 12,5 | 50 | 50 |
| CPD-57 | 25 | ND | ND | ND | ND |
| CPD-69 | =25 | ND | ND | ND | ND |
| CPD-70 | =25 | ND | ND | ND | ND |
| CPD-71 | 25 | ND | ND | ND | ND |
| CPD-72 | =25 | ND | ND | ND | ND |
| CPD-73 | =25 | ND | ND | ND | ND |
| CPD-75 | >25 | >25 | 12,5 | >25 | >25 |
| CPD-76 | 25 | 6,25 | =3,125 | 25 | >25 |
| CPD-77 | 25 | 12,5 | =3,125 | 25 | 25 |
| CPD-78 | 25 | 12,5 | 6,25 | >25 | >25 |
| CPD-79 | >25 | 25 | 12,5 | >25 | >25 |
| CPD-80 | 25 | 12,5 | 6,25 | >25 | >25 |
| CPD-81 | 12,5 | 12,5 | =3,125 | 25 | 25 |
| CPD-82 | 12,5 | 12,5 | =3,125 | 25 | 25 |

EXAMPLE 2: CELLULAR CYTOSTATIC AND/OR CYTOTOXIC EFFECT

Evaluation of the cytostatic and/or cytotoxic effect of compounds on certain specific eukaryotic cells is a standard procedure and well known to the person skilled in the art. The effect of a selection of compounds of the invention on the proliferation of HepG2 cells was measured. All compounds tested did not show a cytostatic or cytotoxic activity on the HepG2 cells at 25 μM.

As an example, table 3 shows the LD30 (lethal dose for 30% of the cells in culture) for a selection of compounds on HepG2 cells.

Table 3:

| **Compound code** | **LD30 48h (μM)** |
|---|---|
| CPD-06 | >25 |
| CPD-07 | >25 |
| CPD-08 | >25 |
| CPD-22 | >25 |
| CPD-49 | >25 |

(continued)

| Compound code | LD30 48h (μM) |
|---|---|
| CPD-51 | >25 |

EXAMPLE 3: IN VIVO EFFICACY AND TOXICITY MEASUREMENT

***C. elegans* model for *C. albicans* infection.**

The *in vivo* efficacy of the compounds was assessed in a *C. elegans* model for *Candida* infection as described by Breger *et al.,* 2007 (Breger, J., et al. 2007. PLoS Pathog. 3:e18). To this end, L4 larvae of a double mutant (glp-4Δsek-1Δ) of *C. elegans* were used and fed for 4 h on *C. albicans* SC5314 agar plates (YPD agar plates on the surface inoculated with 100μl of an overnight culture in YPD and incubated for 16 h at 37°C). Worms were collected and washed with M9 buffer containing 3g/L $KH_2PO_4$, 6g/L $Na_2HPO_4$, 5g/L NaCl, 1mM $MgSO_4$, 10μg/mL cholesterol and 100μg/mL Kanamycin. Fourty-to-fifty worms were suspended in 1 mL in each well of 24-well microtiterplates, in the presence or absence (DMSO control) of the compounds of the invention (at different concentrations). Survival of the worms was monitored daily. The percentage survival of the worms in the presence or absence of antifungal compounds was calculated each day relative to the survival at day 0. Data are means of duplicate measurements and experiments were performed at least twice. As positive controls for efficacy testing, amphotericin B (10 μg/ml) and miconazole (10 μg/ml) in DMSO can be used.

Results of the in vivo evaluation of CPD-06 in this C. elegans model are shown in figure 1 and clearly show an increase in survival of C. albicans infected C. elegans after treatment with CPD-06.

Rodent model for fungal infection

An assay for in vivo efficacy measurement using mouse model for Candidiasis Fungal burden in the kidney of infected mice was used to evaluate the (prophylactic and/or therapeutic) activity of the compounds of the invention in murine models of infection by C. albicans, as described in the prior art (Tavares PM et al. Antimicrob Agents Chemother. 2008 Dec;52(12):4522-5; Clemons KV, DA Stevens, J Antimicrob. Chemother. 2001; 47:183-186; Okawa Y et al., Biol Pharm Bull 2007, 30:1870-1873).

In general, mice can be inoculated intravenously via lateral tail vein with $2x10^5$ to $2x10^3$ C. albicans yeast cells suspended in saline. The compounds can be administered (for example intravenously or intraperitonealy or orally) before (for example 1 hour before) or after (for example 16 hours after) the challenge with C. albicans. After this first injection, four similar subsequent injections can be made with 24 h or other intervals. Control groups can be treated with around 10mg/kg doses of fluconazole or the vehicel (e.g. saline) following the same protocol. Mice are then sacrificed 5 days after fungal infection. Kidneys are then excised, weighted, homogenized and the pellets are resuspended in PBS (1 ml). 100μl-samples can be plated onto solid brain heart infusion (BHI) plates and C. albicans CFU (colony forming units) are counted after 2 days.

More specifically, the in vivo experiment was performed as following (in accordance with Tavares PM et al. Antimicrob Agents Chemother. 2008 Dec;52(12):4522-5).

BALB/c mice were treated during the experiment with 100 mg/kg ciclophosphamide, this at 4 days and 24 hours before onset of infection, and an additional dose at 3 days post infection. To start the fungal infection, mice were inoculated intravenously via the lateral tail vein with 2 10(3) yeast cells of *C. albicans* SC5314 in saline (50 μL). The compound was administered intraperitonealy (at 10 mg/kg) 1 hour before the challenge with C. albicans. Four similar subsequent injections are made with 24 h intervals (referred to as "1 Dose" in Figure 2) for one group of mice and with smaller intervals (between 8 and 12h) allowing a two times a day administration (referred to as "2 Dose" in Figure 2) for another group of mice. Control groups were treated with 10mg/kg doses of fluconazole (referred to as "Fluconazol" in Figure 2) or the vehicle alone (5% DMSO and Methyl cellulose 0.5% - referred to as "Control" in Figure 2) following the same protocol. Mice were sacrificed 5 days after fungal infection. Kidneys were excised, weighted, homogenized and the pellets were resuspended in PBS (1 ml). 100μl-samples were plated onto solid brain heart infusion (BHI) plates and C. albicans CFU (colony forming units) were counted after 2 days.

The IP formulation used was: DMSO/MethylCellulose 0.5% (5/95). This formulation was obtained as following:

- 10mg of Cpd06 was weighed, added to 0.25ml DMSO and vortexed for 30 seconds;
- 4.75ml of Methylcellulose 0.5% was added and vortexed for 30 seconds.

The results of this in vivo experiment are shown in figure 2 and show that the Cpd06 has a strong activity against Candida

albicans infections.

To assess the effect of Cpd06 via PO or IV administration, the following doses can be used: around 10 mg/kg and around 2 mg/kg respectively or can be selected based on the activity of the compounds of the invention.
The PO formulation to be used can be DMSO/MethylCellulose 0.5% (5/95) and can be obtained as described.
The IV formulation can be: DMSO/SOLUTOL HS 15/SALINE (5/5/90). This formulation can be obtained as following:

- 2mg of Cpd06 was weighed, added to 0.25ml DMSO and vortexed for 30 seconds;
- 0.25ml SOLUTOL HS 15 was added and vortexed for 30 seconds;
- 4.5ml SALINE was added and vortexed for 30 seconds.

EXAMPLE 4: MATERIALS AND GENERAL PREPARATION METHODS

All the preparative HPLC purifications mentioned in this experimental part have been carried out with the following system: a Waters 2489 UV/Visible Detector, a Waters 2545 Binary Gradient Module, a Waters Fraction Collector III and a Waters Dual Flex Injector. The separations were performed with a SunFire Prep C18 ODB column (5 μm; 19 x 100 mm) equipped with a SunFire C18 guard column (5 μm; 19 x 10 mm). Elutions were carried out with the methods described in the following tables, and detection wavelengths were fixed at 210 and 254 nm.

HPLC method 1

| Time (min) | Flow Rate (mL/min) | Solvent A (%) | Solvent B (%) |
|---|---|---|---|
| 0 | 20 | 50 | 50 |
| 2.00 | 20 | 50 | 50 |
| 9.00 | 20 | 10 | 90 |
| 11.00 | 20 | 10 | 90 |
| 11.20 | 20 | 50 | 50 |
| 16.00 | 20 | 50 | 50 |
| Solvent A: Formic Acid LC-MS grade 0.1% in milliQ water<br>Solvent B: Acetonitrile HPLC grade. | | | |

HPLC method 2

| Time (min) | Flow Rate (mL/min) | Solvent A (%) | Solvent B (%) |
|---|---|---|---|
| 0 | 20 | 80 | 20 |
| 2.00 | 20 | 80 | 20 |
| 8.00 | 20 | 10 | 90 |
| 10.80 | 20 | 10 | . 90 |
| 11.00 | 20 | 80 | 20 |
| 16.00 | 20 | 80 | 20 |
| Solvent A: Formic Acid LC-MS grade 0.1% in milliQ water<br>Solvent B: Acetonitrile HPLC grade. | | | |

HPLC method 3

| Time (min) | Flow Rate (mL/min) | Solvent A (%) | Solvent B (%) |
|---|---|---|---|
| 0 | 20 | 95 | 5 |
| 2.00 | 20 | 95 | 5 |
| 8.00 | 20 | 50 | 50 |
| 9.00 | 20 | 10 | 90 |
| 13.00 | 20 | 10 | 90 |
| 14.00 | 20 | 95 | 5 |
| 16.00 | 20 | 95 | 5 |
| Solvent A: Formic Acid LC-MS grade 0.1% in milliQ water<br>Solvent B: Acetonitrile HPLC grade. | | | |

General procedure A: Epoxide opening with amine

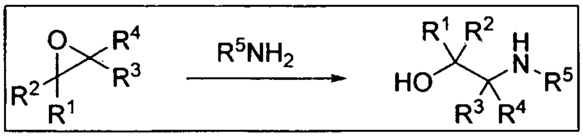


To a solution of an epoxide (1.0 eq) in methanol (1.5 mL/mmol) in a sealed tube was added an amine (2-3 eq). The reaction mixture was heated overnight at 100 °C. The reaction mixture was concentrated under reduced pressure. The residue was purified by distillation under reduced pressure to afford the desired aminoalcohol.

General procedure B: morpholinone formation

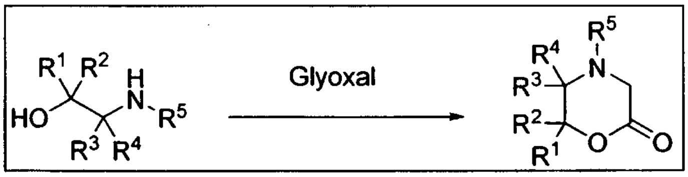


To a mixture of 40% glyoxal solution (1.0 eq) and toluene (0.5 mL/mmol) cooled at 10 °C was added a solution of an aminoalcohol (1.0 eq) in toluene (0.3 mL/mmol). After 2-3 h at 10 °C, the reaction mixture was heated at reflux to azeotrope out solvents. The residue was purified by distillation under reduced pressure or by flash chromatography on silica gel to afford the desired morpholinone.

**General procedure C: Alkylation**

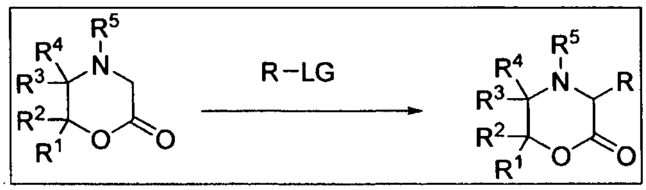

To a solution of an morpholinone (1.0 eq) in tetrahydrofuran (6 mL/mmol) cooled at -70 °C was added dropwise a 1 N lithium bis(trimethylsilyl)amide solution in tetrahydrofuran (1.0 eq). After,1 h at -70 °C, an alkylating reagent (1.1 eq) was added dropwise and the reaction mixture was stirred at -70 °C for 3-5 h. The reaction was quenched by addition of a saturated ammonium chloride solution: After warming to room temperature, the solids were filtered and the filtrate was concentrated under reduced pressure and coevaporated with ethanol. The residue was taken up with ethyl acetate, dried over sodium sulfate, filtered and concentrated under reduced pressure. The residue was purified by flash chromatography on silica gel to afford the desired product.

**General procedure D: Deprotection in acidic condition**

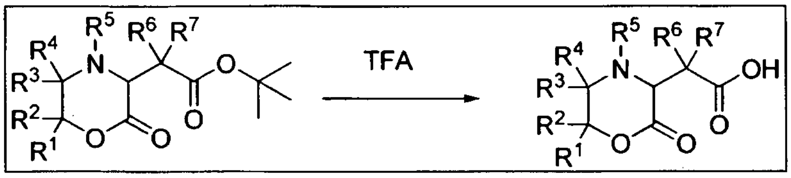


To a solution of a *tert*-butyl ester (1.0 eq) in dichloromethane (4.6 mL/mmol) was added trifluoroacetic acid (1.6 mL/mmol). After 5-6 h at room temperature, the reaction mixture was concentrated and coevaporated with toluene to give the desired acid under its trifluoroacetate salt form.
Crude acid was used in the next step without further purification.

**General procedure E: Amide formation**

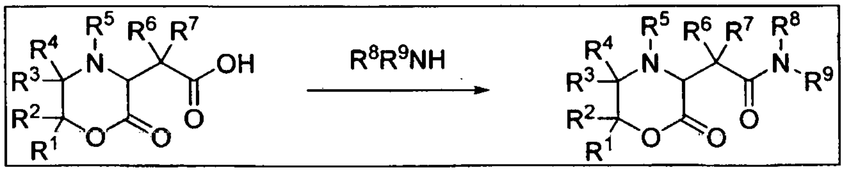


To a solution of an acid (1.0 eq) in DMF (7.4 mL/mmol) were added diisopropylethylamine (4.0-5.0 eq) and *O*-(7-azabenzotriazol-1-yl)-*N,N,N',N'*-tetramethyluronium hexafluorophosphate (HATU, 1.0-1.2 eq). After 30 min at room temperature, the appropriate amine (1.0-1.3 eq) was added and the reaction mixture was stirred at room temperature overnight. The solvent was evaporated under reduced pressure. The residue was partitioned between dichloromethane and a saturated sodium bicarbonate solution. The organic phase was washed with water and brine, dried over sodium sulfate, filtered, concentrated under reduced pressure and purified by flash column chromatography on silica gel followed by a recrystallization or purification by preparative HPLC or preparative TLC if needed.

**General procedure F: Reductive amination**

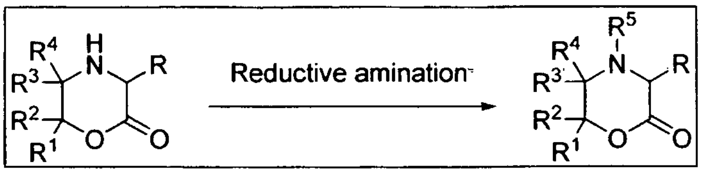


To a solution of a morpholinone (1.0 eq) in tetrahydrofuran (5 mL/mmol) and acetonitrile (14 mL/mmol) was added an aldehyde or a ketone (5.0-5.1 eq). After 15 min at room temperature, sodium cyanoborohydride (2.0 eq) was added portionwise. The reaction mixture was stirred for an additional 15 min and acetic acid (2.1 eq) was added. The reaction mixture was stirred at room temperature for 1-18h. The reaction was quenched by addition of a saturated sodium

bicarbonate solution. The reaction mixture was diluted with ethyl acetate. The phases were separated. The organic phase was washed with water and brine, dried over sodium sulfate, filtered and concentrated. The residue was purified by flash chromatography on silica gel to give the desired compound.

EXAMPLE 5: PREPARATION OF INTERMEDIATES

INTERMEDIATE 1 - PREPARATION OF 1-(Ethylamino)-2-methylpropan-2-ol

The compound was prepared according to the procedure A from isobutylene oxide (3.522 mL, 39.660 mmol) and a 70% ethylamine solution in water (7.200 mL) in methanol (60 mL). Purification by distillation under reduced pressure furnished 4.420 g (95%) of the title compound as a colourless liquid.
$^1$H NMR ($CDCl_3$) $\delta$ 1.11 (3H, t); 1.17 (6H, s); 2.53 (2H, s); 2.70 (2H, q).
ESI/APCI(+): 118 (M+H).

INTERMEDIATE 2 - PREPARATION OF 2-(Ethylamino)-1-phenylethanol

To a solution of 2-amino-1-phenylethanol (2.000 g, 14.579 mmol) in ethanol (15 mL) cooled at 0°C was added acetaldehyde (0.818 mL; 14.577 mmol). After 45 min at 0 °C, sodium borohydride (1.100 g; 29.077 mmol) was added portionwise over 5 min and the reaction mixture was stirred at 0°C for 1 h. The reaction was quenched by addition of water and 5% sodium hydroxide solution. The reaction mixture was extracted twice with ethyl acetate. The organic phases were combined, dried over sodium sulfate, filtered and concentrated. The residue was purified by flash chromatography on silica gel using tetrahydrofuran/ethanol/ammonium hydroxide (20/1/1) as eluent to afford 1.600 g (66%) of the title compound as a white solid.
ESI/APCI(+): 166 (M+H).

INTERMEDIATE 3 - PREPARATION OF (*S*)-2-(Ethylamino)-1-phenylethanol

To a solution of (*S*)-2-amino-1-phenylethanol (0.960 g, 6.998 mmol) in ethanol (7.5 mL) cooled at 0°C was added acetaldehyde (0.410 mL; 7.306 mmol). After 1 h at 0 °C, sodium borohydride (0.550 g; 14.539 mmol) was added portionwise and the reaction mixture was stirred at 0°C for 3 h. The reaction was quenched by addition of water and 5% sodium hydroxide solution. The reaction mixture was extracted twice with ethyl acetate. The organic phases were combined, dried over sodium sulfate, filtered and concentrated under reduced pressure. The residue was purified by flash chromatography on silica gel using tetrahydrofuran/ethanol/ammonium hydroxide (20/1/1) as eluent to afford 0.785 g (86%) of the title compound as a yellow oil.
ESI/APCI(+): 166 (M+H).

INTERMEDIATE 4 - PREPARATION OF (*R*)-2-(Ethylamino)-1-phenylethanol

(Synthesis described in *Tet. Asym.* 1997, 459)

To a solution of (*R*)-2-amino-1-phenylethanol (1.000 g, 7.290 mmol) in ethanol (7.5 mL) cooled at 0°C was added acetaldehyde (0.410 mL; 7.306 mmol). After 1 h at 0 °C, sodium borohydride (0.550 g; 14.539 mmol) was added portionwise and the reaction mixture was stirred at 0°C for 3 h. The reaction was quenched by addition of water and 5% sodium hydroxide solution. The reaction mixture was extracted twice with ethyl acetate. The organic phases were combined, dried over sodium sulfate, filtered and concentrated under reduced pressure. The residue was purified by flash chromatography on silica gel using tetrahydrofuran/ethanol/ammonium hydroxide (20/1/1) as eluent to afford 0.900 g (75%) of the title compound as a yellow oil.
ESI/APCI(+): 166 (M+H).

INTERMEDIATE 5 - PREPARATION OF (*S*)-1-(Ethylamino)propan-2-ol

To a solution of (*S*)-1-amino-2-propanol (1.270 g, 16.909 mmol) in ethanol (16.5 mL) cooled at 0°C was added acetaldehyde (0.948 mL; 18.293 mmol). After 45 min at 0 °C, sodium borohydride (1.270 g; 33.571 mmol) was added portionwise and the reaction mixture was stirred at 0°C for 1 h. The reaction was quenched by addition of water. After dilution with ethyl acetate, the solids were filtered. The filtrate was concentrated under reduced pressure. The residue was taken up with diethyl ether, dried over sodium sulfate, filtered and concentrated under reduced pressure. The residue was purified by distillation under reduced pressure to afford 0.692 g (40%) of the title compound as a colourless liquid.
ESI/APCI(+): 104 (M+H).

INTERMEDIATE 6 - PREPARATION OF (*R*)-1-(Ethylamino)propan-2-ol

To a solution of (*R*)-1-amino-2-propanol (1.830 g, 24.364 mmol) in ethanol (24 mL) cooled at 0°C was added acetaldehyde (1.370 mL; 24.414 mmol). After 50 min at 0 °C, sodium borohydride (1.830 g; 48.374 mmol) was added portionwise and the reaction mixture was stirred at 0°C for 1 h. The reaction was quenched by addition of water. After dilution with diethyl ether, the solids were filtered. The filtrate was concentrated under reduced pressure. The residue was coevaporated with ethanol. The residue was then taken up with diethyl ether, dried over sodium sulfate, filtered and concentrated under reduced pressure. The residue was purified by distillation under reduced pressure to afford 1.530 g (61%) of the title compound as a colourless liquid.
$^1$H NMR ($CDCl_3$) δ 1.11 (3H, t); 1.16 (3H, d); 2.43 (2H, m); 2.66 (2H, m); 3.80 (1H, m).
ESI/APCI(+): 104 (M+H).

INTERMEDIATE 7 - PREPARATION OF 1-(Ethylamino)-2-phenylpropan-2-ol

The compound was prepared according to the procedure A from 2-phenylpropylene oxide (2.610 mL, 19.841 mmol) and a 70% ethylamine solution in water (3.600 mL) in methanol (30 mL). Purification by distillation under reduced pressure furnished 2.17 g (61 %) of the title compound as a yellow liquid.
$^1$H NMR ($CDCl_3$) δ 1.02 (3H, t); 1.47 (3H, s); 2.60 (2H, q); 2.65 (2H, d); 3.08 (2H, d); 7.23 (1H, m); 7.34 (2H, m); 7.45 (2H, m).
ESI/APCI(+): 180 (M+H).

INTERMEDIATE 8 - PREPARATION OF 3-(Ethylamino)-2,3-dimethylbutan-2-ol

To a solution of 2,3-dimethyl-2,3-epoxybutane (3.000 mL; 23.393 mmol) in methanol (40 mL) in a sealed tube was added a 70% ethylamine solution in water (4.240 mL). The reaction mixture was heated at 100 °C for 6 days. The reaction mixture was concentrated under reduced pressure and coevaporated with ethanol. The residue was purified by distillation under reduced pressure to give 0.680 g of the title compound as a colourless liquid.
$^1$H NMR ($CDCl_3$) δ 1.06 (6H, s); 1.09 (3H, t); 1.16 (6H, s); 2.60 (2H, q).
ESI/APCI(+): 146 (M+H).

INTERMEDIATE 9 - PREPARATION OF 3-(Ethylamino)-2-methylbutan-2-ol

The compound was prepared according to the procedure A from 2,3-epoxy-2-methylbutane (2.784 mL, 26.440 mmol) and a 70% ethylamine solution in water (4.800 mL) in methanol (40 mL). Purification by distillation under reduced pressure furnished 3.053 g (88%) of the title compound as a colourless liquid.
$^1$H NMR ($CDCl_3$) δ 1.02 (3H, s); 1.06 (3H, d); 1.10 (3H, t); 1.18 (3H, s); 2.40 (1H, m); 2.51 (1 H, m); 2.85 (1H, m).
ESI/APCI(+): 132 (M+H).

INTERMEDIATE 10 - PREPARATION OF 1-(Benzylamino)-2-methylpropan-2-ol

The compound was prepared according to the procedure A from isobutylene oxide (2.348 mL, 26.440 mmol) and benzylamine (6.300 mL; 57.679 mmol) in methanol (32 mL). Purification by flash chromatography on silica gel using a gradient of methanol (0 - 10%) in dichloromethane furnished 4.077 g (86%) of the title compound as a white solid.
$^1$H NMR ($CDCl_3$) δ 1.18 (6H, s); 2.57 (2H, s); 3.85 (2H, s); 7.33 (5H, m).
ESI/APCI(+): 180 (M+H).

INTERMEDIATE 11 - PREPARATION OF 2,2-Diethyloxirane.

To a solution of 2-ethyl-1-butene (2.000 g; 16.374 mmol) in dichloromethane (90 mL) was added dropwise a solution of 3-chloroperbenzoic acid (6.400 g; 25.960 mmol) in dichloromethane (45 mL) over 2 h. The reaction mixture was stirred at room temperature overnight and was then treated with a saturated sodium sulfite solution. The phases were separated. The organic phase was washed with a saturated sodium bicarbonate solution, water and brine, dried over sodium sulfate, filtered and concentrated to give 1.520 g (93%) of the title compound as a yellow liquid which was used in the next step without further purification:

$^1$H NMR ($CDCl_3$) δ 0.93 (6H, t); 1.61 (4H, m); 2.59 (2H, s).

INTERMEDIATE 12 - PREPARATION OF 3-((Ethylamino)methyl)pentan-3-ol.

The compound was prepared according to the procedure A from 2,2-diethyloxirane (1.520 g, 15.180 mmol) and a 70% ethylamine solution in water (2.800 mL) in methanol (24 mL). Purification by distillation under reduced pressure furnished 1.350 g (61%) of the title compound as a colourless liquid.
$^1H$ NMR ($CDCl_3$) $\delta$ 0.86 (6H, t); 1.10 (3H, t); 1.45 (4H, m); 2.53 (2H, s); 2.68 (2H, q).
ESI/APCI(+): 146 (M+H); 168 (M+Na).

INTERMEDIATE 13 - PREPARATION OF 2-Methyl-1-(phenylamino)propan-2-ol. Synthesis described in *Synthesis* 2004, 1563

To a solution of isobutylene oxide (0.500 mL; 5.630 mmol) and lithium perchlorate (5.850 g; 54.986 mmol) in diethyl ether (11 mL) was added aniline (1.130 mL; 12.401 mmol). The reaction mixture was stirred at room temperature for 1.5 h at was poured into ice-water. The phases were separated. The aqueous phase was extracted with dichloromethane. The organic phases were combined, dried over sodium sulfate, filtered and concentrated. The residue was purified by flash chromatography on silica gel using a gradient of ethyl acetate (10 - 40%) in heptane to give 0.635 g (68%) of the title compound as a white solid.
$^1H$ NMR ($CDCl_3$) $\delta$ 1.31 (6H, s); 3.11 (2H, s); 6.70 (3H, m); 7.19 (2H, t).
ESI/APCI(+): 166 (M+H).

INTERMEDIATE 14 - PREPARATION OF 4-Ethyl-6,6-dimethylmorpholin-2-one.

The compound was prepared according to the procedure B from 40% glyoxal solution (4.450 mL; 39.160 mmol) and 1-(ethylamino)-2-methylpropan-2-ol (4.420 g; 37.717 mmol) in toluene (27 mL). Purification by distillation under reduced pressure furnished 4.720 g (80%) of the title compound as a colourless liquid.
$^1H$ NMR ($CDCl_3$) $\delta$ 1.09 (3H, t); 1.43 (6H, s); 2.44 (2H, q); 2.47 (2H, s); 3.21 (2H, s).
ESI/APCI(+): 158 (M+H); 180 (M+Na).

INTERMEDIATE 15 - PREPARATION OF 4-Ethyl-6-phenylmorpholin-2-one.

To a mixture of 40% glyoxal solution (0.353 mL; 3.106 mmol) in toluene (1 mL) cooled at 10 °C was added 2-(ethylamino)-1-phenylethanol (0.500 g; 3.026 mmol) portionwise. Toluene (1 mL) was added to obtain a clear solution. After 3.5 h at 10 °C, the reaction mixture was heated at reflux to azeotrope out solvents. The residue was partitioned between ethyl acetate and water. The aqueous phase was extracted with ethyl acetate. The organic phases were combined, washed with brine, dried over sodium sulfate, filtered and concentrated. The residue was purified by flash chromatography on silica gel using a gradient of ethyl acetate (20 - 50%) in heptane to afford 0.390 g (63%) of the title compound as a yellow oil.
$^1H$ NMR ($CDCl_3$) $\delta$ 1.12 (3H, t); 2.4-2.6 (3H, m); 3.05 (2H, m); 3.13 (1H, m); 3.75 (1H, m); 5.52 (1H, dd); 7.3-7.4 (5H, m).
ESI/APCI(+): 206 (M+H).

INTERMEDIATE 16 - PREPARATION OF (S)-4-Ethyl-6-phenylmorpholin-2-one.

To a mixture of 40% glyoxal solution (0.548 mL; 4.822 mmol) in toluene (2 mL) cooled at 10 °C was added a solution of (*S*)-2-(ethylamino)-1-phenylethanol (0.775 g; 4.690 mmol) in toluene (2 mL). After 2 h at 10 °C, the reaction mixture was heated at reflux to azeotrope out solvents. The residue was partitioned between ethyl acetate and water. The aqueous phase was extracted with ethyl acetate. The organic phases were combined, washed with brine, dried over sodium sulfate, filtered and concentrated under reduced pressure. The residue was purified by flash chromatography on silica gel using a gradient of ethyl acetate (20 - 50%) in heptane to afford 0.486 g (50%) of the title compound as a yellow oil.
ESI/APCI(+): 206 (M+H); 228 (M+Na).

INTERMEDIATE 17 - PREPARATION OF (R)-4-Ethyl-6-phenylmorpholin-2-one.

To a mixture of 40% glyoxal solution (0.636 mL; 5.597 mmol) in toluene (2 mL) cooled at 10 °C was added a solution of (*R*)-2-(ethylamino)-1-phenylethanol (0.900 g; 5.447 mmol) in toluene (2.6 mL). After 2 h at 10 °C, the reaction mixture was heated at reflux to azeotrope out solvents. The residue was partitioned between ethyl acetate and water. The organic phase was washed with brine, dried over sodium sulfate, filtered and concentrated under reduced pressure. The residue was purified by flash chromatography on silica gel using a gradient of ethyl acetate (20 - 50%) in heptane

to afford 0.638 g (57%) of the title compound as an orange oil.
ESI/APCI(+): 206 (M+H).

INTERMEDIATE 18 - PREPARATION OF (*S*)-4-Ethyl-6-methylmorpholin-2-one.

The compound was prepared according to the procedure B from 40% glyoxal solution (0.790 mL; 6.952 mmol) and (*S*)-1-(ethylamino)propan-2-ol (0.692 g; 6.708 mmol) in toluene (4 mL). Purification by distillation under reduced pressure furnished 0.508 g (53%) of the title compound as a colourless liquid.
ESI/APCI(+): 144 (M+H).

INTERMEDIATE 19 - PREPARATION OF (*R*)-4-Ethyl-6-methylmorpholin-2-one.

The compound was prepared according to the procedure B from 40% glyoxal solution (1.600 mL; 14.080 mmol) and (*R*)-1-(ethylamino)propan-2-ol (1.406 g; 13.629 mmol) in toluene (8.5 mL). Purification by distillation under reduced pressure furnished 1.006 g (52%) of the title compound as a colourless liquid.
ESI/APCI(+): 144 (M+H).

INTERMEDIATE 20 - PREPARATION OF 4-Ethyl-6-methyl-6-phenylmorpholin-2-one.

The compound was prepared according to the procedure B from 40% glyoxal solution (0.326 mL; 2.869 mmol) and 1-(ethylamino)-2-phenylpropan-2-ol (0.500 g; 2.789 mmol) in toluene (2.3 mL). Purification by flash chromatography on silica gel using a gradient of ethyl acetate (10 - 40%) in heptane furnished 0.275 g (45%) of the title compound as a yellow oil.
$^1$H NMR ($CDCl_3$) $\delta$ 1.07 (3H, t); 1.75 (3H, s); 2.45 (2H, m); 2.82 (2H, m); 3.18 (1H, d); 3.42 (1H, m); 7.3-7.4 (5H, m).
ESI/APCI(+): 220 (M+H); 242 (M+Na).

INTERMEDIATE 21 - PREPARATION OF 4-Ethyl-5,5,6,6-tetramethylmorpholin-2-one

The compound was prepared according to the procedure B from 40% glyoxal solution (1.040 mL; 9.152 mmol) and 3-(ethylamino)-2,3-dimethylbutan-2-ol (1.268 g; 8.730 mmol) in toluene (7.0 mL). Purification by distillation under reduced pressure furnished 0.826 g (51%) of the title compound as a yellow liquid.
$^1$H NMR ($CDCl_3$) $\delta$ 1.05 (9H, m); 1.41 (3H, s); 2.49 (2H, q); 3.37 (2H, s).
ESI/APCI(+): 186 (M+H); 208 (M+Na).

INTERMEDIATE 22 - PREPARATION OF 4-Ethyl-5,6,6-trimethylmorpholin-2-one

The compound was prepared according to the procedure B from 40% glyoxal solution (2.800 mL; 24.640 mmol) and 3-(ethylamino)-2-methylbutan-2-ol (3.050 g; 23.243 mmol) in toluene (17.5 mL). Purification by distillation under reduced pressure furnished 2.772 g (70%) of the title compound as a yellow liquid.
$^1$H NMR ($CDCl_3$) $\delta$ 1.04 (6H, m); 1.34 (3H, s); 1.48 (3H, s); 2.47 (1H, m); 2.65 (2H, m); 3.31 (2H, m).
ESI/APCI(+): 172 (M+H); 194 (M+Na).

INTERMEDIATE 23 - PREPARATION OF 4,6,6-Triethylmorpholin-2-one

The compound was prepared according to the procedure B from 40% glyoxal solution (1.120 mL; 9.856 mmol) and 3-((ethylamino)methyl)pentan-3-ol (1.340 g; 9.226 mmol) in toluene (7 mL). Purification by distillation under reduced pressure furnished 0.654 g (38%) of the title compound as a colourless liquid.
$^1$H NMR ($CDCl_3$) $\delta$ 0.92 (6H, t); 1.09 (3H, t); 1.78 (4H, m); 2.42 (2H, q); 2.47 (2H, s); 3.20 (2H, s).
ESI/APCI(+): 186 (M+H); 208 (M+Na).

INTERMEDIATE 24 - PREPARATION OF 4-Benzyl-6,6-dimethylmorpholin-2-one

To a solution of 1-(benzylamino)-2-methylpropan-2-ol (0.500 g; 2.789 mmol) in acetonitrile (8 mL) were added diisopropylethylamine (0.487 mL; 2.788 mmol) and ethyl bromoacetate (0.309 mL; 2.787 mmol). The reaction mixture was stirred at room temperature for 5h and at 55 °C for 2.5 h. The solvents were evaporated under reduced pressure. The residue was partitioned between ethyl acetate and a saturated sodium bicarbonate solution. The phases were separated. The organic phase was washed with water and brine, dried over sodium sulfate, filtered and concentrated to give ethyl 2-(benzyl(2-hydroxy-2-methylpropyl)amino)acetate which was used in the next step without further purification.

To a solution of 2-(benzyl(2-hydroxy-2-methylpropyl)amino)acetate (2.789 mmol) in toluene (65 mL) was added p-toluenesulfonic acid monohydrate (0.054 g; 0.284 mmol). The reaction mixture was refluxed with a Dean-Stark apparatus (until 30 mL of solvents was distilled) and then concentrated to dryness. The residue was dissolved in ethyl acetate and was washed with a saturated sodium bicarbonate solution, water and brine. The organic phase was dried over sodium sulfate, filtered and concentrated. The residue was purified by flash chromatography on silica gel using a gradient of ethyl acetate (10-90%) in heptane to give 0.517 g (85%) of the title compound as a colourless oil.

$^1$H NMR ($CDCl_3$) $\delta$ 1.42 (6H, s); 2.47 (2H, s); 3.27 (2H, s); 3.56 (2H, s); 7.33 (5H, m).

ESI/APCI(+): 220 (M+H); 242 (M+Na).

INTERMEDIATE 25 - PREPARATION OF 6,6-Dimethyl-4-phenylmorpholin-2-one

To a mixture of 2-methyl-1-(phenylamino)propan-2-ol (0.100 g; 0.605 mmol) and potassium carbonate (0.101 g; 0.731 mmol) in DMF (2 mL) was added ethyl bromoacetate (0.162 mL; 1.461 mmol). The reaction mixture was heated at 110 °C for 7 h. After cooling to room temperature, the reaction mixture was concentrated. The residue was partitioned between ethyl acetate and a saturated sodium bicarbonate solution. The phases were separated. The organic phase was washed with water and brine, dried over sodium sulfate, filtered and concentrated. The residue was purified by flash chromatography on silica gel using a gradient of ethyl acetate (10 - 40%) in heptane to give 0.107 g (86%) of the title compound as a pink oil.

$^1$H NMR ($CDCl_3$) $\delta$ 1.53 (6H, s); 3.32 (2H, s); 4.02 (2H, s); 6.82 (2H, d); 6.91 (1H, t); 7.32 (2H, t).

ESI/APCI(+): 206 (M+H).

INTERMEDIATE 26 - PREPARATION OF *tert*-Butyl 2-(4-ethyl-6,6-dimethyl-2-oxomorpholin-3-yl)acetate

The compound was prepared according to the procedure C from 4-ethyl-6,6-dimethylmorpholin-2-one (2.000 g; 12.722 mmol), 1N lithium bis(trimethylsilyl)amide solution in tetrahydrofuran (12.730 mL; 12.730 mmol) and *tert*-butyl bromoacetate (2.070 mL; 14.115 mmol) in tetrahydrofuran (80 mL). Purification by flash chromatography on silica gel using a gradient of ethyl acetate (10 - 40%) in heptane furnished 2.611 g (75%) of the title compound as a white solid.

$^1$H NMR ($CDCl_3$) $\delta$ 1.05 (3H, t); 1.38 (3H, s); 1.46 (9H, s); 1.50 (3H, s); 2.30-2.40 (2H, m); 2.75-2.85 (3H, m); 3.00 (1H, dd); 3.32 (1H, m).

ESI/APCI(+): 272 (M+H); 294 (M+Na).

INTERMEDIATE 27 - PREPARATION OF Methyl 2-(4-ethyl-2-oxo-6-phenylmorpholin-3-yl)acetate

To a solution of 4-ethyl-6-phenylmorpholin-2-one (0.360 g; 1.754 mmol) in tetrahydrofuran (11 mL) cooled at -70 °C was added dropwise a 1N lithium bis(trimethylsilyl)amide solution in tetrahydrofuran/ethylbenzene (1.760 mL; 1.760 mmol). After 1.5 h at -70 °C, methyl bromoacetate (0.184 mL; 1.944 mmol) was added dropwise and the reaction mixture was stirred at -70 °C for 3 h. The reaction was quenched by addition of a saturated ammonium chloride solution. After warming to room temperature, the reaction mixture was extracted with ethyl acetate. The organic phase was washed with a saturated sodium bicarbonate solution, water and brine, dried over sodium sulfate, filtered and concentrated. The residue was purified by flash chromatography on silica gel using a gradient of ethyl acetate (10 - 50%) in heptane to afford 0.110 g (23%) of the title compound as a yellow oil.

ESI/APCI(+): 278 (M+H); 300 (M+Na).

INTERMEDIATE 28 - PREPARATION OF Methyl 2-((6S)-4-ethyl-2-oxo-6-phenylmorpholin-3-yl)acetate

To a solution of (S)-4-ethyl-6-phenylmorpholin-2-one (0.483 g; 2.353 mmol) in tetrahydrofuran (14.5 mL) cooled at -70 °C was added dropwise a 1N lithium bis(trimethylsilyl)amide solution in tetrahydrofuran/ethylbenzene (2.360 mL; 2.360 mmol). After 1.5 h at -70 °C, methyl bromoacetate (0.248 mL; 2.620 mmol) was added dropwise and the reaction mixture was stirred at -70 °C for 2.5 h. The reaction was quenched by addition of a saturated ammonium chloride solution. After warming to room temperature, the reaction mixture was extracted with ethyl acetate. The organic phase was washed with a saturated sodium bicarbonate solution, water and brine, dried over sodium sulfate, filtered and concentrated under reduced pressure. The residue was purified by flash chromatography on silica gel using a gradient of ethyl acetate (10-50%) in heptane to afford 0.470 g (72%) of the title compound as a yellow oil.

ESI/APCI(+): 278 (M+H); 300 (M+Na).

INTERMEDIATE 29 - PREPARATION OF Methyl 2-((6*R*)-4-ethyl-2-oxo-6-phenylmorpholin-3-yl)acetate

To a solution of (*R*)-4-ethyl-6-phenylmorpholin-2-one (0.635 g; 3.094 mmol) in tetrahydrofuran (19 mL) cooled at -70 °C was added dropwise a 1N lithium bis(trimethylsilyl)amide solution in tetrahydrofuran/ethylbenzene (3.100 mL; 3.100 mmol). After 1 h at -70 °C, methyl bromoacetate (0.325 mL; 3.433 mmol) was added dropwise and the reaction mixture was stirred at -70 °C for 3 h. The reaction was quenched by addition of a saturated ammonium chloride solution. After warming to room temperature, the reaction mixture was extracted with ethyl acetate. The organic phase was washed with a saturated sodium bicarbonate solution, water and brine, dried over sodium sulfate, filtered and concentrated under reduced pressure. The residue was purified by flash chromatography on silica gel using a gradient of ethyl acetate (10 - 50%) in heptane to afford 0.479 g (56%) of the title compound as a yellow oil.
ESI/APCI(+): 278 (M+H); 300 (M+Na).

INTERMEDIATE 30 - PREPARATION OF Methyl 2-((6*S*)-4-ethyl-6-methyl-2-oxomorpholin-3-yl)acetate

The compound was prepared according to the procedure C from (S)-4-ethyl-6-methylmorpholin-2-one (0.500 g; 3.492 mmol), 1N lithium bis(trimethylsilyl)amide solution in tetrahydrofuran/ethylbenzene (3.490 mL; 3.490 mmol) and methyl bromoacetate (0.349 mL; 3.687 mmol) in tetrahydrofuran (23 mL). Purification by flash chromatography on silica gel using a gradient of ethyl acetate (30 - 80%) in heptane furnished 0.501 g (67%) of the title compound as a yellow oil.
ESI/APCI(+): 216 (M+H); 238 (M+Na).

INTERMEDIATE 31 - PREPARATION OF Methyl 2-((6R)-4-ethyl-6-methyl-2-oxomorpholin-3-yl)acetate

The compound was prepared according to the procedure C from (R)-4-ethyl-6-methylmorpholin-2-one (0.500 g; 3.492 mmol), 1N lithium bis(trimethylsilyl)amide solution in tetrahydrofuran/ethylbenzene (3.490 mL; 3.490 mmol) and methyl bromoacetate (0.349 mL; 3.687 mmol) in tetrahydrofuran (23 mL). Purification by flash chromatography on silica gel using a gradient of ethyl acetate (30 - 80%) in heptane furnished 0.395 g (53%) of the title compound as a yellow oil.
ESI/APCI(+): 216 (M+H); 238 (M+Na).

INTERMEDIATE 32 - PREPARATION OF *tert*-Butyl 2-(4-ethyl-6-methyl-2-oxo-6-phenylmorpholin-3-yl)acetate

To a solution of 4-ethyl-6-methyl-6-phenylmorpholin-2-one (0.270 g; 1.231 mmol) in tetrahydrofuran (8 mL) cooled at -70 °C was added dropwise a 1N lithium bis(trimethylsilyl)amide solution in tetrahydrofuran (1.230 mL; 1.230 mmol). After 1 h at-70 °C, *tert*-butyl bromoacetate (0.200 mL; 1.364 mmol) was added dropwise and the reaction mixture was stirred at -70 °C for 4 h. The reaction was quenched by addition of a saturated ammonium chloride solution. After warming to room temperature, the reaction mixture was diluted with ethyl acetate. The phases were separated. The organic phase was washed with water and brine, dried over sodium sulfate, filtered and concentrated under reduced pressure. The residue was purified by flash chromatography on silica gel using a gradient of ethyl acetate (10 - 30%) in heptane to afford 0.334 g (81%) of the title compound as a yellow oil.
ESI/APCI(+): 334 (M+H); 356 (M+Na), 689 (2M+Na).

INTERMEDIATE 33 - PREPARATION OF *tert*-Butyl 2-(4-ethyl-5,5,6,6-tetramethyl-2-oxomorpholin-3-yl)acetate

The compound was prepared according to the procedure C from 4-ethyl-5,5,6,6-tetramethylmorpholin-2-one (0.150 g; 0.810 mmol), 1N lithium bis(trimethylsilyl)amide solution in tetrahydrofuran (0.820 mL; 0.820 mmol) and *tert*-butyl bromoacetate (0.131 mL; 0.893 mmol) in tetrahydrofuran (5 mL). Purification by flash chromatography on silica gel using a gradient of ethyl acetate (10 - 40%) in heptane furnished 0.165 g (68%) of the title compound as a yellow oil.
$^1H$ NMR ($CDCl_3$) $\delta$ 0.51 (6H, m); 0.57 (3H, s); 0.75 (3H, s); 0.89 (9H, s); 0.92 (3H, s); 1.65 (1H, m); 2.01 (1H, m); 2.06 (1H, dd); 2.2-2.4 (2H, m); 2.96 (1H, m).
ESI/APCI(+): 300 (M+H); 322 (M+Na); 621 (2M+Na).

INTERMEDIATE 34 - PREPARATION OF *tert*-Butyl 2-(4-ethyl-5,6,6-trimethyl-2-oxomorpholin-3-yl)acetate

The compound was prepared according to the procedure C from 4-ethyl-5,6,6-trimethylmorpholin-2-one (0.300 g; 1.752 mmol), 1N lithium bis(trimethylsilyl)amide solution in tetrahydrofuran (1.800 mL; 1.800 mmol) and *tert*-butyl bromoacetate (0.285 mL; 1.943 mmol) in tetrahydrofuran (11 mL). Purification by flash chromatography on silica gel using a gradient of ethyl acetate (10 - 40%) in heptane furnished 0.399 g (80%) of the title compound as a yellow oil.
ESI/APCI(+): 286 (M+H); 308 (M+Na); 593 (2M+Na).

INTERMEDIATE 35 - PREPARATION OF *tert*-Butyl 2-(4,6,6-triethyl-2-oxomorpholin-3-yl)acetate

To a solution of 4,6,6-triethylmorpholin-2-one (0.300 g; 1.619 mmol) in tetrahydrofuran (10 mL) cooled at -70 °C was added dropwise a 1N lithium bis(trimethylsilyl)amide solution in tetrahydrofuran (1.650 mL; 1.650 mmol). After 1 h at -70 °C, *tert*-butyl bromoacetate (0.264 mL; 1.800 mmol) was added dropwise and the reaction mixture was stirred at -70 °C for 4.5 h. The reaction was quenched by addition of a saturated ammonium chloride solution. After warming to room temperature, the reaction mixture was diluted with ethyl acetate. The phases were separated. The organic phase was washed with water and brine, dried over sodium sulfate, filtered and concentrated under reduced pressure. The residue was purified by flash chromatography on silica gel using a gradient of ethyl acetate (10 - 40%) in heptane to afford 0.369 g (76%) of the title compound as a yellow oil.
$^1$H NMR ($CDCl_3$) $\delta$ 0.87 (3H, t); 0.94 (3H, t); 1.05 (3H, t); 1.45 (9H, s); 1.64 (2H, m); 1.88 (2H, m); 2.30 (2H, m); 2.7-3.0 (4H, m); 3.35 (1H, m).
ESI/APCI(+): 300 (M+H); 322 (M+Na); 621 (2M+Na).

INTERMEDIATE 36 - PREPARATION OF *tert*-Butyl 2-(4-ethyl-6,6-dimethyl-2-oxomorpholin-3-yl)-2-methylpropanoate

The compound was prepared according to the procedure C from 4-ethyl-6,6-dimethylmorpholin-2-one (0.295 g; 1.878 mmol), 1N lithium bis(trimethylsilyl)amide solution in tetrahydrofuran (2.0 mL; 2.0 mmol) and *tert*-butyl 2-bromoisobutyrate (0.360 mL; 1.904 mmol) in tetrahydrofuran (12 mL). Purification by flash chromatography on silica gel using a gradient of ethyl acetate (5 - 40%) in heptane furnished 0.053 g (9%) of the title compound as a gum.
$^1$H NMR ($CDCl_3$) $\delta$ 1.10 (3H, t); 1.14 (3H, s); 1.26 (3H, s); 1.36 (3H, s); 1.43 (3H, s); 1.47 (9H, s); 2.69-2.96 (4H, m); 3.85 (1 H, s).
ESI/APCI(+): 300 (M+H), 322 (M+Na).

INTERMEDIATE 37 - PREPARATION OF *tert*-Butyl 2-(4-benzyl-6,6-dimethyl-2-oxomorpholin-3-yl)acetate

The compound was prepared according to the procedure C from 4-benzyl-6,6-dimethylmorpholin-2-one (0.507 g; 2.312 mmol), 1N lithium bis(trimethylsilyl)amide solution in tetrahydrofuran (2.310 mL; 2.310 mmol) and *tert*-butyl bromoacetate (0.376 mL; 2.564 mmol) in tetrahydrofuran (14 mL). Purification by flash chromatography on silica gel using a gradient of ethyl acetate (10 - 90%) in heptane furnished 0.658 g (85%) of the title compound as a colourless oil.
$^1$H NMR ($CDCl_3$) $\delta$ 1.29 (3H, s); 1.45 (3H, s); 1.48 (9H, s); 2.24 (1H, d); 2.66 (1H, d); 2.95 (1 H, dd); 3.14 (2H, m); 3.39 (1 H, m); 4.08 (1 H, d); 7.31 (5H, m).
ESI/APCI(+): 334 (M+H); 356 (M+Na); 689 (2M+Na).

INTERMEDIATE 38 - PREPARATION OF *tert*-Butyl 2-(6,6-dimethyl-2-oxo-4-phenylmorpholin-3-yl)acetate

To a solution of 6,6-dimethyl-4-phenylmorpholin-2-one (0.100 g; 0.487 mmol) in tetrahydrofuran (3 mL) cooled at -70 °C was added dropwise a 1N lithium bis(trimethylsilyl)amide solution in tetrahydrofuran (0.490 mL; 0.490 mmol). After 1 h at-70 °C, *tert*-butyl bromoacetate (0.162 mL; 1.105 mmol) was added dropwise and the reaction mixture was stirred at -70 °C for 3.5 h. The reaction was quenched by addition of a saturated ammonium chloride solution. After warming to room temperature, the reaction mixture was diluted with ethyl acetate. The phases were separated. The organic phase was washed with water and brine, dried over sodium sulfate, filtered and concentrated under reduced pressure. The residue was purified by flash chromatography on silica gel using a gradient of ethyl acetate (10 - 40%) in heptane to afford 0.121 g (78%) of the title compound as a yellow oil.
$^1$H NMR ($CDCl_3$) $\delta$ 1.37 (3H, s); 1.41 (9H, s); 1.53 (3H, s); 2.78 (2H, m); 3.47 (2H, q); 4.67 (1H, m); 6.88 (3H, m); 7.29 (2H, m).
ESI/APCI(+): 320 (M+H); 342 (M+Na); 661 (2M+Na).

INTERMEDIATE 39 - PREPARATION OF 2-(4-Ethyl-6,6-dimethyl-2-oxomorpholin-3-yl)acetic acid

The compound was prepared according to the procedure D from *tert*-butyl 2-(4-ethyl-6,6-dimethyl-2-oxomorpholin-3-yl)acetate (0.300 g; 1.106 mmol) and trifluoroacetic acid (1.7 mL) in dichloromethane (5 mL). Crude 2-(4-ethyl-6,6-dimethyl-2-oxomorpholin-3-yl)acetic acid was used in the next step without further purification.
ESI/APCI(+): 216 (M+H); 238 (M+Na).

INTERMEDIATE 40 - PREPARATION OF 2-(4-Ethyl-6-methyl-2-oxo-6-phenylmorpholin-3-yl)acetic acid

The compound was prepared according to the procedure D from *tert*-butyl 2-(4-ethyl-6-methyl-2-oxo-6-phenylmorpholin-3-yl)acetate (0.100 g; 0.300 mmol) and trifluoroacetic acid (0.465 mL) in dichloromethane (1.5 mL).
Crude 2-(4-ethyl-6-methyl-2-oxo-6-phenylmorpholin-3-yl)acetic acid was used in the next step without further purification.
ESI/APCI(+): 278 (M+H); 290 (M+Na).
ESI/APCI(-): 276 (M-H).

INTERMEDIATE 41 - PREPARATION OF 2-(4-Ethyl-5,5,6,6-tetramethyl-2-oxomorpholin-3-yl)acetic acid

The compound was prepared according to the procedure D from *tert*-butyl 2-(4-ethyl-5,5,6,6-tetramethyl-2-oxomorpholin-3-yl)acetate (0.158 g; 0.528 mmol) and trifluoroacetic acid (0.850 mL) in dichloromethane (2.5 mL).
Crude 2-(4-ethyl-5,5,6,6-tetramethyl-2-oxomorpholin-3-yl)acetic acid was used in the next step without further purification.
ESI/APCI(+): 244 (M+H); 266 (M+Na).
ESI/APCI(-): 242 (M-H).

INTERMEDIATE 42 - PREPARATION OF 2-(4-Ethyl-5,6,6-trimethyl-2-oxomorpholin-3-yl)acetic acid

The compound was prepared according to the procedure D from *tert*-butyl 2-(4-ethyl-5,6,6-trimethyl-2-oxomorpholin-3-yl)acetate (0.100 g; 0.350 mmol) and trifluoroacetic acid (0.570 mL) in dichloromethane (1.6 mL).
Crude 2-(4-ethyl-5,6,6-trimethyl-2-oxomorpholin-3-yl)acetic acid was used in the next step without further purification.
ESI/APCI(+): 230 (M+H); 252 (M+Na).
ESI/APCI(-): 228 (M-H).

INTERMEDIATE 43 - PREPARATION OF 2-(4,6,6-Triethyl-2-oxomorpholin-3-yl)acetic acid

The compound was prepared according to the procedure D from *tert*-butyl 2-(4,6,6-triethyl-2-oxomorpholin-3-yl)acetate (0.100 g; 0.334 mmol) and trifluoroacetic acid (0.490 mL) in dichloromethane (1.5 mL).
Crude 2-(4,6,6-triethyl-2-oxomorpholin-3-yl)acetic acid was used in the next step without further purification.
ESI/APCI(+): 244 (M+H); 266 (M+Na).
ESI/APCI(-): 242 (M-H).

INTERMEDIATE 44 - PREPARATION OF 2-(4-Ethyl-6,6-dimethyl-2-oxomorpholin-3-yl)-2-methylpropanoic acid

The compound was prepared according to the procedure D from *tert*-butyl 2-(4-ethyl-6,6-dimethyl-2-oxomorpholin-3-yl)-2-methylpropanoate (0.053 g; 0.177 mmol) and trifluoroacetic acid (0.285 mL) in dichloromethane (2 mL).
Crude 2-(4-ethyl-6,6-dimethyl-2-oxomorpholin-3-yl)-2-methylpropanoic acid was used in the next step without further purification.
ESI/APCI(+): 244 (M+H); 266 (M+Na).
ESI/APCI(-): 242 (M-H).

INTERMEDIATE 45 - PREPARATION OF 2-(4-Benzyl-6,6-dimethyl-2-oxomorpholin-3-yl)acetic acid

The compound was prepared according to the procedure D from *tert*-butyl 2-(4-benzyl-6,6-dimethyl-2-oxomorpholin-3-yl)acetate (0.646 g; 1.937 mmol) and trifluoroacetic acid (3 mL) in dichloromethane (8.7 mL):

Crude 2-(4-benzyl-6,6-dimethyl-2-oxomorpholin-3-yl)acetic acid was used in the next step without further purification.
ESI/APCI(+): 278 (M+H); 300 (M+Na).
ESI/APCI(-): 276 (M-H).

INTERMEDIATE 46 - PREPARATION OF 2-(6,6-Dimethyl-2-oxo-4-phenylmorpholin-3-yl)acetic acid

The compound was prepared according to the procedure D from *tert*-butyl 2-(6,6-dimethyl-2-oxo-4-phenylmorpholin-3-yl)acetate (0.115 g; 0.360 mmol) and trifluoroacetic acid (0.760 mL) in dichloromethane (1.7 mL).
Crude 2-(6,6-dimethyl-2-oxo-4-phenylmorpholin-3-yl)acetic acid was used in the next step without further purification.
ESI/APCI(+): 264 (M+H); 286 (M+Na).

EXAMPLE 6 - PREPARATION OF 2-(4-Ethyl-6,6-dimethyl-2-oxomorpholin-3-yl)-*N*-(1-methyl-1*H*-indol-5-yl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.378 mmol), HATU (0.127 g; 0.334 mmol) and 1-methyl-1*H*-indol-5-amine (0.053 g; 0.363 mmol) in DMF (2 mL). Purification by flash chromatography on silica gel using a gradient of ethyl acetate (20 - 80%) in heptane furnished 0.085 g (90%) of the title compound as a yellow foam.
$^1$H NMR ($CDCl_3$) $\delta$ 1.10 (3H, t); 1.41 (3H, s); 1.46 (3H, s); 2.43 (1H, d); 2.52 (1H, m); 2.9-3.1 (4H, m); 3.45 (1 H, m); 3.76 (3H, s); 6.42 (1H, d); 7.03 (1 H, d); 7.23 (2H, s); 7.81 (1H, s); 8.45 (1 H, brs).
ESI/APCI(+): 344 (M+H); 366 (M+Na); 709 (2M+Na).
ESI/APCI(-): 342 (M-H).

EXAMPLE 7 - PREPARATION OF *N*-(4-Chlorobenzyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.271 mmol), diisopropylethylamine (0.235 mL; 1.346 mmol), HATU (0.125 g; 0.329 mmol) and 4-chlorobenzylamine (0.043 mL; 0.352 mmol) in DMF (2 mL). Purification by flash chromatography on silica gel using a gradient of ethyl acetate (30 - 70%) in heptane furnished 0.070 g (76%) of the title compound as a yellow solid.
$^1$H NMR ($CDCl_3$) $\delta$ 1.05 (3H, t); 1.35 (3H, s); 1.38 (3H, s); 2.38 (1H, d); 2.46 (1H, m); 2.7-3.0 (4H, m); 3.46 (1H, m); 4.40 (2H, qd); 6.82 (1H, brs); 7.20-7.30 (4H, m).
ESI/APCI(+): 339 (M+H); 361 (M+Na); 701 (2M+Na).
ESI/APCI(-): 337 (M-H).

EXAMPLE 8 - PREPARATION OF *N*-(2,3-Dihydro-1*H*-inden-1-yl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.271 mmol), diisopropylethylamine (0.235 mL; 1.346 mmol), HATU (0.125 g; 0.329 mmol) and 1-aminoindane (0.041 mL; 0.320 mmol) in DMF (2 mL). Purification by flash chromatography on silica gel using a gradient of ethyl acetate (30 - 70%) in heptane furnished 0.065 g (73%) of the title compound as a beige powder.
ESI/APCI(+): 331 (M+H); 353 (M+Na); 683 (2M+Na).
ESI/APCI(-): 329 (M-H).

EXAMPLE 9 - PREPARATION OF *N*-(Benzo[*d*]thiazol-5-yl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.271 mmol), diisopropylethylamine (0.235 mL; 1.346 mmol), HATU (0.125 g; 0.329 mmol) and 1,3-benzothiazol-5-amine (0.047 g; 0.313 mmol) in DMF (2 mL). Purification by flash chromatography on silica gel using a gradient of ethyl acetate (20 - 80%) in heptane furnished 0.075 g (80%) of the title compound as a white foam.
$^1$H NMR ($CDCl_3$) $\delta$ 1.14 (3H, t); 1.42 (3H, s); 1.47 (3H, s); 2.45 (1H, d); 2.53 (1H, m); 2.9-3.2 (4H, m); 3.45 (1H, m); 7.69 (1H, dd); 7.86 (1H, d); 8.27 (1H, d); 8.94 (1H, brs); 8.99 (1H, s).
ESI/APCI(+): 348 (M+H); 370 (M+Na); 717 (2M+Na).
ESI/APCI(-): 346 (M-H).

EXAMPLE 10 - PREPARATION OF 2-(4-Ethyl-6,6-dimethyl-2-oxomorpholin-3-yl)-*N*-(1,2,3,4-tetrahydronaphthalen-1-yl) acetamide

The compound was prepared according to the procedure E from 2-(4-ethyl-6,6-dimethyl-2-oxomorpholin-3-yl)acetic acid (0.276 mmol), diisopropylethylamine (0.235 mL; 1.346 mmol), HATU (0.127 g; 0.334 mmol) and 1,2,3,4-tetrahydro-1-naphtylane (0.046 mL; 0.319 mmol) in DMF (2 mL). Purification by flash chromatography on silica gel using a gradient of ethyl acetate (30 - 70%) in heptane followed by recrystallization from ethyl acetate and heptane furnished 0.045 g (47%) of the title compound as a white powder.
ESI/APCI(+): 345 (M+H); 367 (M+Na); 711 (2M+Na).
ESI/APCI(-): 343 (M-H).

EXAMPLE 11 - PREPARATION OF *N*-Benzyl-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.276 mmol), diisopropylethylamine (0.235 mL; 1.346 mmol), HATU (0.127 g; 0.334 mmol) and benzylamine (0.035 mL; 0.320 mmol) in DMF (2 mL). Purification by flash chromatography on silica gel using a gradient of ethyl acetate (30 - 70%) in heptane furnished 0.039 g (46%) of the title compound as a yellow oil.
$^1$H NMR ($CDCl_3$) $\delta$ 1.04 (3H, t); 1.35 (3H, s); 1.37 (3H, s); 2.37 (1H, d); 2.45 (1H, m); 2.7-3.0 (4H, m); 3.37 (1H, m); 4.45

(2H, qd); 6.75 (1H, brs); 7.2-7.35 (4H, m).
ESI/APCI(+): 305 (M+H); 327 (M+Na); 631 (2M+Na).
ESI/APCI(-): 303 (M-H).

EXAMPLE 12 - PREPARATION OF 2-(4-Ethyl-6,6-dimethyl-2-oxomorpholin-3-yl)-N-(pyridin-3-yl)acetamide

The compound was prepared according to the procedure E from 2-(4-ethyl-6,6-dimethyl-2-oxomorpholin-3-yl)acetic acid (0.276 mmol), diisopropylethylamine (0.235 mL; 1.346 mmol), HATU (0.127 g; 0.334 mmol) and 3-aminopyridine (0.030 g; 0.319 mmol) in DMF (2 mL). Purification by flash chromatography on silica gel using a gradient of ethyl acetate (30 - 70%) in heptane followed by crystallization from ethyl acetate and heptane furnished 0.029 g (36%) of the title compound as white needles.
$^1$H NMR ($CDCl_3$) $\delta$ 1.13 (3H, t); 1.42 (3H, s); 1.47 (3H, s); 2.44 (1H, d); 2.49 (1H, m); 2.9-3.1 (4H, m); 3.42 (1H, m); 7.26 (1H, m); 8.16 (1H, m); 8.33 (1H, m); 8.54 (1H, d); 8.92 (1H, s).
ESI/APCI(+): 292 (M+H); 314 (M+Na); 605 (2M+Na).
ESI/APCI(-): 290 (M-H).

EXAMPLE 13 - PREPARATION OF *N*-(1-Benzylpiperidin-4-yl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.276 mmol), diisopropylethylamine (0.235 mL; 1.346 mmol), HATU (0.127 g; 0.334 mmol) and 4-amino-1-benzylpiperidine (0.066 mL; 0.322 mmol) in DMF (2 mL). Purification by flash chromatography on silica gel using a gradient of methanol (0 - 10%) in dichloromethane furnished 0.083 g (78%) of the title compound as an orange oil.
$^1$H NMR ($CDCl_3$) $\delta$ 1.06 (3H, t); 1.39 (3H, s); 1.47 (3H, s); 1.86 (4H, m); 2.11 (2H, m); 2.37 (1H, d); 2.44 (1 H, m); 2.6-2.9 (6H, m); 3.32 (1 H, m); 3.49 (2H, s); 3.78 (1 H, m); 6.33 (1 H, d); 7.28 (5H, m).
ESI/APCI(+): 388 (M+H); 410 (M+Na); 797 (2M+Na).
ESI/APCI(-): 386 (M-H).

EXAMPLE 14 - PREPARATION OF 2-(4-Ethyl-6,6-dimethyl-2-oxomorpholin-3-yl)-*N*-(1-phenylethyl)acetamide

The compound was prepared according to the procedure E from 2-(4-ethyl-6,6-dimethyl-2-oxomorpholin-3-yl)acetic acid (0.276 mmol), diisopropylethylamine (0.235 mL; 1.346 mmol), HATU (0.127 g; 0.334 mmol) and $\alpha$-methylbenzylamine (0.042 mL; 0.328 mmol) in DMF (2 mL). Purification by flash chromatography on silica gel using a gradient of ethyl acetate (30 - 70%) in heptane furnished 0.072 g (82%) of the title compound as a yellow oil.
$^1$H NMR ($CDCl_3$) $\delta$ 1.03 (3H, m); 1.35 (6H, m); 1.47 (3H, m); 2.41 (2H, m); 2.7-3.0 (4H, m); 3.34 (1H, m); 5.12 (1H, m); 6.71 (1H, m); 7.29 (5H, m).
ESI/APCI(+): 319 (M+H); 341 (M+Na); 659 (2M+Na).
ESI/APCI(-): 317 (M-H).

EXAMPLE 15 - PREPARATION OF 3-(2-(4-Benzylpiperidin-1-yl)-2-oxoethyl)-4-ethyl-6,6-dimethylmorpholin-2-one

The compound was prepared according to the procedure E from 2-(4-ethyl-6,6-dimethyl-2-oxomorpholin-3-yl)acetic acid (0.276 mmol), diisopropylethylamine (0.235 mL; 1.346 mmol), HATU (0.127 g; 0.334 mmol) and 4-benzylpiperidine (0.059 mL; 0.333 mmol) in DMF (2 mL). Purification by flash chromatography on silica gel using a gradient of ethyl acetate (30 - 70%) in heptane furnished 0.079 g (77%) of the title compound as a yellow oil.
$^1$H NMR ($CDCl_3$) $\delta$ 1.04 (3H, t); 1.39 (3H, s); 1.52 (3H, s); 1.64 (2H, m); 2.35 (2H, m); 2.54 (3H, m); 2.6-3.1 (5H, m); 3.62 (1H, dt); 3.87 (1H, m); 4.61 (1H, m); 7.1-7.3 (5H, m).
ESI/APCI(+): 373 (M+H); 395 (M+Na); 767 (2M+Na).

EXAMPLE 16 - PREPARATION OF 2-(4-Ethyl-6,6-dimethyl-2-oxomorpholin-3-yl)-*N*-(pyridin-4-yl)acetamide

The compound was prepared according to the procedure E from 2-(4-ethyl-6,6-dimethyl-2-oxomorpholin-3-yl)acetic acid (0.276 mmol), diisopropylethylamine (0.235 mL; 1.346 mmol), HATU (0.127 g; 0.334 mmol) and 4-aminopyridine (0.031 g; 0.329 mmol) in DMF (2 mL). Purification by flash chromatography on silica gel using a gradient of methanol (0 - 10%) in dichloromethane furnished 0.060 g (75%) of the title compound as a white powder.
$^1$H NMR ($CDCl_3$) $\delta$ 1.12 (3H, t); 1.42 (3H, s); 1.45 (3H, s); 2.47 (2H, m); 2.9-3.1 (4H, m); 3.41 (1 H, m); 7.46 (2H, d); 8.47 (2H, d); 9.01 (1H, brs).
ESI/APCI(+): 292 (M+H); 314 (M+Na).
ESI/APCI(-): 290 (M-H).

EXAMPLE 17 - PREPARATION OF 2-(4-Ethyl-6,6-dimethyl-2-oxomorpholin-3-yl)-*N*-(quinazolin-2-yl)acetamide

The compound was prepared according to the procedure E from 2-(4-ethyl-6,6-dimethyl-2-oxomorpholin-3-yl)acetic acid (0.276 mmol), diisopropylethylamine (0.235 mL; 1.346 mmol), HATU (0.127 g; 0.334 mmol) and 2-aminoquinazoline (0.048 g; 0.331 mmol) in DMF (2 mL). Purification by flash chromatography on silica gel using a gradient of methanol (0 - 5%) in dichloromethane furnished 0.016 g (17%) of the title compound as a beige solid.
$^1$H NMR ($CDCl_3$) δ 1.10 (3H, t); 1.42 (3H, s); 1.57 (3H, s); 2.46 (2H, m); 2.94 (3H, m); 3.41 (1H, m); 3.5-3.7 (2H, m); 7.52 (1H, m); 7.86 (2H, m); 9.13 (1H, brs); 9.27 (1H, s). ESI/APCI(+): 343 (M+H); 365 (M+Na); 707 (2M+Na).
ESI/APCI(-): 341 (M-H).

EXAMPLE 18 - PREPARATION OF 2-(4-Ethyl-6,6-dimethyl-2-oxomorpholin-3-yl)-*N*-phenethylacetamide

The compound was prepared according to the procedure E from 2-(4-ethyl-6,6-dimethyl-2-oxomorpholin-3-yl)acetic acid (0.276 mmol), diisopropylethylamine (0.235 mL; 1.346 mmol), HATU (0.127 g; 0.334 mmol) and phenethylamine (0.042 mL; 0.333 mmol) in DMF (2 mL). Purification by flash chromatography on silica gel using a gradient of ethyl acetate (30 - 70%) in heptane furnished 0.039 g (44%) of the title compound as a yellow oil.
$^1$H NMR ($CDCl_3$) δ 1.04 (3H, t); 1.16 (3H, s); 1.17 (3H, s); 2.3-2.8 (6H, m); 2:91 (2H, t); 3.76 (2H, m); 4.03 (1H, m); 7.2-7.3 (5H, m).
ESI/APCI(+): 319 (M+H); 341 (M+Na).
ESI/APCI(-): 317 (M-H).

EXAMPLE 19 - PREPARATION OF 4-Ethyl-6,6-dimethyl-3-(2-oxo-2-(4-phenylpiperazin-1-yl)ethyl)morpholin-2-one

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and 1-phenylpiperazine (0.051 mL; 0.334 mmol) in DMF (2 mL). Purification by flash chromatography on silica gel using a gradient of ethyl acetate (30 - 70%) in heptane furnished 0.081 g (82%) of the title compound as a yellow oil.
$^1$H NMR ($CDCl_3$) δ 1.06 (3H, t); 1.40 (3H, s); 1.54 (3H, s); 2.39 (2H, m); 2.7-2.9 (3H, m); 3.09 (1H, d); 3.17 (4H, m); 3.63 (1H, t); 3.67 (2H, m); 3.80 (2H, m); 6.91 (3H, m); 7.29 (2H, m).
ESI/APCI(+): 360 (M+H); 382 (M+Na); 741 (2M+Na).

EXAMPLE 20 - PREPARATION OF 2-(4-Ethyl-6,6-dimethyl-2-oxomorpholin-3-yl)-*N*-(pyridin-2-yl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and 2-aminopyridine (0.032 g; 0.340 mmol) in DMF (2 mL). Purification by flash chromatography on silica gel using a gradient of ethyl acetate (30 - 100%) in heptane furnished 0.030 g (27%) of the title compound as a yellow oil.
$^1$H NMR ($CDCl_3$) δ 1.12 (3H, t); 1.41 (3H, s); 1.53 (3H, s); 2.45 (2H, m); 2.9-3.2 (4H, m); 3.44 (1H, m); 7.02 (1H, m); 7.69 (1H, m); 8.23 (2H, m); 9.42 (1H,s).
ESI/APCI(+): 292 (M+H); 314 (M+Na); 605 (2M+Na).
ESI/APCI(-): 290 (M-H).

EXAMPLE 21 - PREPARATION OF 2-(4-Ethyl-6,6-dimethyl-2-oxomorpholin-3-yl)-*N*-(thiophen-2-ylmethyl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and 2-thiophenemethylamine (0.034 mL; 0.330 mmol) in DMF (2 mL). Purification by flash chromatography on silica gel using a gradient of ethyl acetate (30 - 70%) in heptane furnished 0.042 g (49%) of the title compound as a yellow oil.
$^1$H NMR ($CDCl_3$) δ 1.05 (3H, t); 1.37 (6H, s); 2.37 (1H, d); 2.44 (1H, m); 2.7-3.0 (4H, m); 3.35 (1H, m); 4.60 (2H, d); 6.88 (1H, brs); 6.94 (2H, m); 7.20 (1H, dd).
ESI/APCI(+): 311 (M+H); 333 (M+Na); 643 (2M+Na).
ESI/APCI(-): 309 (M-H).

EXAMPLE 22 - PREPARATION OF 2-(4-Ethyl-6,6-dimethyl-2-oxomorpholin-3-yl)-N-(furan-2-ylmethyl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and furfurylamine (0.029 mL; 0.328 mmol) in DMF (2 mL). Purification by flash chromatography on silica gel using a gradient of

ethyl acetate (30 - 70%) in heptane furnished 0.064 g (79%) of the title compound as a yellow oil.
$^1$H NMR ($CDCl_3$) δ 1.05 (3H, t); 1.38 (3H, s); 1.39 (3H, s); 2.37 (1H, d); 2.43 (1H, m); 2.7-3.0 (4H, m); 3.34 (1H, m); 4.42 (2H, m); 6.21 (1H, m); 6.30 (1 H, m); 6.89 (1H, brs); 7.33 (1H, m).
ESI/APCI(+): 295 (M+H); 317 (M+Na); 611 (2M+Na).
ESI/APCI(-): 293 (M-H).

EXAMPLE 23 - PREPARATION OF 2-(4-Ethyl-6,6-dimethyl-2-oxomorpholin-3-yl)-*N*-methyl-N-phenylacetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and *N*-methylaniline (0.036 mL; 0.332 mmol) in DMF (2 mL). Purification by flash chromatography on silica gel using a gradient of ethyl acetate (30 - 70%) in heptane furnished 0.047 g (56%) of the title compound as a yellow oil.
$^1$H NMR ($CDCl_3$) δ 1.01 (3H, t); 1.36 (3H, s); 1.45 (3H, s); 2.25 (1 H, m); 2.31 (1H, d); 2.5-2.8 (4H, m); 3.28 (3H, s); 3.48 (1 H, t); 7.22 (2H, m); 7.36 (1H, m); 7.42 (2H, m).
ESI/APCI(+): 305 (M+H); 327 (M+Na); 631 (2M+Na).

EXAMPLE 24 - PREPARATION OF 2-(4-Ethyl-6,6-dimethyl-2-oxomorpholin-3-yl)-*N*-(pyrimidin-4-yl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and 4-aminopyrimidine (0.031 g; 0.326 mmol) in DMF (2 mL). Purification by flash chromatography on silica gel using a gradient of ethyl acetate (30 - 100%) in heptane furnished 0.015 g (19%) of the title compound as a yellow solid.
$^1$H NMR ($CDCl_3$) δ 1.14 (3H, t); 1.42 (3H, s); 1.54 (3H, s); 2.75 (2H, m); 2.99 (3H, m); 3.21 (1H, m); 3.41 (1H, m); 8.17 (1H, dd); 8.61 (1H, d); 8.83 (1H, s), 9.84 (1H, brs).
ESI/APCI(+): 293 (M+H); 315 (M+Na).
ESI/APCI(-): 291 (M-H).

EXAMPLE 25 - PREPARATION OF N-(3,5-dimethylisoxazol-4-yl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and 3,5-dimethyl-4-isoxazolamine (0.031 g; 0.276 mmol) in DMF (2 mL). Purification by flash chromatography on silica gel using a gradient of methanol (0 - 10%) in dichloromethane followed by purification by preparative TLC using 5% methanol in dichloromethane as eluent furnished 0.050 g (59%) of the title compound as a yellow solid.
$^1$H NMR ($CDCl_3$) δ 1.12 (3H, t); 1.42 (3H, s); 1.46 (3H, s); 2.18 (3H, s); 2.31 (3H, s); 2.45 (1H, d); 2.51 (1H, m); 2.8-3.1 (4H, m); 3.42 (1H, m); 7.78 (1H, brs).
ESI/APCI(+): 310 (M+H); 332 (M+Na); 641 (2M+Na).
ESI/APCI(-): 308 (M-H).

EXAMPLE 26 - PREPARATION OF 2-(4-Ethyl-6,6-dimethyl-2-oxomorpholin-3-yl)-*N*-(1-methyl-1*H*-pyrazol-5-yl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and 5-amino-1-methyl-1*H*-pyrazole (0.032 g; 0.329 mmol) in DMF (2 mL). Purification by flash chromatography on silica gel using a gradient of methanol (0 - 10%) in dichloromethane followed by purification by preparative TLC using 5% methanol in dichloromethane as eluent furnished 0.035 g (43%) of the title compound as an orange foam.
$^1$H NMR ($CDCl_3$) δ 1.11 (3H, t); 1.43 (3H, s); 1.46 (3H, s); 2.45 (1H, d); 2.51 (1H, m); 2.9-3.1 (4H, m); 3.42 (1H, m); 3.74 (3H, s); 6.27 (1 H, d); 7.40 (1H, d); 8.80 (1 H, s).
ESI/APCI(+): 295 (M+H); 317 (M+Na); 611 (2M+Na).
ESI/APCI(-): 293 (M-H).

EXAMPLE 27 - PREPARATION OF 2-(4-Ethyl-6,6-dimethyl-2-oxomorpholin-3-yl)-*N*-(3-phenyl-1,2,4-thiadiazol-5-yl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and 3-phenyl-1,2,4-thiadiazol-5-amine (0.059 g; 0.333 mmol) in DMF (2 mL). Purification by flash chromatography on silica gel using a gradient of ethyl acetate (20 - 60%) in heptane furnished 0.012 g (12%) of the title compound as a yellow solid.
$^1$H NMR ($CDCl_3$) δ 1.15 (3H, t); 1.43 (3H, s); 1.56 (3H, s); 2.52 (2H, m); 2.9-3.1 (3H, m); 3.31 (1H, m); 3.46 (1H, m); 7.45 (3H, m); 8.21 (2H, m); 11.21 (1 H, brs).
ESI/APCI(+): 375 (M+H); 397 (M+Na).
ESI/APCI(-): 373 (M-H).

EXAMPLE 28 - PREPARATION OF 2-(4-Ethyl-6,6-dimethyl-2-oxomorpholin-3-yl)-*N*-(1-methyl-1 H-pyrazol-3-yl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and 1-methyl-1*H*-pyrazole-3-amine (0.032 g; 0.329 mmol) in DMF (2 mL). Purification by flash chromatography on silica gel using a gradient of methanol (0 - 20%) in dichloromethane furnished 0.049 g (60%) of the title compound as an orange foam.
$^1$H NMR ($CDCl_3$) δ 1.10 (3H, t); 1.40 (3H, s); 1.51 (3H, s); 2.42 (1 H, d); 2.49 (1 H, m); 2.9-3.1 (4H, m); 3.43 (1 H, m); 3.77 (3H, s); 6.63 (1 H, d); 7.21 (1 H, d); 9.04 (1H, brs).
ESI/APCI(+): 295 (M+H); 317 (M+Na); 611 (2M+Na).
ESI/APCI(-): 293 (M-H).

EXAMPLE 29 - PREPARATION OF 2-(4-Ethyl-6,6-dimethyl-2-oxomorpholin-3-yl)-*N*-(5-(trifluoromethyl)-1,3,4-thiadiazol-2-yl)acetamide

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and 5-(trifluoromethyl)-1,3,4-thiadiazol-2-amine (0.056 g; 0.331 mmol) in DMF (2 mL). Purification by flash chromatography on silica gel using a gradient of ethyl acetate (20 - 60%) in heptane furnished 0.062 g (67%) of the title compound as an orange foam.
$^1$H NMR ($CDCl_3$) δ 1.10 (3H, t); 1.43 (3H, s); 1.53 (3H, s); 2.51 (2H, m); 2.94 (2H, m); 3.2-3.4 (2H, m); 3.56 (1H, m); 11.94 (1H, brs).
ESI/APCI(+): 367 (M+H); 389 (M+Na).
ESI/APCI(-): 365 (M-H).

EXAMPLE 30 - PREPARATION OF 4-Ethyl-6,6-dimethyl-3-(2-oxo-2-(1*H*-pyrazol-1-yl)ethyl)morpholin-2-one

The compound was prepared according to the procedure E from 2-(4-ethyl-6,6-dimethyl-2-oxomorpholin-3-yl)acetic acid (0.276 mmol), diisopropylethylamine (0.240 mL; 1.374 mmol), HATU (0.127 g; 0.334 mmol) and pyrazole (0.023 g; 0.338 mmol) in DMF (2 mL). Purification by flash chromatography on silica gel using a gradient of ethyl acetate (30 - 50%) in heptane furnished 0.057 g (78%) of the title compound as a beige powder.
$^1$H NMR ($CDCl_3$) δ 0.99 (3H, t); 1.41 (3H, s); 1.53 (3H, s); 2.39 (2H, m); 2.82 (2H, m); 3.59 (1H, m); 3.64 (1H, m); 4.06 (1H, m); 6.46 (1H, m); 7.72 (1H, s); 8.27 (1H, d).
ESI/APCI(+): 266 (M+H); 288 (M+Na); 553 (2M+Na).

EXAMPLE 31 - PREPARATION OF *N*-(4,5-Dimethylthiazol-2-yl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.276 mmol), diisopropylethylamine (0.300 mL; 1.718 mmol), HATU (0.127 g; 0.334 mmol) and 4,5-dimethyl-1,3-thiazol-2-amine hydrobromide (0.069 g; 0.330 mmol) in DMF (2 mL). Purification by flash chromatography on silica gel using a gradient of ethyl acetate (30 - 100%) in heptane furnished 0.038 g (42%) of the title compound as a white powder.
$^1$H NMR ($CDCl_3$) δ 1.09 (3H, t); 1.41 (3H, s); 1.51 (3H, s); 2.20 (3H, s); 2.27 (3H, s); 2.44 (1 H, d); 2.47 (1H, m); 2.8-3.0 (3H, m); 3.14 (1H, m); 3.44 (1H, m).
ESI/APCI(+): 326 (M+H); 348 (M+Na).
ESI/APCI(-): 324 (M-H).

EXAMPLE 32 - PREPARATION OF *N*,*N*-Diethyl-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.277 mmol), diisopropylethylamine (0.240 mL; 1.374 mmol), HATU (0.135 g; 0.355 mmol) and diethylamine (0.040 mL; 0.387 mmol) in DMF (1 mL). Purification by flash chromatography on silica gel using a gradient of ethyl acetate (25 - 100%) in heptane furnished 0.048 g (64%) of the title compound as a yellow oil.
$^1$H NMR ($CDCl_3$) δ 1.04 (3H, t); 1.12 (3H, t); 1.20 (3H, t); 1.38 (3H, s); 1.52 (3H, s); 2.32-2.40 (2H, m); 2.68-2.85 (3H, m); 3.05 (1H, dd); 3.30-3.43 (4H, m); 3.68 (1H, t).
ESI/APCI(+): 271 (M+H).

EXAMPLE 33 - PREPARATION OF 2-(4-Ethyl-6,6-dimethyl-2-oxomorpholin-3-yl)-*N*-isobutylacetamide

The compound was prepared according to the procedure E from 2-(4-ethyl-6,6-dimethyl-2-oxomorpholin-3-yl)acetic acid (0.277 mmol), diisopropylethylamine (0.240 mL; 1.374 mmol), HATU (0.126 g; 0.331 mmol) and isobutylamine (0.040 mL; 0.402 mmol) in DMF (1 mL). Purification by flash chromatography on silica gel using a gradient of ethyl acetate (25 - 100%) in heptane furnished 0.034 g (45%) of the title compound as a yellow solid.
$^1$H NMR ($CDCl_3$) δ 0.89 (3H, s); 0.92 (3H, s); 1.07 (3H, t); 1.40 (3H, s); 1.47 (3H, s); 1.71-1.80 (2H, m); 2.75 (1H, dd), 2.84-2.96 (3H, m); 3.07 (2H, t); 3.35 (1H, m); 6.44 (1H, brs).
ESI/APCI(+): 271 (M+H).

EXAMPLE 34 - PREPARATION OF *N*-Cyclohexyl-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.277 mmol), diisopropylethylamine (0.240 mL; 1.374 mmol), HATU (0.136 g; 0.357 mmol) and cyclohexylamine (0.040 mL; 0.349 mmol) in DMF (1 mL). Purification by flash chromatography on silica gel using a gradient of ethyl acetate (25 - 100%) in heptane furnished 0.062 g (76%) of the title compound as a yellow solid.
$^1$H NMR ($CDCl_3$) δ 1.04-1.21 (6H, m)□; 1.26-1.37 (2H, m); 1.39 (3H, s); 1.49 (3H, s); 1.57-1.72 (3H, m); 1.90 (2H, m); 2.36-2.48 (2H, m); 2.72 (1H, dd); 2.84-2.95 (3H, m); 3.34 (1H, m); 3.75 (1H, m); 6.30 (1H, brs).
ESI/APCI(+): 297 (M+H).

EXAMPLE 35 - PREPARATION OF 2-(4-Ethyl-6,6-dimethyl-2-oxomorpholin-3-yl)-N-propylacetamide

The compound was prepared according to the procedure E from 2-(4-ethyl-6,6-dimethyl-2-oxomorpholin-3-yl)acetic acid (0.285 mmol), diisopropylethylamine (0.240 mL; 1.374 mmol), HATU (0.128 g; 0.337 mmol) and propylamine (0.040 mL; 0.487 mmol) in DMF (1 mL). Purification by flash chromatography on silica gel using a gradient of ethyl acetate (25 - 100%) in heptane furnished 0.018 g (25%) of the title compound as a yellow solid.
$^1$H NMR ($CDCl_3$) δ 0.91 (3H, t); 1.07 (3H, t); 1.40 (3H, s); 1.45-1.57 (5H, m); 2.37-2.52 (2H, m); 2.71-2.98 (4H, m); 3.20 (2H, q); 3.35 (1H, m); 6.40 (1H, brs).
ESI/APCI(+): 257 (M+H).

EXAMPLE 36 - PREPARATION OF *N-tert*-Butyl-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.285 mmol), diisopropylethylamine (0.240 mL; 1.374 mmol), HATU (0.142 g; 0.373 mmol) and *tert*-butylamine (0.040 mL; 0.381 mmol) in DMF (1 mL). Purification by flash chromatography on silica gel using a gradient of ethyl acetate (25 - 100%) in heptane furnished 0.071 g (92%) of the title compound as a yellow oil.
$^1$H NMR ($CDCl_3$) δ 1.07 (3H, t); 1.33 (9H, s); 1.39 (3H, s); 1.49 (3H, s); 2.35-2.46 (2H, m); 2.66 (1H, dd); 2.81-2.94 (3H, m); 3.35 (1H, m); 6.09 (1 H, brs).
ESI/APCI(+): 271 (M+H).

EXAMPLE 37 - PREPARATION OF *N*-(3,3-Dimethylbutyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.285 mmol), diisopropylethylamine (0.24 mL; 1.374 mmol), HATU (0.129 g; 0.339 mmol) and 3,3-dimethylbutylamine (0.040 mL; 0.297 mmol) in DMF (1 mL). Purification by flash chromatography on silica gel using a gradient of ethyl acetate (25 - 100%) in heptane furnished 0.014 g (16%) of the title compound as a yellow oil.
$^1$H NMR ($CDCl_3$) δ 0.91 (9H, s); 1.07 (3H, t); 1.36-1.40 (5H, m); 1.47 (3H, s); 2.37-2.50 (2H, m); 2.72 (1H, dd); 2.83-2.95 (3H, m); 3.23 (2H, m); 3.35 (1H, m); 6.20 (1H, brs).
ESI/APCI(+): 299 (M+H).

EXAMPLE 38 - PREPARATION OF 2-(4-Ethyl-6,6-dimethyl-2-oxomorpholin-3-yl)-N-(2,2,2-trifluoroethyl)acetamide

The compound was prepared according to the procedure E from 2-(4-ethyl-6,6-dimethyl-2-oxomorpholin-3-yl)acetic acid (0.277 mmol), diisopropylethylamine (0.240 mL; 1.374 mmol), HATU (0.135 g; 0.355 mmol) and 2,2,2-trifluoroethylamine (0.040 mL; 0.508 mmol) in DMF (1 mL). Purification by flash chromatography on silica gel using a gradient of ethyl acetate (25 - 100%) in heptane furnished 0.055 g (65%) of the title compound as a yellow oil.
$^1$H NMR ($CDCl_3$) $\delta$ 1.08 (3H, t); 1.41 (3H, s); 1.47 (3H, s); 2.37-2.44 (2H, m); 2.79-3.03 (4H, m); 3.32 (1 H, m); 3.78 (1 H, m); 4.01 (1 H, m); 7.14 (1 H, brs).
ESI/APCI(+): 297 (M+H).

EXAMPLE 39 - PREPARATION OF *N*-Cyclopentyl-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.295 mmol), diisopropylethylamine (0.240 mL; 1.374 mmol), HATU (0.134 g; 0.352 mmol) and cyclopentylamine (0.040 mL; 0.405 mmol) in DMF (1 mL). Purification by flash chromatography on silica gel using a gradient of ethyl acetate (25 - 100%) in heptane furnished 0.049 g (59%) of the title compound as a yellow oil.
$^1$H NMR ($CDCl_3$) $\delta$ 1.07 (3H, t); 1.32-1.39 (5H, m); 1.48 (3H, s); 1.54-1.68 (4H, m); 1.94 (2H, m); 2.36-2.48 (2H, m); 2.72 (1H, dd); 2.85-3.0 (3H, m); 3.35 (1H, m); 4.18 (1H, m); 6.44 (1H, brs).
ESI/APCI(+): 283 (M+H).

EXAMPLE 40 - PREPARATION OF 4-Ethyl-6,6-dimethyl-3-(2-oxo-2-(pyrrolidin-1-yl)ethyl)morpholin-2-one

The compound was prepared according to the procedure E from 2-(4-ethyl-6,6-dimethyl-2-oxomorpholin-3-yl)acetic acid (0.295 mmol), diisopropylethylamine (0.240 mL; 1.374 mmol), HATU (0.134 g; 0.352 mmol) and pyrrolidine (0.040 mL; 0.479 mmol) in DMF (1 mL). Purification by flash chromatography on silica gel using a gradient of ethyl acetate (25 - 100%) in heptane furnished 0.050 g (63%) of the title compound as a yellow oil.
$^1$H NMR ($CDCl_3$) $\delta$ 1.05 (3H, t); 1.39 (3H, s); 1.54 (3H, s); 1.83-2.02 (4H, m); 2.38 (2H, m); 2.7-3.0 (4H, m); 3.43-3.50 (4H, m); 3.62 (1H, m).
□ESI/APCI(+): 269 (M+H).

EXAMPLE 41 - PREPARATION OF *N*-Ethyl-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.295 mmol), diisopropylethylamine (0.240 mL; 1.374 mmol), HATU (0.130 g; 0.342 mmol) and a 2M ethylamine solution in tetrahydrofuran (0.300 mL; 0.600 mmol) in DMF (1 mL). Purification by flash chromatography on silica gel using a gradient of ethyl acetate (25 - 100%) in heptane furnished 0.035 g (49%) of the title compound as a yellow solid.
$^1$H NMR ($CDCl_3$) $\delta$ 1.07 (3H, t); 1.12 (3H, t); 1.40 (3H, s); 1.48 (3H, s); 2.37-2.51 (2H, m); 2.73 (1H, dd); 2.84-2.97 (3H, m); 3.27 (2H, m); 3.36 (1H, m); 6.37 (1H, brs).
□ESI/APCI(+): 243 (M+H).

EXAMPLE 42 PREPARATION OF *N*-*sec*-Butyl-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.295 mmol), diisopropylethylamine (0.240 mL; 1.374 mmol), HATU (0.144 g; 0.379 mmol) and *sec*-butylamine (0.040 mL; 0.396 mmol) in DMF (1 mL). Purification by flash chromatography on silica gel using a gradient of ethyl acetate (25 - 100%) in heptane furnished 0.061 g (76%) of the title compound as a yellow solid.
$^1$H NMR ($CDCl_3$) $\delta$ 0.89 (3H, t); 1.05-1.12 (6H, m); 1.39-1.49 (8H, m); 2.36-2.48 (2H, m); 2.69-2.75 (1H, m); 2.85-2.97 (3H, m); 3.35 (1H, m); 3.87 (1H, m); 6.25 (1H, brs).
ESI/APCI(+): 271 (M+H).

EXAMPLE 43 - PREPARATION OF *N*-(Cyclohexylmethyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.302 mmol), diisopropylethylamine (0.240 mL; 1.374 mmol), HATU (0.131 g; 0.345 mmol) and cyclohexylmethylamine (0.040 mL; 0.307 mmol) in DMF (1 mL). Purification by flash chromatography on silica gel using a gradient of ethyl acetate (25 - 100%) in heptane furnished 0.044 g (47%) of the title compound as a yellow solid.
$^1$H NMR ($CDCl_3$) $\delta$ 0.92 (3H, m); 1.07 (3H, t); 1.19 (3H, m); 1.39 (3H, s); 1.47 (3H, s); 1.64-1.72 (5H, m); 2.37-2.49 (2H,

m); 2.74 (1H, dd); 2.84-2.96 (3H, m); 3.07 (2H, m); 3.35 (1H, m); 6.45 (1H, brs).
ESI/APCI(+): 311 (M+H).

EXAMPLE 44 - PREPARATION OF 2-(4-Ethyl-6,6-dimethyl-2-oxomorpholin-3-yl)-*N,N*-dimethylacetamide

The compound was prepared according to the procedure E from 2-(4-ethyl-6,6-dimethyl-2-oxomorpholin-3-yl)acetic acid (0.302 mmol), diisopropylethylamine (0.240 mL; 1.374 mmol), HATU (0.125 g; 0.329 mmol) and a 2M dimethylamine solution in methanol (0.300 mL; 0.600 mmol) in DMF (1 mL). Purification by flash chromatography on silica gel using a gradient of ethyl acetate (25 - 100%) in heptane furnished 0.017 g (23%) of the title compound as a yellow solid.
$^1$H NMR ($CDCl_3$) δ 1.05 (3H, t); 1.39 (3H, s); 1.54 (3H, s); 2.32-2.40 (2H, m); 2.70-2.86 (3H, m); 2.97 (3H, s); 3.03 (3H, s); 3.06 (1H, m); 3.35 (1H, m).
ESI/APCI(+): 243 (M+H).

EXAMPLE 45 - PREPARATION OF *N*-(3-Chlorobenzyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.283 mmol), diisopropylethylamine (0.240 mL; 1.374 mmol), HATU (0.132 g; 0.347 mmol) and 3-chlorobenzylamine (0.040 mL; 0.327 mmol) in DMF (1 mL). Purification by flash chromatography on silica gel using a gradient of ethyl acetate (25 - 100%) in heptane furnished 0.035 g (37%) of the title compound as a yellow oil.
$^1$H NMR ($CDCl_3$) δ 1.05 (3H, t); 1.38 (6H, s); 2.35-2.46 (2H, m); 2.76-3.01 (4H, m); 3.37 (1H, m); 4.40 (2H, m); 7.01 (1H, brs); 7.15-7.29 (4H, m).
ESI/APCI(+): 339 (M+H).

EXAMPLE 46 - PREPARATION OF *N*-(3,4-Dichlorobenzyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.283 mmol), diisopropylethylamine (0.240 mL; 1.374 mmol), HATU (0.141 g; 0.371 mmol) and 3,4-dichlorobenzylamine (0.040 mL; 0.264 mmol) in DMF (1 mL). Purification by flash chromatography on silica gel using a gradient of ethyl acetate (25 - 100%) in heptane furnished 0.053 g (54%) of the title compound as a yellow oil.
$^1$H NMR ($CDCl_3$) δ 1.06 (3H, t); 1.37 (3H, s); 1.38 (3H, s); 2.36-2.48 (2H, m); 2.7-3.3 (4H, m); 3.36 (1H, m); 4.38 (2H, m); 6.89 (1H, brs); 7.12 (1H, m); 7.36-7.39 (2H, m).
ESI/APCI(+): 373 (M+H).

EXAMPLE 47 - PREPARATION OF *N*-(2,6-Dichlorobenzyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.283 mmol), diisopropylethylamine (0.240 mL; 1.374 mmol), HATU (0.130 g; 0.342 mmol) and 2,6-dichlorobenzylamine (0.040 mL; 0.266 mmol) in DMF (1 mL). Purification by flash chromatography on silica gel using a gradient of ethyl acetate (25 - 100%) in heptane furnished 0.057 g (57%) of the title compound as a yellow oil.
$^1$H NMR ($CDCl_3$) δ 1.02 (3H, t); 1.28 (3H, s); 1.35 (3H, s); 2.31-2.42 (2H, m); 2.75-2.99 (4H, m); 3.32 (1H, m); 4.75 (2H, d); 6.88 (1H, brs); 7.14-7.19 (3H, m).
ESI/APCI(+): 373 (M+H).

EXAMPLE 48 - PREPARATION OF *N*-(2-Chlorobenzyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.295 mmol), diisopropylethylamine (0.240 mL; 1.374 mmol), HATU (0.131 g; 0.345 mmol) and 2-chlorobenzylamine (0.040 mL; 0.331 mmol) in DMF (1 mL). Purification by flash chromatography on silica gel using a gradient of ethyl acetate (25 - 100%) in heptane furnished 0.036 g (36%) of the title compound as a yellow oil.
$^1$H NMR ($CDCl_3$) δ 1.03 (3H, t); 1.35 (3H, s); 1.37 (3H, s); 2.34-2.44 (2H, m); 2.74-3.00 (4H, m); 3.33 (1H, m); 4.50 (2H, d); 7.03 (1H, brs); 7.20-7.41 (4H, m).
ESI/APCI(+): 339 (M+H).

EXAMPLE 49 - PREPARATION OF 4-Ethyl-6,6-dimethyl-3-(2-(4-methylpiperazin-1-yl)-2-oxoethyl)morpholin-2-one

The compound was prepared according to the procedure E from 2-(4-ethyl-6,6-dimethyl-2-oxomorpholin-3-yl)acetic acid (0.295 mmol), diisopropylethylamine (0.240 mL; 1.374 mmol), HATU (0.131 g; 0.345 mmol) and 1-methylpiperazine (0.040 mL; 0.361 mmol) in DMF (1 mL). Purification by flash chromatography on silica gel using a gradient of methanol (0 - 20%) in dichloromethane furnished 0.024 g (29%) of the title compound as a yellow oil.
$^1$H NMR ($CDCl_3$) δ 1.05 (3H, t); 1.39 (3H, s); 1.53 (3H, s); 2.32 (3H, s); 2.34-2.41 (6H, m); 2.63-2.86 (3H, m); 3.06 (1 H, m); 3.52-3.65 (5H, m).
ESI/APCI(+): 298 (M+H).

EXAMPLE 50 - PREPARATION OF *N*-Cyclopropyl-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.295 mmol), diisopropylethylamine (0.240 mL; 1.374 mmol), HATU (0.139 g; 0.366 mmol) and cyclopropylamine (0.040 mL, 0.577 mmol) in DMF (1 mL). Purification by flash chromatography on silica gel using a gradient of ethyl acetate (25 - 100%) in heptane furnished 0.018 g (24%) of the title compound as a yellow solid.
$^1$H NMR ($CDCl_3$) δ 0.47 (2H, m); 0.75 (2H, m); 1.06 (3H, t); 1.39 (3H, s); 1.47 (3H, s); 2.36-2.45 (2H, m); 2.66-2.74 (2H, m); 2.83-2.94 (3H, m); 3.34 (1H, m); 6.48 (1H, brs).
ESI/APCI(+): 255 (M+H).

EXAMPLE 51 - PREPARATION OF 2-(4-Ethyl-6,6-dimethyl-2-oxomorpholin-3-yl)-*N*-(4-phenoxyphenyl)acetamide

The compound was prepared according to the procedure E from 2-(4-ethyl-6,6-dimethyl-2-oxomorpholin-3-yl)acetic acid (0.295 mmol), diisopropylethylamine (0.240 mL; 1.374 mmol), HATU (0.127 g; 0.334 mmol) and 4-phenoxyaniline (0.072 g; 0.577 mmol) in DMF (1 mL). Purification by flash chromatography on silica gel using a gradient of ethyl acetate (25 - 100%) in heptane furnished 0.028g (25%) of the title compound as a yellow solid.
$^1$H NMR ($CDCl_3$) δ 1.12 (3H, t); 1.42 (3H, s); 1.47 (3H, s); 2.41-2.53 (2H, m); 2.89-3.09 (4H, m); 3.41 (1H, m); 6.95-6.99 (4H, m); 7.07 (1H, m); 7.28-7.34 (2H, m); 7.44-7.47 (2H, m); 8.55 (1 H, brs).
ESI/APCI(+): 383 (M+H).

EXAMPLE 52 - PREPARATION OF *N*-(4-*tert*-Butylthiazol-2-yl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.285 mmol), diisopropylethylamine (0.240 mL; 1.374 mmol), HATU (0.125 g; 0.329 mmol) and 4-*tert*-butylthiazol-2-amine (0.046 g; 0.294 mmol) in DMF (1 mL). Purification by flash chromatography on silica gel using a gradient of ethyl acetate (25 - 100%) in heptane furnished 0.036 g (36%) of the title compound as a yellow oil.
$^1$H NMR ($CDCl_3$) δ 1.14 (3H, t); 1.26 (9H, s); 1.42 (3H, s); 1.56 (3H, s); 2.43-2.53 (2H, m); 2.95-3.03 (3H, m); 3.19 (1 H, dd); 3.43 (1H, m); 6.49 (1H, s); 10.37 (1 H, brs).
ESI/APCI(+): 354 (M+H).

EXAMPLE 53 - PREPARATION OF 2-(4-Ethyl-6,6-dimethyl-2-oxomorpholin-3-yl)-*N*-(2-methylbenzyl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.128 g; 0.337 mmol) and 2-methylbenzylamine (0.050 mL; 0.403 mmol) in DMF (2 mL). Purification by flash chromatography on silica gel using a gradient of ethyl acetate (25 - 100%) in heptane furnished 0.007 g (7%) of the title compound as a yellow oil.
$^1$H NMR ($CDCl_3$) δ 1.03 (3H, t); 1.17 (6H, s); 2.44 (3H, s); 2.54-2.72 (4H, m); 2.81-2.96 (2H, m); 4.14 (1H, m); 4.69 (2H, m); 7.12-7.20 (5H, m).
ESI/APCI(+): 319 (M+H).

EXAMPLE 54 - PREPARATION OF 2-(4-Ethyl-6,6-dimethyl-2-oxomorpholin-3-yl)-*N*-(3-methylbenzyl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.136 g; 0.358 mmol) and 3-methylbenzylamine (0.050 mL; 0.399 mmol) in DMF (2 mL). Purification by flash chromatography on silica gel using a gradient of ethyl acetate (25 - 100%) in heptane furnished 0.009 g (9%) of the title compound as a yellow oil.
$^1$H NMR ($CDCl_3$) δ 1.04 (3H, t); 1.36 (3H, s); 1.37 (3H, s); 2.33 (3H, s); 2.35-2.47 (2H, m); 2.75-3.00 (4H, m); 3.37 (1H, m); 4.39 (2H, m); 6.69 (1H, brs) 7.05-7.23 (4H, m).
ESI/APCI(+): 319 (M+H).

EXAMPLE 55 - PREPARATION OF 2-(4-Ethyl-6,6-dimethyl-2-oxomorpholin-3-yl)-*N*-(4-methylbenzyl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.128 g; 0.337 mmol) and 4-methylbenzylamine (0.050 mL; 0.393 mmol) in DMF (2 mL). Purification by flash chromatography on silica gel using a gradient of ethyl acetate (25 - 100%) in heptane followed by recrystallization from ethyl acetate and heptane furnished 0.044 g (44%) of the title as a white solid.
$^1$H NMR ($CDCl_3$) $\delta$ 1.04 (3H, t); 1.36 (3H, s); 1.37 (3H, s); 2.32 (3H, s); 2.34-2.46 (2H, m); 2.73-2.98 (4H, m); 3.36 (1H, m); 4.40 (2H, m); 6.71 (1H, brs); 7.10-7.18 (4H, m).
ESI/APCI(+): 319 (M+H).

EXAMPLE 56 - PREPARATION OF 2-(4-Ethyl-6,6-dimethyl-2-oxomorpholin-3-yl)-*N*-(2-(trifluoromethyl)benzyl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.125 g; 0.329 mmol) and 2-trifluoromethylbenzylamine (0.050 mL; 0.357 mmol) in DMF (2 mL). Purification by flash chromatography on silica gel using a gradient of ethyl acetate (25 - 100%) in heptane followed by purification by preparative HPLC (method 2) furnished 0.058 g (49%) of the title compound as a yellow oil.
$^1$H NMR ($CDCl_3$) $\delta$ 1.04 (3H, t); 1.34 (3H, s); 1.38 (3H, s); 2.36-2.47 (2H, m); 2.77-3.03 (4H, m); 3.37 (1H, m); 4.49 (2H, m); 6.97 (1H, brs); 7.43-7.56 (4H, m).
ESI/APCI(+): 373 (M+H).

EXAMPLE 57 - PREPARATION OF 2-(4-Ethyl-6,6-dimethyl-2-oxomorpholin-3-yl)-*N*-(3-(trifluoromethyl)benzyl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.137 g; 0.360 mmol) and 3-trifluoromethylbenzylamine (0.050 mL; 0.348 mmol) in DMF (2 mL). Purification by flash chromatography on silica gel using a gradient of ethyl acetate (25 - 100%) in heptane followed by recrystallization from ethyl acetate and heptane furnished 0.058 g (49%) of the title compound as a white solid.
$^1$H NMR ($CDCl_3$) $\delta$ 1.02 (3H, t); 1.32 (3H, s); 1.36 (3H, s); 2.34-2.44 (2H, m); 2.74-3.01 (4H, m); 3.34 (1H, m); 4.61 (2H, m); 6.96 (1 H, brs); 7.33-7.64 (4H, m).
ESI/APCI(+): 373 (M+H).

EXAMPLE 58 - PREPARATION OF 2-(4-Ethyl-6,6-dimethyl-2-oxomorpholin-3-yl)-*N*-(4-(trifluoromethyl)benzyl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.136 g; 0.358 mmol) and 4-trifluoromethylbenzylamine (0.050 mL; 0.351 mmol) in DMF (2 mL). Purification by flash chromatography on silica gel using a gradient of ethyl acetate (25 - 100%) in heptane furnished 0.018 g (15%) of the title compound as a yellow oil.
$^1$H NMR ($CDCl_3$) $\delta$ 1.07 (3H, t); 1.15 (3H, s); 1.16 (3H, s); 2.34-2.94 (6H, m); 4.14 (1H, m); 4.71 (2H, m); 6.96 (1H, brs); 7.49 (2H, d); 7.57 (2H, d).
ESI/APCI(+): 373 (M+H).

EXAMPLE 59 - PREPARATION OF 2-(4-Ethyl-6,6-dimethyl-2-oxomorpholin-3-yl)-*N*-(2-methoxybenzyl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.118 g; 0.310 mmol) and 2-methoxybenzylamine (0.050 mL, 0.383 mmol) in DMF (2 mL). Purification by flash chromatography on silica gel using a gradient of ethyl acetate (25 - 100%) in heptane followed by purification by preparative HPLC (method 3) furnished 0.063 g (60%) of the title compound as a yellow oil.
$^1$H NMR ($CDCl_3$) $\delta$ 1.01 (3H, t); 1.32 (3H, s); 1.39 (3H, s); 2.33-2.45 (2H, m); 2.72-2.94 (4H, m); 3.34 (1H, m); 3.84 (3H, s); 4.42 (2H, m); 6.87-6.91 (3H, m); 7.21-7.27 (2H, m).
ESI/APCI(+): 335 (M+H).

EXAMPLE 60 - PREPARATION OF 2-(4-Ethyl-6,6-dimethyl-2-oxomorpholin-3-yl)-*N*-(3-methoxybenzyl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.136 g; 0.358 mmol) and 3-methoxybenzylamine (0.050 mL, 0.390 mmol) in DMF (2 mL). Purification by flash chromatography on silica gel using a gradient of ethyl acetate (25 - 100%) in heptane followed by recrystallization from ethyl acetate and heptane furnished 0.041 g (39%) of the title compound as a white solid.
$^1$H NMR ($CDCl_3$) $\delta$ 1.05 (3H, t); 1.35 (3H, s); 1.37 (3H, s); 2.34-2.47 (2H, m); 2.70-3.04 (4H, m); 3.37 (1H, m); 3.80 (3H, s); 4.41 (2H, m); 6.78-6.84 (4H, m); 7.22 (1 H, m).
ESI/APCI(+): 335 (M+H).

EXAMPLE 61 - PREPARATION OF 2-(4-Ethyl-6,6-dimethyl-2-oxomorpholin-3-yl)-*N*-(4-methoxybenzyl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.128 g; 0.337 mmol) and 4-methoxybenzylamine (0.050 mL, 0.383 mmol) in DMF (2 mL). Purification by flash chromatography on silica gel using a gradient of ethyl acetate (25 - 100%) in heptane furnished 0.020 g (19%) of the title compound as a yellow oil.
$^1$H NMR ($CDCl_3$) $\delta$ 1.04 (3H, t); 1.35 (3H, s); 1.37 (3H, s); 2.34-2.46 (2H, m); 2.73-3.34 (4H, m); 3.36 (1H, m); 3.79 (3H, s); 4.36 (2H, m); 6.68 (1H, brs); 6.84 (2H, d); 7.20 (2H, d).
ESI/APCI(+): 335 (M+H).

EXAMPLE 62 - PREPARATION OF *N*-(Biphenyl-2-ylmethyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.122 g; 0.321 mmol) and 2-phenylbenzylamine (0.058 g, 0.317 mmol) in DMF (2 mL). Purification by flash chromatography on silica gel using a gradient of ethyl acetate (25 - 100%) in heptane followed by recrystallization from ethyl acetate furnished 0.020 g (19%) of the title compound as a white solid.
$^1$H NMR ($CDCl_3$) $\delta$ 1.03 (3H, t); 1.29 (3H, s); 1.36 (3H, s); 2.32-2.43 (2H, m); 2.67-3.03 (4H, m); 3.30 (1H, m); 4.41 (2H, m); 6.51 (1H, brs); 7.22-7.43 (8H, m).
ESI/APCI(+): 381 (M+H).

EXAMPLE 63 - PREPARATION OF *N*-(Biphenyl-3-ylmethyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.131 g; 0.345 mmol) and 3-phenylbenzylamine (0.054 g, 0.295 mmol) in DMF (2 mL). Purification by flash chromatography on silica gel using a gradient of ethyl acetate (25 - 100%) in heptane followed by purification by preparative HPLC (method 2) furnished 0.070 g (58%) of the title compound as a yellow oil.
$^1$H NMR ($CDCl_3$) $\delta$ 1.02 (3H, t); 1.33 (3H, s); 1.35 (3H, s); 2.34-2.44 (2H, m); 2.72-3.02 (4H, m); 3.36 (1H, m); 4.49 (2H, m); 6.82 (1H, brs); 7.24-7.59 (8H, m).
ESI/APCI(+): 381 (M+H).

EXAMPLE 64 - PREPARATION OF *N*-(Biphenyl-4-ylmethyl)-2-(4-ethyl-6,6-dimethyl-2-oxomorpholin-3-yl)acetamide

The compound was prepared according to the procedure E from 2-(4-ethyl-6,6-dimethyl-2-oxomorpholin-3-yl)acetic acid (0.316 mmol), diisopropylethylamine (0.240 mL; 1.374 mmol), HATU (0.130 g; 0.342 mmol) and 4-phenylbenzylamine (0.051 g, 0.278 mmol) in DMF (2 mL). Purification by flash chromatography on silica gel using a gradient of ethyl acetate (25 - 100%) in heptane furnished 0.027 g (22%) of the title compound as an amorphous solid.
$^1$H NMR ($CDCl_3$) $\delta$ 1.06 (3H, t); 1.36 (3H, s); 1.37 (3H, s); 2.36-2.49 (2H, m); 2.77-3.01 (4H, m); 3.38 (1 H, m); 4.48 (2H, m); 6.76 (1 H, brs); 7.32-7.58 (8H, m).
ESI/APCI(+): 381 (M+H).

EXAMPLE 65 - PREPARATION OF 2-(4-Ethyl-2-oxo-6-phenylmorpholin-3-yl)-N-(4-isopropylphenyl)acetamide

To a solution of methyl 2-(4-ethyl-2-oxo-6-phenylmorpholin-3-yl)acetate (0.106 g; 0,382 mmol) in a mixture of dioxane/water (1/1; 4 mL) was added dropwise a 1M lithium hydroxide solution (0.760 mL; 0.760 mmol). After 40 min at room temperature, the mixture was acidified with 1 N HCl (pH = 5-7), concentrated *in vacuo* and coevaporated with

toluene. The crude material was used in the next step without any further purification.

The crude acid was suspended in DMF (3 mL) and the mixture was cooled at 0 °C. HATU (0.244 g; 0.642 mmol) and diisopropylethylamine (0.196 mL; 1.122 mmol) were added. After 1 hour at 0 °C, 4-isopropylaniline (0.065 mL; 0.475 mmol) was added. The reaction mixture was stirred for an additional hour at 0 °C and overnight at room temperature. The solvent was evaporated under reduced pressure. The residue was partitioned between ethyl acetate and a saturated sodium bicarbonate solution. The organic phase was washed with water and brine, dried over sodium sulfate, filtered, concentrated under reduced pressure and purified by flash column chromatography on silica gel using a gradient of ethyl acetate (20 - 100%) in heptane to afford 0.064 g (44%) of the title compound as a yellow oil.
ESI/APCI(+): 381 (M+H); 403 (M+Na).
ESI/APCI(-): 379 (M-H).

EXAMPLE 66 - PREPARATION OF 2-((6*S*)-4-Ethyl-2-oxo-6-phenylmorpholin-3-yl)-*N*-(4-isopropylphenyl)acetamide

To a solution of methyl 2-((6*S*)-4-ethyl-2-oxo-6-phenylmorpholin-3-yl)acetate (0.106 g; 0.382 mmol) in a mixture of dioxane/water (1/1; 4 mL) was added dropwise a 1M lithium hydroxide solution (0.760 mL; 0.760 mmol). After 40 min at room temperature, the mixture was acidified with 1 N HCl (pH = 5-7), concentrated *in vacuo* and coevaporated with toluene. The crude material was used in the next step without any further purification.

The crude acid was suspended in DMF (3 mL) and the mixture was cooled at 0 °C. HATU (0.244 g; 0.642 mmol) and diisopropylethylamine (0.196 mL; 1.122 mmol) were added. After 1 hour at 0 °C, 4-isopropylaniline (0.065 mL; 0.475 mmol) was added. The reaction mixture was stirred for an additional hour at 0 °C and overnight at room temperature. The solvent was evaporated under reduced pressure. The residue was partitioned between ethyl acetate and a saturated sodium bicarbonate solution. The organic phase was washed with water and brine, dried over sodium sulfate, filtered, concentrated under reduced pressure and purified by flash column chromatography on silica gel using a gradient of ethyl acetate (20 - 100%) in heptane to afford 0.048 g (33%) of the title compound as a yellow oil.
ESI/APCI(+): 381 (M+H); 403 (M+Na).
ESI/APCI(-): 379 (M-H).

EXAMPLE 67 - PREPARATION OF 2-((6*R*)-4-Ethyl-2-oxo-6-phenylmorpholin-3-yl)-*N*-(4-isopropylphenyl)acetamide

To a solution of methyl 2-((6*R*)-4-ethyl-2-oxo-6-phenylmorpholin-3-yl)acetate (0.112 g; 0.404 mmol) in a mixture of dioxane/water (1/1; 4 mL) was added dropwise a 1M lithium hydroxide solution (0.803 mL; 0.803 mmol). After 45 min at room temperature, the mixture was acidified with 1N HCl (pH = 5-7), concentrated *in vacuo* and coevaporated with toluene. The crude material was used in the next step without any further purification.

The crude acid was suspended in DMF (3 mL) and the mixture was cooled at 0 °C. HATU (0.258 g; 0.679 mmol) and diisopropylethylamine (0.207 mL; 1.185 mmol) were added. After 1 hour at 0 °C, 4-isopropylaniline (0.069 mL; 0.505 mmol) was added. The reaction mixture was stirred for an additional hour at 0 °C and overnight at room temperature. The solvent was evaporated under reduced pressure. The residue was partitioned between ethyl acetate and a saturated sodium bicarbonate solution. The organic phase was washed with water and brine, dried over sodium sulfate, filtered, concentrated and purified by flash column chromatography on silica gel using a gradient of ethyl acetate (20 - 100%) in heptane to afford 0.050 g (34%) of the title compound as a yellow oil.
ESI/APCI(+): 381 (M+H); 403 (M+Na).
ESI/APCI(-): 379 (M-H).

EXAMPLE 68 - PREPARATION OF 2-((6*S*)-4-Ethyl-6-methyl-2-oxomorpholin-3-yl)-*N*-(4-isopropylphenyl)acetamide

To a solution of methyl 2-((6*S*)-4-ethyl-6-methyl-2-oxomorpholin-3-yl)acetate (0.100 g; 0.465 mmol) in a mixture of dioxane/water (1/1; 4.6 mL) was added dropwise a 1 M lithium hydroxide solution (0.930 mL; 0.930 mmol). After 35 min at room temperature, the mixture was acidified with 1 N HCl (pH = 5-7), concentrated *in vacuo* and coevaporated with toluene. The crude material was used in the next step without any further purification.

The crude acid was suspended in DMF (3 mL) and the mixture was cooled at 0 °C. HATU (0.281 g; 0.739 mmol) and diisopropylethylamine (0.240 mL; 1.374 mmol) were added. After 1 hour at 0 °C, 4-isopropylaniline (0.080 mL; 0.585 mmol) was added. The reaction mixture was stirred for an additional hour at 0 °C and overnight at room temperature. The solvent was evaporated under reduced pressure. The residue was partitioned between ethyl acetate and a saturated sodium bicarbonate solution. The organic phase was washed with water and brine, dried over sodium sulfate, filtered, concentrated under reduced pressure and purified by flash column chromatography on silica gel using a gradient of ethyl acetate (20 - 100%) in heptane to afford 0.038 g (26%) of the title compound as a yellow oil.
ESI/APCI(+): 319 (M+H); 341 (M+Na).
ESI/APCI(-): 317 (M-H).

EXAMPLE 69 - PREPARATION OF 2-((6*R*)-4-Ethyl-6-methyl-2-oxomorpholin-3-yl)-*N*-(4-isopropylphenyl)acetamide

To a solution of methyl 2-((6*R*)-4-ethyl-6-methyl-2-oxomorpholin-3-yl)acetate (0.100 g; 0.465 mmol) in a mixture of dioxane/water (1/1; 4.6 mL) was added dropwise a 1M lithium hydroxide solution (0.930 mL; 0.930 mmol). After 45 min at room temperature, the mixture was acidified with 1N HCl (pH = 5-7), concentrated *in vacuo* and coevaporated with toluene. The crude material was used in the next step without any further purification.

The crude acid was suspended in DMF (3 mL) and the mixture was cooled at 0 °C. HATU (0.281 g; 0.739 mmol) and diisopropylethylamine (0.240 mL; 1.374 mmol) were added. After 1 hour at 0 °C, 4-isopropylaniline (0.080 mL; 0.585 mmol) was added. The reaction mixture was stirred for an additional hour at 0 °C and overnight at room temperature. The solvent was evaporated under reduced pressure. The residue was partitioned between ethyl acetate and a saturated sodium bicarbonate solution. The organic phase was washed with water and brine, dried over sodium sulfate, filtered, concentrated under reduced pressure and purified by flash column chromatography on silica gel using a gradient of ethyl acetate (30 - 80%) in heptane to give 0.050 g (34%) of the title compound as a yellow oil.
ESI/APCI(+): 319 (M+H); 341 (M+Na).
ESI/APCI(-): 317 (M-H).

EXAMPLE 70 - PREPARATION OF 2-(4-Ethyl-6-methyl-2-oxo-6-phenylmorpholin-3-yl)-*N*-(4-isopropylphenyl)acetamide

The compound was prepared according to the procedure E from 2-(4-ethyl-6-methyl-2-oxo-6-phenylmorpholin-3-yl)acetic acid (0.300 mmol), diisopropylethylamine (0.261 mL; 1.494 mmol), HATU (0.138 g; 0.363 mmol) and 4-isopropylaniline (0.050 mL; 0.366 mmol) in DMF (2 mL). Purification by flash chromatography on silica gel using a gradient of ethyl acetate (10 - 80%) in heptane allowed to isolate 0.039g and 0.056g of two diastereoisomers (global yield: 80%) as brown foams.
Less polar diastereoisomer:

$^1$H NMR ($CDCl_3$) $\delta$ 1.10 (3H, t); 1.22 (6H, d); 1.81 (3H, s); 2.52 (1H, m); 2.64 (1H, m); 2.87 (1H, m); 2.95-3.15 (3H, m); 3.22 (1H, m); 3.51 (1H, m); 7.17 (2H, d); 7.3-7.5 (7H, m); 8.41 (1 H, brs).
ESI/APCI(+): 395 (M+H); 417 (M+Na).

More polar diastereoisomer:

$^1$H NMR ($CDCl_3$) $\delta$ 1.10 (3H, t); 1.21 (6H, d); 1.64 (3H, s); 2.44 (1H, m); 2.74 (1H, d); 2.8-3.1 (4H, m); 3.50 (1H, m); 3.72 (1H, m); 6.81 (1H, d); 6.98 (2H, d); 7.0-7.3 (2H, m); 7.30 (3H, m); 8.72 (1 H, brs).
ESI/APCI(+): 395 (M+H); 417 (M+Na); 811 (2M+Na).

EXAMPLE 71 - PREPARATION OF 2-(4-Ethyl-5,5,6,6-tetramethyl-2-oxomorpholin-3-yl)-*N*-(4-isopropylphenyl)acetamide

The compound was prepared according to the procedure E from 2-(4-ethyl-5,5,6,6-tetramethyl-2-oxomorpholin-3-yl)acetic acid (0.528 mmol), diisopropylethylamine (0.460 mL; 2.634 mmol), HATU (0.243 g; 0.639 mmol) and 4-isopropylaniline (0.087 mL; 0.651 mmol) in DMF (3.8 mL). Purification by flash chromatography on silica gel using a gradient of ethyl acetate (20 - 50%) in heptane furnished 0.136 g (71%) of the title compound as a white powder.
$^1$H NMR ($CDCl_3$) $\delta$ 1.13 (6H, d); 1.17 (3H, t); 1.21 (3H, s); 1.23 (3H, s); 1.35 (3H, s); 1.46 (3H, s); 2.44 (1 H, m); 2.8-3.0 (4H, m); 3.77 (1H, m); 7.16 (2H, d); 7.43 (2H, d); 8.21 (1H, brs).
ESI/APCI(+): 361 (M+H); 383 (M+Na); 743 (2M+Na).
ESI/APCI(-): 359 (M-H).

EXAMPLE 72 - PREPARATION OF 2-(4-Ethyl-5,6,6-trimethyl-2-oxomorpholin-3-yl)-*N*-(4-isopropylphenyl)acetamide

The compound was prepared according to the procedure E from 2-(4-ethyl-5,6,6-trimethyl-2-oxomorpholin-3-yl)acetic acid (0.350 mmol), diisopropylethylamine (0.304 mL; 1.741 mmol), HATU (0.161 g; 0.423 mmol) and 4-isopropylaniline (0.057 mL; 0.417 mmol) in DMF (2.5 mL). Purification by flash chromatography on silica gel using a gradient of ethyl acetate (20 - 60%) in heptane furnished 0.085 g (70%) of the title compound as a white foam.
ESI/APCI(+): 347 (M+H); 369 (M+Na); 715 (2M+Na).
ESI/APCI(-): 345 (M-H).

EXAMPLE 73 - PREPARATION OF *N*-(4-Isopropylphenyl)-2-(4,6,6-triethyl-2-oxomorpholin-3-yl)acetamide

The compound was prepared according to the procedure E from 2-(4,6,6-triethyl-2-oxomorpholin-3-yl)acetic acid (0.334 mmol), diisopropylethylamine (0.290 mL; 1.660 mmol), HATU (0.156 g; 0.402 mmol) and 4-isopropylaniline (0.054 mL; 0.395 mmol) in DMF (2.4 mL). Purification by flash chromatography on silica gel using a gradient of ethyl acetate (10 - 50%) in heptane furnished 0.070 g (58%) of the title compound as a yellow oil.
$^1$H NMR ($CDCl_3$) δ 0.85 (3H, t); 0.95 (3H, t); 1.12 (3H, t); 1.22 (6H, d); 1.66 (2H, q); 1.83 (2H, m); 2.34 (1H, d); 2.42 (1H, m); 2.8-3.0 (5H, m); 3.39 (1H, m); 7.16 (2H, d); 7.40 (2H, d); 8.55 (1 H, brs).
ESI/APCI(+): 361 (M+H); 383 (M+Na); 743 (2M+Na).

EXAMPLE 74 - PREPARATION OF 2-(4-Ethyl-6,6-dimethyl-2-oxomorpholin-3-yl)-*N*-(4-isopropylphenyl)-2-methyl propanamide

The compound was prepared according to the procedure E from 2-(4-ethyl-6,6-dimethyl-2-oxomorpholin-3-yl)-2-methylpropanoic acid (0.177 mmol), diisopropylethylamine (0.290 mL; 0.887 mmol), HATU (0.081 g; 0.213 mmol) and 4-isopropylaniline (0.029 mL; 0.212 mmol) in DMF (2 mL). Purification by flash chromatography on silica gel using a gradient of ethyl acetate (20 - 60%) in heptane furnished 0.033 g (52%) of the title compound as a yellow oil.
$^1$H NMR ($CDCl_3$) δ 1.09 (3H, t); 1.22 (6H, d); 1.36 (3H, s); 1.37 (3H, s); 1.43 (3H, s); 1.45 (3H, s); 2.71 (1H, d); 2.8-3.1 (4H, m); 3.91 (1H, s); 7.18 (2H, d); 7.44 (2H, d); 7.66 (1H, brs).
ESI/APCI(+): 361 (M+H); 383 (M+Na); 743 (2M+Na).
ESI/APCI(-): 359 (M-H).

EXAMPLE 75 - PREPARATION OF 2-(4-Benzyl-6,6-dimethyl-2-oxomorpholin-3-yl)-*N*-(4-isopropylphenyl)acetamide

The compound was prepared according to the procedure E from 2-(4-benzyl-6,6-dimethyl-2-oxomorpholin-3-yl)acetic acid (1.937 mmol), diisopropylethylamine (1.690 mL; 9.677 mmol); HATU (0.893 g; 2.349 mmol) and 4-isopropylaniline (0.320 mL; 2.341 mmol) in DMF (8 mL). Purification by flash chromatography on silica gel using a gradient of ethyl acetate (10 - 50%) in heptane followed by recrystallization from ethyl acetate and heptane furnished 0.592 g (77%) of the title compound as a white powder.
$^1$H NMR ($CDCl_3$) δ 1.23 (6H, d); 1.31 (3H, s); 1.39 (3H, s); 2.31 (2H, d); 2.74 (2H, d); 2.88 (1H, m); 3.0-3.3 (3H, m); 3.50 (1H, m); 4.28 (1H, d); 7.17 (2H, d); 7.31 (5H, m); 7.42 (2H, m); 8.00 (1 H, d).
ESI/APCI(+): 395 (M+H); 417 (M+Na); 811 (2M+Na).

EXAMPLE 76 - PREPARATION OF 2-(6,6-Dimethyl-2-oxomorpholin-3-yl)-*N*-(4-isopropylphenyl)acetamide

To a mixture of 2-(4-benzyl-6,6-dimethyl-2-oxomorpholin-3-yl)-*N*-(4-isopropylphenyl) acetamide (0.500 g; 1.267 mmol) in methyl acetate (20 mL) was added 10% palladium on carbon (0.500 g). The reaction mixture was stirred at room temperature for 5 h under a hydrogen atmosphere and then was filtered through celite. The filtrate was concentrated and the residue was purified by flash chromatography on silica gel using a gradient of methanol (3 - 8%) in dichloromethane to give 0.345 g (89%) of the title compound as a white foam.
$^1$H NMR ($CDCl_3$) δ 1.22 (6H, d); 1.40 (3H, s); 1.52 (3H, s); 2.8-3.1 (6H, m); 3.80 (1 H, m); 7.17 (2H, d); 7.38 (2H, d); 7.61 (1H, s).
ESI/APCI(+): 305 (M+H); 327 (M+Na); 609 (2M+H); 631 (2M+Na).
ESI/APCI(-): 303 (M-H).

EXAMPLE 77 - PREPARATION OF *N*-(4-Isopropylphenyl)-2-(4,6,6-trimethyl-2-oxomorpholin-3-yl)acetamide

The compound was prepared according to the procedure F from 2-(6,6-dimethyl-2-oxomorpholin-3-yl)-*N*-(4-isopropylphenyl)acetamide (0.080 g; 0.263 mmol), a 37% formaldehyde solution in water (0.098 mL; 1.339 mmol), sodium cyanoborohydride (0.033 g; 0.525 mmol) and acetic acid (0.031 mL; 0.542 mmol) in tetrahydrofuran (1.4 mL) and acetonitrile (3.8 mL). Purification by flash chromatography on silica gel using a gradient of ethyl acetate (30 - 80%) in heptane furnished 0.045 g (54%) of the title compound as a white foam.
$^1$H NMR ($CDCl_3$) δ 1.21 (6H, d); 1.39 (3H, s); 1.49 (3H, s); 2.47 (3H, s); 2.49 (1H, d); 2.81 (1H, d); 2.85-2.95 (2H, m); 3.05-3.15 (2H, m); 7.15 (2H, d); 7.40 (2H, m); 8.21 (1H, s).
ESI/APCI(+): 319 (M+H); 341 (M+Na); 659 (2M+Na).
ESI/APCI(-): 317 (M-H).

EXAMPLE 78 - PREPARATION OF 2-(6,6-Dimethyl-2-oxo-4-propylmorpholin-3-yl)-*N*-(4-isopropylphenyl)acetamide

The compound was prepared according to the procedure F from 2-(6,6-dimethyl-2-oxomorpholin-3-yl)-*N*-(4-isopropylphenyl)acetamide (0.080 g; 0.263 mmol), propionaldehyde (0.095 mL; 1.325 mmol), sodium cyanoborohydride (0.033 g; 0.525 mmol) and acetic acid (0.031 mL; 0.542 mmol) in tetrahydrofuran (1.4 mL) and acetonitrile (3.8 mL). Purification by flash chromatography on silica gel using a gradient of ethyl acetate (20 - 70%) in heptane furnished 0.036 g (40%) of the title compound as a white powder.
$^1$H NMR ($CDCl_3$) $\delta$ 0.94 (3H, t); 1.22 (6H, d); 1.41 (3H, s); 1.47 (3H, s); 1.55 (2H, m); 2.34 (2H, m); 2.8-3.1 (5H, m); 3.37 (1H, m); 7.16 (2H, d); 7.41 (2H, m); 8.45 (1H, s). ESI/APCI(+): 347 (M+H); 369 (M+Na); 715 (2M+Na).
ESI/APCI(-): 345 (M-H).

EXAMPLE 79 - PREPARATION OF 2-(4-Cyclopentyl-6,6-dimethyl-2-oxomorpholin-3-yl)-*N*-(4-isopropylphenyl)acetamide

The compound was prepared according to the procedure F from 2-(6,6-dimethyl-2-oxomorpholin-3-yl)-*N*-(4-isopropylphenyl)acetamide (0.080 g; 0.263 mmol), cyclopentanone (0.116 mL; 1.311 mmol), sodium cyanoborohydride (0.033 g; 0.525 mmol) and acetic acid (0.031 mL; 0.542 mmol) in tetrahydrofuran (1.4 mL) and acetonitrile (3.8 mL). Purification by flash chromatography on silica gel using a gradient of ethyl acetate (20 - 50%) in heptane furnished 0.032 g (33%) of the title compound as a white powder.
$^1$H NMR ($CDCl_3$) $\delta$ 1.22 (6H, d); 1.43 (3H, s); 1.44 (3H, s); 1.63 (7H, m); 1.91 (1H, m); 2.54 (1H, d); 2.79 (1H, d); 2.8-3.1 (3H, m); 3.46 (1H, m); 3.75 (1H, m); 7.16 (2H, d); 7.41 (2H, m); 8.67 (1 H, s).
ESI/APCI(+): 373 (M+H); 767 (2M+Na).
ESI/APCI(-): 371 (M-H).

EXAMPLE 80 - PREPARATION OF 2-(4-Isopropyl-6,6-dimethyl-2-oxomorpholin-3-yl)-*N*-(4-isopropylphenyl)acetamide

To a solution of 2-(6,6-dimethyl-2-oxomorpholin-3-yl)-*N*-(4-isopropylphenyl)acetamide (0.080 g; 0.263 mmol) in tetrahydrofuran (1.4 mL) and acetonitrile (3.8 mL) was added acetone (0.097 mL, 1.321 mmol). After 25 min at room temperature, sodium cyanoborohydride (0.033 g; 0.525 mmol) was added portionwise. The reaction mixture was stirred at room temperature for 15 min and acetic acid (0.031 mL; 0.542 mmol) was added. The reaction mixture was stirred at room temperature for 4 days. The reaction was quenched by addition of a saturated sodium bicarbonate solution. The reaction mixture was diluted with ethyl acetate. The phases were separated. The organic phase was washed with water and brine, dried over sodium sulfate, filtered and concentrated. The residue was purified by flash chromatography on silica gel using a gradient of ethyl acetate (20 - 90%) in heptane to give 0.007 g (8%) of the title compound as a white solid.
$^1$H NMR ($CDCl_3$) $\delta$ 0.95 (3H, d); 1.17 (3H, d); 1.22 (6H, d); 1.41 (3H, s); 1.45 (3H, s); 2.42 (1H, d); 2.81 (1H, d); 2.85-3.15 (3H, m); 3.39 (1H, m); 3.62 (1H, m); 7.16 (2H, d); 7.40 (2H, m); 8.52 (1H, s).
ESI/APCI(+): 347 (M+H); 369 (M+Na); 715 (2M+Na).

EXAMPLE 81 - PREPARATION OF 2-(4-Cyclopropyl-6,6-dimethyl-2-oxomorpholin-3-yl)-*N*-(4-isopropylphenyl)acetamide

A mixture of 2-(6,6-dimethyl-2-oxomorpholin-3-yl)-*N*-(4-isopropylphenyl)acetamide (0.080 g; 0.263 mmol), 3A molecular sieves, (1-ethoxycyclopropoxy)trimethylsilane (0.211 mL; 1.049 mmol), sodium cyanoborohydride (0.050 g; 0.796 mmol) and acetic acid (0.151 mL; 2.638 mmol) in tetrahydrofuran (3 mL) was stirred at room temperature for 22 h and refluxed for 4 h. After cooling to room temperature, the reaction was diluted with ethyl acetate and washed with a saturated sodium bicarbonate solution, water and brine. The organic phase was dried over sodium sulfate, filtered and concentrated. The residue was purified by flash chromatography on silica gel using a gradient of ethyl acetate (20 - 60%) in heptane to give 0.047 g (52%) of the title compound as a white powder.
$^1$H NMR ($CDCl_3$) $\delta$ 0.33 (1H, m); 0.56 (1H, m); 0.72 (1H, m); 0.82 (1H, m); 1.22 (6H, d); 1.39 (3H, s); 1.41 (3H, s); 1.77 (1H, m); 2.63 (1H, d); 2.86 (1H, m); 3.01 (1H, m); 3.16 (2H, m); 3.62 (1H, m); 7.16 (2H, d); 7.38 (2H, m); 7.86 (1H, brs).
ESI/APCI(+): 345 (M+H); 367 (M+Na); 711 (2M+Na).
ESI/APCI(+): 343 (M+H).

EXAMPLE 82 - PREPARATION OF 2-(6,6-Dimethyl-2-oxo-4-(2,2,2-trifluoroethyl)morpholin-3-yl)-*N*-(4-isopropylphenyl) acetamide

To a solution of 2-(6,6-dimethyl-2-oxomorpholin-3-yl)-*N*-(4-isopropylphenyl)acetamide (0.072 g; 0.237 mmol)

and diisopropylethylamine (0.051 mL; 0.292 mmol) in tetrahydrofuran (5 mL) was added 2,2,2-trifluoroethyltrifluoromethane sulfonate (0.042 mL; 0.291 mmol). After 24 h at reflux, diisopropylethylamine (0.051 mL; 0.292 mmol) and 2,2,2-trifluoroethyltrifluoromethane sulfonate (0.042 mL; 0.291 mmol) were added. The reaction mixture was refluxed for 24 h and the same amounts of diisopropylethylamine and 2,2,2-trifluoroethyltrifluoromethane sulfonate were added. After 18 h at reflux, the reaction mixture was cooled to room temperature and concentrated to dryness. The residue was partitioned between ethyl acetate and a saturated sodium bicarbonate solution. The phases were-separated. The organic phase was washed with water and brine, dried over sodium sulfate, filtered and concentrated. The residue was purified by flash chromatography on silica gel using a gradient of ethyl acetate (20 - 60%) in heptane and by recrystallization from ethyl acetate and heptane to give 0.017 g (19%) of the title compound as a white powder.
$^1$H NMR ($CDCl_3$) $\delta$ 1.22 (6H, d); 1.41 (3H, s); 1.46 (3H, s); 2.8-3.0 (5H, m); 3.37 (2H, q); 3.86 (1H, t); 7.17 (2H, d); 7.38 (2H, m); 7.64 (1H, brs).
ESI/APCI(+): 387 (M+H); 409 (M+Na).

EXAMPLE 83 - PREPARATION OF 2-(4-(2-Fluoroethyl)-6,6-dimethyl-2-oxomorpholin-3-yl)-*N*-(4-isopropylphenyl)acetamide

To a solution of 2-(6,6-dimethyl-2-oxomorpholin-3-yl)-*N*-(4-isopropylphenyl)acetamide (0.059 g; 0.194 mmol) and diisopropylethylamine (0.100 mL; 0.573 mmol) in tetrahydrofuran (3 mL) was added 1-fluoro-2-iodoethane (0.100 g; 0.575 mmol). After 2 days at 80 °C, diisopropylethylamine (0.033 mL; 0.189 mmol) and 1-fluoro-2-iodoethane (0.033 g; 0.190 mmol) were added. The reaction mixture was heated at 80 °C for 24 h and the same amounts of diisopropylethylamine and 1-fluoro-2-iodoethane were added. After 8 h at 80 °C, diisopropylethylamine (0.100 mL; 0.573 mmol) and 1-fluoro-2-iodoethane (0.100 g; 0.575 mmol) were added. The reaction mixture was heated at 80 °C for 64 h and was concentrated to dryness. The residue was dissolved in ethyl acetate and washed with a saturated sodium bicarbonate solution, water and brine. The organic phase was dried over sodium sulfate, filtered and concentrated. The residue was purified by flash chromatography on silica gel using a gradient of ethyl acetate (20 - 80%) in heptane to give 0.022 g (32%) of the title compound as a white foam.
$^1$H NMR ($CDCl_3$) $\delta$ 1.22 (6H, d); 1.40 (3H, s); 1.46 (3H, s); 1.65 (1H, d); 2.7-3.2 (6H, m); 3.58 (1H, m); 4.55 (1H, m); 4.71 (1H, m); 7.16 (2H, d); 7.39 (2H, m); 7.99 (1H, brs). ESI/APCI(+): 351 (M+H); 373 (M+Na); 723 (2M+Na).

EXAMPLE 84 - PREPARATION OF 2-(4-Acetyl-6,6-dimethyl-2-oxomorpholin-3-yl)-*N*-(4-isopropylphenyl)acetamide

To a mixture of 2-(6,6-dimethyl-2-oxomorpholin-3-yl)-*N*-(4-isopropylphenyl)acetamide (0.080 g; 0.263 mmol) and sodium carbonate (0.084 g; 0.793 mmol) in dichloromethane (5 mL) was added acetyl chloride (0.028 mL; 0.394 mmol). After 3 h at room temperature, sodium carbonate (0.042 g; 0.396 mmol) and acetyl chloride (0.028 mL; 0.394 mmol) were added. The reaction mixture was stirred overnight at room temperature. The reaction mixture was washed with a saturated sodium bicarbonate solution, water and brine. The organic phase was dried over sodium sulfate, filtered and concentrated. The residue was purified by flash chromatography on silica gel using a gradient of methanol (0 - 8%) in dichloromethane and by recrystallization from ethyl acetate to give 0.053 g (58%) of the title compound as a white powder.
$^1$H NMR ($CDCl_3$) $\delta$ 1.21 (6H, d); 1.37 (3H, s); 1.50 (3H, s); 2.14 (3H, s); 2.86 (1H, m); 3.31 (2H, m); 3.55 (1H, d); 4.11 (1H, d); 4.90 (1H, m); 7.16 (2H, d); 7.39 (2H, m); 7.69 (1 H, brs).
ESI/APCI(+): 347 (M+H); 369 (M+Na); 715 (2M+Na).
ESI/APCI(-): 345 (M+H).

EXAMPLE 85 - PREPARATION OF 2-(6,6-Dimethyl-4-(methylsulfonyl)-2-oxomorpholin-3-yl)-*N*-(4-isopropylphenyl) acetamide

To a solution of 2-(6,6-dimethyl-2-oxomorpholin-3-yl)-*N*-(4-isopropylphenyl)acetamide (0.080 g; 0.263 mmol) and diisopropylethylamine (0.092 mL; 0.527 mmol) in dichloromethane (5 mL) cooled at 0 °C was added mesyl chloride (0.031 mL; 0.401 mmol). After 4 h at 0 °C, diisopropylethylamine (0.092 mL; 0.527 mmol) and mesyl chloride (0.031 mL; 0.401 mmol) were added. The reaction mixture was stirred at 0 °C for 4 h and overnight at room temperature. The reaction mixture was diluted with dichloromethane and washed with a saturated sodium bicarbonate solution, water and brine. The organic phase was dried over sodium sulfate, filtered and concentrated. The residue was purified by flash chromatography on silica gel using a gradient of ethyl acetate (20 - 60%) in heptane to give 0.076 g (76%) of the title compound as a white foam.
$^1$H NMR ($CDCl_3$) $\delta$ 1.22 (6H, d); 1.43 (3H, s); 1.49 (3H, s); 2.87 (1H, m); 3.06 (3H, s); 3.21 (2H, m); 3.60 (2H, m); 4.78 (1H, t); 7.18 (2H, d); 7.35 (2H, m); 7.39 (1H, brs).
ESI/APCI(+): 383 (M+H); 405 (M+Na); 787 (2M+Na).

ESI/APCI(-): 381 (M+H).

EXAMPLE 86 - PREPARATION OF 2-(6,6-Dimethyl-2-oxo-4-phenylmorpholin-3-yl)-*N*-(4-isopropylphenyl)acetamide

The compound was prepared according to the procedure E from 2-(6,6-dimethyl-2-oxo-4-phenylmorpholin-3-yl)acetic acid (0.360 mmol), diisopropylethylamine (0.313 mL; 1.792 mmol), HATU (0.166 g; 0.437 mmol) and 4-isopropylaniline (0.059 mL; 0.431 mmol) in DMF (2.6 mL). Purification by flash chromatography on silica gel using a gradient of ethyl acetate (10 - 40%) in heptane followed by crystallization from ethyl acetate and heptane furnished 0.068 g (50%) of the title compound as a white powder.
$^1$H NMR ($CDCl_3$) δ 1.21 (6H, d); 1.39 (3H, s); 1.53 (3H, s); 2.8-3.1 (3H, m); 3.49 (2H, s); 4.75 (1 H, m); 6.90 (3H, m); 7.14 (2H, d); 7.33 (4H, m); 7.51 (1 H, brs).
ESI/APCI(+): 381 (M+H); 403 (M+Na); 783 (2M+Na).
ESI/APCI(-): 379 (M-H).

EXAMPLE 87 - PREPARATION OF 4-Ethyl-6,6-dimethyl-3-(2-morpholino-2-oxoethyl)morpholin-2-one

The compound was prepared according to the procedure E from 2-(4-ethyl-6,6-dimethyl-2-oxomorpholin-3-yl)acetic acid (0.277 mmol), diisopropylethylamine (0.240 mL; 1.374 mmol), HATU (0.125 g; 0.328 mmol) and morpholine (0.040 mL; 0.457 mmol) in DMF (1 mL). Purification by flash chromatography on silica gel using a gradient of ethyl acetate (25 - 100%) in heptane furnished 0.038 g (48%) of the title compound as a yellow oil.
$^1$H NMR ($CDCl_3$): δ 1.06 (3H, t); 1.39 (3H, s); 1.53 (3H, s); 2.36-2.41 (2H, m); 2.72-3.08 (4H, m); 3.52 (2H, m); 3.59-3.68 (7H, m).
ESI/APCI(+): 285 (M+H).

EXAMPLE 88 - PREPARATION OF 2-(4-Ethyl-6,6-dimethyl-2-oxomorpholin-3-yl)-N-(4-isopropylphenyl)acetamide

The compound was prepared according to the procedure E from 2-(4-ethyl-6,6-dimethyl-2-oxomorpholin-3-yl)acetic acid (0.442 mmol), diisopropylethylamine (0.385 mL; 2.204 mmol), HATU (0.203 g; 0.534 mmol) and 4-isopropylaniline (0.074 mL; 0.541 mmol) in DMF (2.9 mL). Purification by flash chromatography on silica gel using a gradient of ethyl acetate (30 - 80%) in heptane furnished 0.132 g (90%) of the title compound as a beige powder.
$^1$H NMR ($CDCl_3$): δ 1.11 (3H, t); 1.22 (6H, d); 1.41 (3H, s); 1.47 (3H, s); 2.4-2.55 (2H, m); 2.8-3.1 (5H, m); 3.41 (1H, m); 7.16 (2H, d); 7.40 (2H, d); 8.49 (1H, s).
ESI/APCI(+): 333 (M+H); 355 (M+Na).
ESI/APCI(-): 331 (M-H).

All other compounds of the invention can be prepared with the same procedures as described herein.

## Claims

**1.** A compound having a structure according to the formula (A):

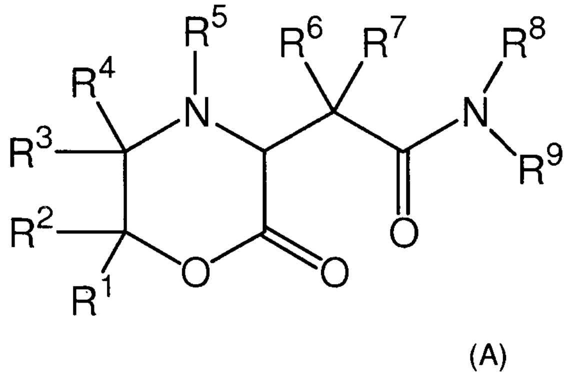

(A)

wherein,

- each $R^1$ and $R^2$ is independently selected from hydrogen; alkyl; alkenyl; alkynyl; heteroalkyl; heteroalkenyl;

heteroalkynyl; aryl; arylalkyl; arylalkenyl; arylalkynyl; arylheteroalkyl; arylheteroalkenyl; and arylheteroalkynyl; provided that at least one of $R^1$ and $R^2$ is not hydrogen;
- each $R^3$ and $R^4$ is independently selected from hydrogen; alkyl; alkenyl; alkynyl; heteroalkyl; heteroalkenyl; and heteroalkynyl;
- $R^5$ is independently selected from hydrogen; alkyl; alkenyl; alkynyl; heteroalkyl; heteroalkenyl; heteroalkynyl; aryl; arylalkyl; arylalkenyl; arylalkynyl; $-C(O)R^{10}$; and - $S(O)_2R^{10}$;
wherein each of said alkyl, alkenyl, alkynyl, heteroalkyl, heteroalkenyl, heteroalkynyl, aryl, arylalkyl, arylalkenyl, and arylalkynyl can be unsubstituted or substituted with one or more substituents each independently selected from alkyl, alkenyl, alkynyl, heteroalkyl, heteroalkenyl, heteroalkynyl, hydroxyl, =O, halogen, - SH, =S, trifluoromethyl, $-OCF_3$, cyano, nitro, -C(O)OH, or $-NH_2$;
- each $R^6$ and $R^7$ is independently selected from hydrogen; alkyl; alkenyl; alkynyl; heteroalkyl; heteroalkenyl; and heteroalkynyl;
- each $R^8$ and $R^9$ is independently selected from hydrogen; alkyl; alkenyl; alkynyl; heteroalkyl; heteroalkenyl; heteroalkynyl; aryl; arylalkyl; arylalkenyl; arylalkynyl; arylheteroalkyl; arylheteroalkenyl; arylheteroalkynyl; heterocycle; heterocycle-alkyl; heterocycle-alkenyl; heterocylce-alkynyl; heterocycle-heteroalkyl; heterocycle-heteroalkenyl; and heterocycle-heteroalkynyl; and wherein $R^8$ and $R^9$ can be taken together in order to form a (5-, 6-, or 7-membered) heterocycle;
provided that at least one of $R^8$ and $R^9$ is not hydrogen; and
wherein each of said alkyl, alkenyl, alkynyl, heteroalkyl, heteroalkenyl, heteroalkynyl, aryl, arylalkyl, arylalkenyl, arylalkynyl, arylheteroalkyl, arylheteroalkenyl, arylheteroalkynyl, heterocycle, heterocycle-alkyl, heterocycle-alkenyl, heterocylce-alkynyl, heterocycle-heteroalkyl, heterocycle-heteroalkenyl, and heterocycle-heteroalkynyl, can be unsubstituted or substituted with one or more $R^{11}$;
- each $R^{10}$ is independently selected from hydrogen; alkyl; alkenyl; alkynyl; heteroalkyl; heteroalkenyl; heteroalkynyl; aryl; arylalkyl; arylalkenyl; and arylalkynyl;
wherein each of said alkyl, alkenyl, alkynyl, heteroalkyl, heteroalkenyl, heteroalkynyl, aryl, arylalkyl, arylalkenyl, and arylalkynyl can be unsubstituted or substituted with one or more substituents each independently selected from alkyl, alkenyl, alkynyl, heteroalkyl, heteroalkenyl, heteroalkynyl, hydroxyl, =O, halogen,-SH, =S, trifluoromethyl, $-OCF_3$, cyano, nitro, -C(O)OH, or $-NH_2$;
- each $R^{11}$ is independently selected from the group consisting of halogen; hydroxyl; sulfhydryl; $-OZ^2$; =O; $-SZ^2$; =S; $-S(O)Z^2$; $-S(O)_2Z^3$; $-S(O)_2NZ^4Z^5$; trifluoromethyl; nitro;$-NZ^4Z^5$; $-NZ^4S(O)_2Z^2$; $-NZ^4C(O)Z^2$; $-NZ^4C(O)NZ^4Z^5$; cyano; $-C(O)Z^3$; $-C(O)NZ^4Z^5$; -C(O)H; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl, heterocycle-heteroalkenyl; or heterocycle-heteroalkynyl;
and wherein said alkyl, cyclic alkyl, alkenyl, cyclic alkenyl, alkynyl, cyclic alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more substituents selected from the group of alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH, or $-NH_2$;
- each $Z^2$ is independently selected from alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl; heterocycle-heteroalkenyl; or heterocycle-heteroalkynyl;
wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with alkyl, alkenyl, alkynyl, heteroalkyl, heteroalkenyl, heteroalkynyl, hydroxyl, =O, halogen, -SH, =S, trifluoromethyl,$-OCF_3$, cyano, nitro, -C(O)OH, or $-NH_2$;
- each $Z^3$ is independently selected from hydroxyl; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl; heterocycle-heteroalkenyl; or heterocycle-heteroalkynyl;
wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more selected from the group of alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH, or

$-NH_2$;

- each $Z^4$ and $Z^5$ is independently selected from hydrogen; alkyl; alkenyl; alkynyl; heteroalkyl; heteroalkenyl; heteroalkynyl; aryl; heterocycle; arylalkyl; arylalkenyl; arylalkynyl; arylheteroalkyl; arylheteroalkenyl; arylheteroalkynyl; heterocycle-alkyl; heterocycle-alkenyl; heterocycle-alkynyl; heterocycle-heteroalkyl; heterocycle-heteroalkenyl; or heterocycle-heteroalkynyl;

wherein said alkyl, alkenyl, alkynyl, heteroalkyl, heteroalkenyl, heteroalkynyl, aryl, heterocycle, arylalkyl, arylalkenyl, arylalkynyl, arylheteroalkyl, arylheteroalkenyl, arylheteroalkynyl, heterocycle-alkyl, heterocycle-alkenyl, heterocycle-alkynyl, heterocycle-heteroalkyl, heterocycle-heteroalkenyl, or heterocycle-heteroalkynyl can be unsubstituted or substituted with one or more selected from the group of alkyl, alkenyl, alkynyl, heteroalkyl, heteroalkenyl, heteroalkynyl, hydroxyl, =O, halogen, -SH, =S, trifluoromethyl, $-OCF_3$, cyano, nitro, -C(O)OH, or $-NH_2$; and wherein $Z^4$ and $Z^5$ can be taken together in order to form a (5-, 6-, or 7-membered) heterocycle which can be unsubstituted or substituted with alkyl, alkenyl, alkynyl, heteroalkyl, heteroalkenyl, heteroalkynyl, hydroxyl, =O, halogen, - SH, =S, trifluoromethyl, $-OCF_3$, cyano, nitro, -C(O)OH, or $-NH_2$; and stereo-isomers, tautomers solvates, or pharmaceutically acceptable salts thereof.

**2.** The compound according to claim 1 wherein $R^5$ is alkyl.

**3.** The compound according to claims 1 and 2 wherein $R^8$ is hydrogen.

**4.** The compound according to claims 1 to 3, wherein $R^3$ and $R^4$ are hydrogen.

**5.** The compound according to claims 1 to 4, wherein $R^1$ and $R^2$ are each alkyl.

**6.** The compound according to claims 1 to 5, wherein the compound has a structure according to formula (Fa) to (Fd):

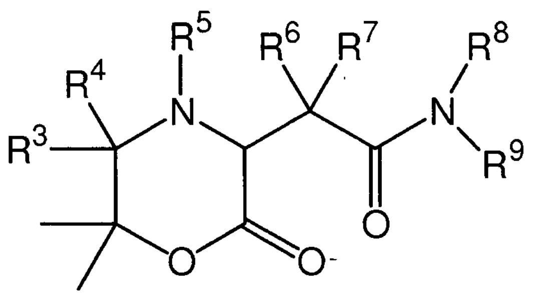

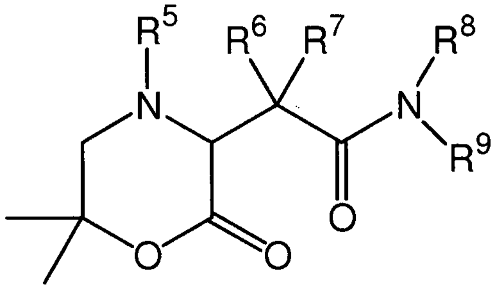

(Fa) (Fb)

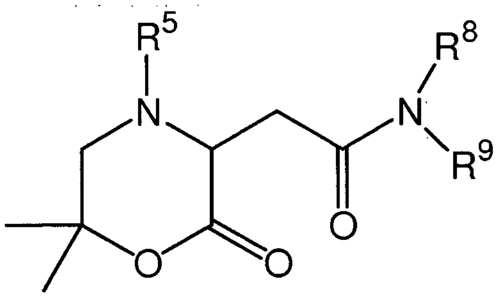

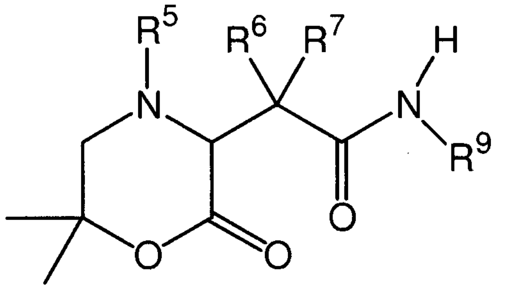

(Fc) (Fd).

**7.** The compound according to claims 1 to 6 selected from the list of

2-((4-ethyl-6,6-dimethyl-2-oxomorpholin-3-yl)-N-(1-methyl-1H-indol-5-yl)acetamide;
N-(4-chlorobenzyl)-2-(4-ethyl-6,6-dimethyl-2-oxomorpholin-3-yl)acetamide;
N-(2,3-dihydro-1H-inden-1-yl)-2-(4-ethyl-6,6-dimethyl-2-oxomorpholin-3-yl)acetamide;
N-(benzo[d]thiazol-5-yl)-2-(4-ethyl-6,6-dimethyl-2-oxomorpholin-3-yl)acetamide;
2-((4-ethyl-6,6-dimethyl-2-oxomorpholin-3-yl)-N-(1,2,3,4-tetrahydronaphthalen-1-yl)acetamide;
N-benzyl-2-(4-ethyl-6,6-dimethyl-2-oxomorpholin-3-yl)acetamide;
2-((4-ethyl-6,6-dimethyl-2-oxomorpholin-3-yl)-N-(pyridin-3-yl)acetamide;
N-(1-benzylpiperidin-4-yl)-2-(4-ethyl-6,6-dimethyl-2-oxomorpholin-3-yl)acetamide;
2-((4-ethyl-6,6-dimethyl-2-oxomorpholin-3-yl)-N-(1-phenylethyl)acetamide;
3-((2-(4-benzylpiperidin-1-yl)-2-oxoethyl)-4-ethyl-6,6-dimethylmorpholin-2-one;
2-((4-ethyl-6,6-dimethyl-2-oxomorpholin-3-yl)-N-(pyridin-4-yl)acetamide;
2-((4-ethyl-6,6-dimethyl-2-oxomorpholin-3-yl)-N-(quinazolin-2-yl)acetamide;
2-((4-ethyl-6,6-dimethyl-2-oxomorpholin-3-yl)-N-phenethylacetamide;
4-ethyl-6,6-dimethyl-3-(2-oxo-2-(4-phenylpiperazin-1-yl)ethyl)morpholin-2-one;
2-((4-ethyl-6,6-dimethyl-2-oxomorpholin-3-yl)-N-(pyridin-2-yl)acetamide;
2-((4-ethyl-6,6-dimethyl-2-oxomorpholin-3-yl)-N-(thiophen-2-ylmethyl)acetamide;
2-((4-ethyl-6,6-dimethyl-2-oxomorpholin-3-yl)-N-(furan-2-ylmethyl)acetamide;
2-((4-ethyl-6,6-dimethyl-2-oxomorpholin-3-yl)-N-methyl-N-phenylacetamide;
2-((4-ethyl-6,6-dimethyl-2-oxomorpholin-3-yl)-N-(pyrimidin-4-yl)acetamide;
N-(3,5-dimethylisoxazol-4-yl)-2-(4-ethyl-6,6-dimethyl-2-oxomorpholin-3-yl)acetamide;
2-((4-ethyl-6,6-dimethyl-2-oxomorpholin-3-yl)-N-(1-methyl-1H-pyrazol-5-yl)acetamide;
2-((4-ethyl-6,6-dimethyl-2-oxomorpholin-3-yl)-N-(3-phenyl-1,2,4-thiadiazol-5-yl)acetamide;
2-((4-ethyl-6,6-dimethyl-2-oxomorpholin-3-yl)-N-(1-methyl-1H-pyrazol-3-yl)acetamide;
2-((4-ethyl-6;6-dimethyl-2-oxomorpholin-3-yl)-N-(5-(trifluoromethyl)-1,3,4-thiadiazol-2-yl)acetamide;
4-ethyl-6,6-dimethyl-3-(2-oxo-2-(1H-pyrazol-1-yl)ethyl)morpholin-2-one;
N-(4,5-dimethylthiazol-2-yl)-2-(4-ethyl-6,6-dimethyl-2-oxomorpholin-3-yl)acetamide;
N,N-diethyl-2-(4-ethyl-6,6-dimethyl-2-oxomorpholin-3-yl)acetamide;
2-((4-ethyl-6,6-dimethyl-2-oxomorpholin-3-yl)-N-isobutylacetamide;
N-cyclohexyl-2-(4-ethyl-6,6-dimethyl-2-oxomorpholin-3-yl)acetamide;
2-((4-ethyl-6,6-dimethyl-2-oxomorpholin-3-yl)-N-propylacetamide;
N-tert-butyl-2-(4-ethyl-6,6-dimethyl-2-oxomorpholin-3-yl)acetamide;
N-(3,3-dimethylbutyl)-2-(4-ethyl-6,6-dimethyl-2-oxomorpholin-3-yl)acetamide;

2-((4-ethyl-6,6-dimethyl-2-oxomorpholin-3-yl)-N-(2,2,2-trifluoroethyl)acetamide;
N-cyclopentyl-2-(4-ethyl-6,6-dimethyl-2-oxomorpholin-3-yl)acetamide;
4-ethyl-6,6-dimethyl-3-(2-oxo-2-(pyrrolidin-1-yl)ethyl)morpholin-2-one;
N-ethyl-2-(4-ethyl-6,6-dimethyl-2-oxomorpholin-3-yl)acetamide;
N-sec-butyl-2-(4-ethyl-6,6-dimethyl-2-oxomorpholin-3-yl)acetamide;
N-(cyclohexylmethyl)-2-(4-ethyl-6,6-dimethyl-2-oxomorpholin-3-yl)acetamide;.
2-((4-ethyl-6,6-dimethyl-2-oxomorpholin-3-yl)-N,N-dimethylacetamide;
N-(3-chlorobenzyl)-2-(4-ethyl-6,6-dimethyl-2-oxomorpholin-3-yl)acetamide;
N-(3,4-dichlorobenzyl)-2-(4-ethyl-6,6-dimethyl-2-oxomorpholin-3-yl)acetamide;
N-(2,6-dichlorobenzyl)-2-(4-ethyl-6,6-dimethyl-2-oxomorpholin-3-yl)acetamide;
N-(2-chlorobenzyl)-2-(4-ethyl-6,6-dimethyl-2-oxomorpholin-3-yl)acetamide;
4-ethyl-6,6-dimethyl-3-(2-(4-methylpiperazin-1-yl)-2-oxoethyl)morpholin-2-one;
N-cyclopropyl-2-(4-ethyl-6,6-dimethyl-2-oxomorpholin-3-yl)acetamide;
2-((4-ethyl-6,6-dimethyl-2-oxomorpholin-3-yl)-N-(4-phenoxyphenyl)acetamide;
N-(4-tert-butylthiazol-2-yl)-2-(4-ethyl-6,6-dimethyl-2-oxomorpholin-3-yl)acetamide;
2-((4-ethyl-6,6-dimethyl-2-oxomorpholin-3-yl)-N-(2-methylbenzyl)acetamide;
2-((4-ethyl-6,6-dimethyl-2-oxomorpholin-3-yl)-N-(3-methylbenzyl)acetamide;
2-((4-ethyl-6,6-dimethyl-2-oxomorpholin-3-yl)-N-(4-methylbenzyl)acetamide;
2-((4-ethyl-6,6-dimethyl-2-oxomorpholin-3-yl)-N-(2-(trifluoromethyl)benzyl)acetamide;
2-((4-ethyl-6,6-dimethyl-2-oxomorpholin-3-yl)-N-(3-(trifluoromethyl)benzyl)acetamide;
2-((4-ethyl-6,6-dimethyl-2-oxomorpholin-3-yl)-N-(4-(trifluoromethyl)benzyl)acetamide;
2-((4-ethyl-6,6-dimethyl-2-oxomorpholin-3-yl)-N-(2-methoxybenzyl)acetamide;
2-((4-ethyl-6,6-dimethyl-2-oxomorpholin-3-yl)-N-(3-methoxybenzyl)acetamide;
2-((4-ethyl-6,6-dimethyl-2-oxomorpholin-3-yl)-N-(4-methoxybenzyl)acetamide;
N-(biphenyl-2-ylmethyl)-2-(4-ethyl-6,6-dimethyl-2-oxomorpholin-3-yl)acetamide;
N-(biphenyl-3-ylmethyl)-2-(4-ethyl-6,6-dimethyl-2-oxomorpholin-3-yl)acetamide;
N-(biphenyl-4-ylmethyl)-2-(4-ethyl-6,6-dimethyl-2-oxomorpholin-3-yl)acetamide;
2-((4-ethyl-2-oxo-6-phenylmorpholin-3-yl)-N-(4-isopropylphenyl)acetamide;
2-(((6S)-4-ethyl-2-oxo-6-phenylmorpholin-3-yl)-N-(4-isopropylphenyl)acetamide;
2-(((6R)-4-ethyl-2-oxo-6-phenylmorpholin-3-yl)-N-(4-isopropylphenyl)acetamide;
2-(((6S)-4-ethyl-6-methyl-2-oxomorpholin-3-yl)-N-(4-isopropylphenyl)acetamide;
2-(((6R)-4-ethyl-6-methyl-2-oxomorpholin-3-yl)-N-(4-isopropylphenyl)acetamide;
2-((4-ethyl-6-methyl-2-oxo-6-phenylmorpholin-3-yl)-N-(4-isopropylphenyl)acetamide;
2-((4-ethyl-6-methyl-2-oxo-6-phenylmorpholin-3-yl)-N-(4-isopropylphenyl)acetamide;
2-((4-ethyl-5,5,6,6-tetramethyl-2-oxomorpholin-3-yl)-N-(4-isopropylphenyl)acetamide;
2-((4-ethyl-5,6,6-trimethyl-2-oxomorpholin-3-yl)-N-(4-isopropylphenyl)acetamide;
N-(4-isopropylphenyl)-2-(4,6,6-triethyl-2-oxomorpholin-3-yl)acetamide;
2-((4-ethyl-6,6-dimethyl-2-oxomorpholin-3-yl)-N-(4-isopropylphenyl)-2-methylpropanamide;
2-((4-benzyl-6,6-dimethyl-2-oxomorpholin-3-yl)-N-(4-isopropylphenyl)acetamide;
2-((6,6-dimethyl-2-oxomorpholin-3-yl)-N-(4-isopropylphenyl)acetamide;
N-(4-isopropylphenyl)-2-(4,6,6-trimethyl-2-oxomorpholin-3-yl)acetamide;
2-((6,6-dimethyl-2-oxo-4-propylmorpholin-3-yl)-N-(4-isopropylphenyl)acetamide;
2-((4-cyclopentyl-6,6-dimethyl-2-oxomorpholin-3-yl)-N-(4-isopropylphenyl)acetamide;
2-((4-isopropyl-6,6-dimethyl-2-oxomorpholin-3-yl)-N-(4-isopropylphenyl)acetamide;
2-((4-cyclopropyl-6,6-dimethyl-2-oxomorpholin-3-yl)-N-(4-isopropylphenyl)acetamide;
2-((6,6-dimethyl-2-oxo-4-(2,2,2-trifluoroethyl)morpholin-3-yl)-N-(4-isopropylphenyl)acetamide;
2-((4-(2-fluoroethyl)-6,6-dimethyl-2-oxomorpholin-3-yl)-N-(4-isopropylphenyl)acetamide;
2-((4-acetyl-6,6-dimethyl-2-oxomorpholin-3-yl)-N-(4-isopropylphenyl)acetamide;
2-((6,6-dimethyl-4-(methylsulfonyl)-2-oxomorpholin-3-yl)-N-(4-isopropylphenyl)acetamide;
2-((6,6-dimethyl-2-oxo-4-phenylmorpholin-3-yl)-N-(4-isopropylphenyl)acetamide;
2-((4-ethyl-6,6-dimethyl-2-oxomorpholin-3-yl)-N-(4-isopropylphenyl)acetamide;
4-ethyl-6,6-dimethyl-3-(2-morpholino-2-oxoethyl)morpholin-2-one.

**8.** The compound according to claims 1 to 7 for use as a medicament.

**9.** The compound according to claim 8, wherein the medicament is for the prevention or treatment of fungal infection in a subject.

**10.** The compound according to claim 9, wherein said fungal infection is caused by Candida species, Aspergillus species or Fusarium species.

**11.** The compound according to claim 10, wherein said fungal infection is a systemic infection.

**12.** The compound according to claim 10, wherein said fungal infection is a topical infection.

**13.** A pharmaceutical composition comprising a compound according to claims 1 to 7 as an active ingredient in admixture with at least a pharmaceutically acceptable carrier.

**14.** A pharmaceutical composition according to claim 13 having antifungal activity.

**15.** A method for the preparation of the compound according to claims 1 to 7, comprising the steps of:

- preparing a substituted morpholin-2-one derivative by condensation of a suitable amino-alcohol derivative with glyoxal or with a suitable acetate derivative of formula LG-$CH_2$-COOR (R being preferably an alkyl or a benzyl radical)
- reacting this morpholin-2-one derivative with a suitable acetate derivative of formula LG-$CH_2$-COOR (R being preferably an alkyl or a benzyl radical) in order to obtain a 2-(substituted-2-oxomorpholin-3-yl)acetate derivative;
- converting the obtained ester in a carboxylic acid derivative via a saponification reaction
- converting the acid derivative in a desired amide derivative using standard peptide-coupling reagents.
- preparing a 2-(4-benzyl-2-oxomorpholin-3-yl)-N-substituted acetamide derivative using the previous detailed step
- removing the N-benzyl protecting group from the morpholin-2-one derivative in order to obtain a 2-(2-oxomorpholin-3-yl)-N-substituted acetamide derivative
- condensing the 2-(2-oxomorpholin-3-yl)-N-substituted acetamide derivative with a suitable reagent in order to substitute the free nitrogen atom from the morpholin-2-one ring and obtain the desired compounds.

## Patentansprüche

**1.** Verbindung mit einer Struktur gemäß der Formel (A):

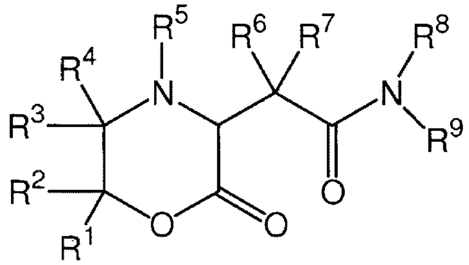

(A)

worin

- jedes $R^1$ und $R^2$ unabhängig aus Wasserstoff; Alkyl; Alkenyl; Alkinyl; Heteroalkyl; Heteroalkenyl; Heteroalkinyl; Aryl; Arylalkyl; Arylalkenyl; Arylalkinyl; Arylheteroalkyl; Arylheteroalkenyl und Arylheteroalkinyl ausgewählt ist; mit der Maßgabe, dass mindestens einer der Reste $R^1$ und $R^2$ nicht für Wasserstoff steht;
- jedes $R^3$ und $R^4$ unabhängig aus Wasserstoff; Alkyl; Alkenyl; Alkinyl; Heteroalkyl; Heteroalkenyl und Heteroalkinyl ausgewählt ist;
- $R^5$ unabhängig aus Wasserstoff; Alkyl; Alkenyl; Alkinyl; Heteroalkyl; Heteroalkenyl; Heteroalkinyl; Aryl; Arylalkyl; Arylalkenyl; Arylalkinyl; -C(O)$R^{10}$ und -S(O)$_2$$R^{10}$ ausgewählt ist;

wobei das Alkyl, Alkenyl, Alkinyl, Heteroalkyl, Heteroalkenyl, Heteroalkinyl, Aryl, Arylalkyl, Arylalkenyl und Arylalkinyl jeweils unsubstituiert oder durch einen oder mehrere Substituenten, die unabhängig voneinander aus Alkyl, Alkenyl, Alkinyl, Heteroalkyl, Heteroalkenyl, Heteroalkinyl, Hydroxyl, =O, Halogen,-SH, =S, Trifluormethyl, -$OCF_3$, Cyano, Nitro, -C(O)OH oder -$NH_2$ ausgewählt sind, substituiert sein kann;
- jedes $R^6$ und $R^7$ unabhängig aus Wasserstoff; Alkyl; Alkenyl; Alkinyl; Heteroalkyl; Heteroalkenyl und Hete-

roalkinyl ausgewählt ist;
- jedes $R^8$ und $R^9$ unabhängig aus Wasserstoff; Alkyl; Alkenyl; Alkinyl; Heteroalkyl; Heteroalkenyl; Heteroalkinyl; Aryl; Arylalkyl; Arylalkenyl; Arylalkinyl; Arylheteroalkyl; Arylheteroalkenyl; Arylheteroalkinyl; Heterocyclyl; Heterocyclyl-alkyl; Heterocyclyl-alkenyl; Heterocyclyl-alkinyl; Heterocyclyl-heteroalkyl; Heterocyclyl-heteroalkenyl und Heterocyclyl-heteroalkinyl ausgewählt ist und wobei $R^8$ und $R^9$ zusammengenommen einen (5-, 6- oder 7-gliedrigen) Heterocyclus bilden können;
mit der Maßgabe, dass mindestens einer der Reste $R^8$ und $R^9$ nicht für Wasserstoff steht; und
wobei das Alkyl, Alkenyl, Alkinyl, Heteroalkyl, Heteroalkenyl, Heteroalkinyl, Aryl, Arylalkyl, Arylalkenyl, Arylalkinyl, Arylheteroalkyl, Arylheteroalkenyl, Arylheteroalkinyl, Heterocyclyl, Heterocyclyl-alkyl, Heterocyclyl-alkenyl, Heterocyclyl-alkinyl, Heterocyclyl-heteroalkyl, Heterocyclyl-heteroalkenyl und Heterocyclyl-heteroalkinyl jeweils unsubstituiert oder durch ein oder mehrere $R^{11}$ substituiert sein kann;
- jedes $R^{10}$ unabhängig aus Wasserstoff; Alkyl; Alkenyl; Alkinyl; Heteroalkyl; Heteroalkenyl; Heteroalkinyl; Aryl; Arylalkyl; Arylalkenyl und Arylalkinyl ausgewählt ist;
wobei das Alkyl, Alkenyl, Alkinyl, Heteroalkyl, Heteroalkenyl, Heteroalkinyl, Aryl, Arylalkyl, Arylalkenyl und Arylalkinyl jeweils unsubstituiert oder durch einen oder mehrere Substituenten, die unabhängig voneinander aus Alkyl, Alkenyl, Alkinyl, Heteroalkyl, Heteroalkenyl, Heteroalkinyl, Hydroxyl, =O, Halogen,-SH, =S, Trifluormethyl, $-OCF_3$, Cyano, Nitro, -C(O)OH oder $-NH_2$ ausgewählt sind, substituiert sein kann;
- jedes $R^{11}$ unabhängig aus der Gruppe bestehend aus Halogen; Hydroxyl; Sulfhydryl; $-OZ^2$; =O; $-SZ^2$; =S; $-S(O)Z^2$, $-S(O)_2Z^3$, $-S(O)_2NZ^4Z^5$; Trifluormethyl; Nitro; $-NZ^4Z^5$;$-NZ^4S(O)_2Z^2$; $-NZ^4C (O) Z^2$; $-NZ^4C (O) NZ^4Z^5$; Cyano; $-C(O)Z^3$;$-C(O)NZ^4Z^5$; -C(O)H; Alkyl; Alkenyl; Alkinyl; Heteroalkyl; Heteroalkenyl; Heteroalkinyl; Aryl; Heterocyclyl; Arylalkyl; Arylalkenyl; Arylalkinyl; Arylheteroalkyl; Arylheteroalkenyl; Arylheteroalkinyl; Heterocyclyl-alkyl; Heterocyclyl-alkenyl; Heterocyclyl-alkinyl; Heterocyclylheteroalkyl; Heterocyclyl-heteroalkenyl oder Heterocyclylheteroalkinyl ausgewählt ist;
und wobei das Alkyl, cyclische Alkyl, Alkenyl, cyclische Alkenyl, Alkinyl, cyclische Alkinyl, Heteroalkyl, Heteroalkenyl, Heteroalkinyl, Aryl, Heterocyclyl, Arylalkyl, Arylalkenyl, Arylalkinyl, Arylheteroalkyl, Arylheteroalkenyl, Arylheteroalkinyl, Heterocyclyl-alkyl, Heterocyclyl-alkenyl, Heterocyclyl-alkinyl, Heterocyclyl-heteroalkyl, Heterocyclyl-heteroalkenyl oder Heterocyclyl-heteroalkinyl unsubstituiert oder durch einen oder mehrere Substituenten, die unabhängig voneinander aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $-NH_2$ ausgewählt sind, substituiert sein kann;
- jedes $Z^2$ unabhängig aus Alkyl; Alkenyl; Alkinyl; Heteroalkyl; Heteroalkenyl; Heteroalkinyl; Aryl; Heterocyclyl; Arylalkyl; Arylalkenyl; Arylalkinyl; Arylheteroalkyl; Arylheteroalkenyl; Arylheteroalkinyl; Heterocyclyl-alkyl; Heterocyclyl-alkenyl; Heterocyclyl-alkinyl; Heterocyclyl-heteroalkyl; Heterocyclyl-heteroalkenyl oder Heterocyclyl-heteroalkinyl ausgewählt ist;
wobei das Alkyl, Alkenyl, Alkinyl, Heteroalkyl, Heteroalkenyl, Heteroalkinyl, Aryl, Heterocyclyl, Arylalkyl, Arylalkenyl, Arylalkinyl, Arylheteroalkyl, Arylheteroalkenyl, Arylheteroalkinyl, Heterocyclyl-alkyl, Heterocyclyl-alkenyl, Heterocyclyl-alkinyl, Heterocyclyl-heteroalkyl, Heterocyclyl-heteroalkenyl oder Heterocyclyl-heteroalkinyl unsubstituiert oder durch Alkyl, Alkenyl, Alkinyl, Heteroalkyl, Heteroalkenyl, Heteroalkinyl, Hydroxyl, =O, Halogen,-SH, =S, Trifluormethyl, $-OCF_3$, Cyano, Nitro, -C(O)OH oder $-NH_2$ substituiert sein kann;
- jedes $Z^3$ unabhängig aus Hydroxyl; Alkyl; Alkenyl; Alkinyl; Heteroalkyl; Heteroalkenyl; Heteroalkinyl; Aryl; Heterocyclyl; Arylalkyl; Arylalkenyl; Arylalkinyl; Arylheteroalkyl; Arylheteroalkenyl; Arylheteroalkinyl; Heterocyclyl-alkyl; Heterocyclyl-alkenyl; Heterocyclyl-alkinyl; Heterocyclyl-heteroalkyl; Heterocyclyl-heteroalkenyl oder Heterocyclyl-heteroalkinyl ausgewählt ist;
wobei das Alkyl, Alkenyl, Alkinyl, Heteroalkyl, Heteroalkenyl, Heteroalkinyl, Aryl, Heterocyclyl, Arylalkyl, Arylalkenyl, Arylalkinyl, Arylheteroalkyl, Arylheteroalkenyl, Arylheteroalkinyl, Heterocyclyl-alkyl, Heterocyclyl-alkenyl, Heterocyclyl-alkinyl, Heterocyclyl-heteroalkyl, Heterocyclyl-heteroalkenyl oder Heterocyclyl-heteroalkinyl unsubstituiert oder durch einen oder mehrere Substituenten, die aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $-NH_2$ ausgewählt sind, substituiert sein kann;
- jedes $Z^4$ und $Z^5$ unabhängig aus Wasserstoff; Alkyl; Alkenyl; Alkinyl; Heteroalkyl; Heteroalkenyl; Heteroalkinyl; Aryl; Heterocyclyl; Arylalkyl; Arylalkenyl; Arylalkinyl; Arylheteroalkyl; Arylheteroalkenyl; Arylheteroalkinyl; Heterocyclyl-alkyl; Heterocyclyl-alkenyl; Heterocyclyl-alkinyl; Heterocyclyl-heteroalkyl; Heterocyclyl-heteroalkenyl oder Heterocyclyl-heteroalkinyl ausgewählt ist;
wobei das Alkyl, Alkenyl, Alkinyl, Heteroalkyl, Heteroalkenyl, Heteroalkinyl, Aryl, Heterocyclyl, Arylalkyl, Arylalkenyl, Arylalkinyl, Arylheteroalkyl, Arylheteroalkenyl, Arylheteroalkinyl, Heterocyclyl-alkyl, Heterocyclyl-alkenyl, Heterocyclyl-alkinyl, Heterocyclyl-heteroalkyl, Heterocyclyl-heteroalkenyl oder Heterocyclyl-heteroalkinyl unsubstituiert oder durch einen oder mehrere Substituenten, die aus Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $-NH_2$

ausgewählt sind, substituiert sein kann;
und wobei $Z^4$ und $Z^5$ zusammengenommen einen (5-, 6- oder 7-gliedrigen) Heterocyclus bilden können, der unsubstituiert oder durch Alkyl, Alkenyl, Alkinyl, Heteroalkyl, Heteroalkenyl, Heteroalkinyl, Hydroxyl, =O, Halogen, -SH, =S, Trifluormethyl, $-OCF_3$, Cyano, Nitro, -C(O)OH oder $-NH_2$ substituiert sein kann;
und Stereoisomere, Tautomere, Solvate oder pharmazeutisch unbedenkliche Salze davon.

**2.** Verbindung nach Anspruch 1, wobei $R^5$ für Alkyl steht.

**3.** Verbindung nach den Ansprüchen 1 und 2, wobei $R^8$ für Wasserstoff steht.

**4.** Verbindung nach den Ansprüchen 1 bis 3, wobei $R^3$ und $R^4$ für Wasserstoff stehen.

**5.** Verbindung nach den Ansprüchen 1 bis 4, wobei $R^1$ und $R^2$ jeweils für Alkyl stehen.

**6.** Verbindung nach den Ansprüchen 1 bis 5, wobei die Verbindung eine Struktur gemäß Formel (Fa) bis (Fd) aufweist:

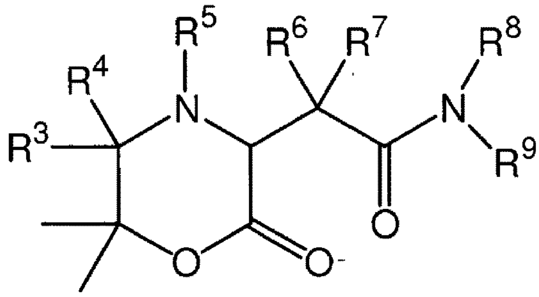

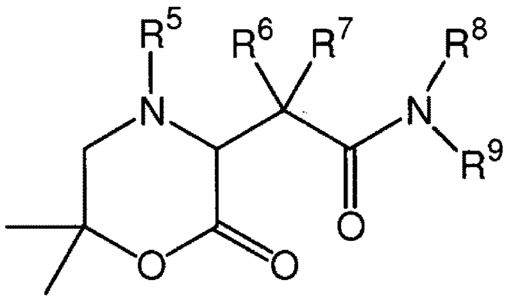

(Fa) (Fb)

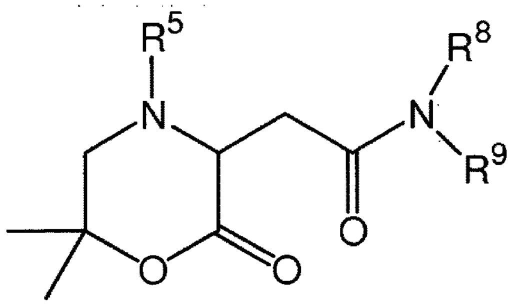

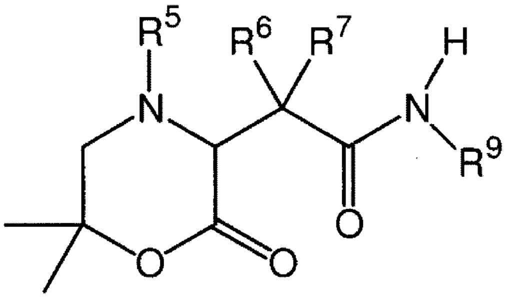

(Fc) (Fd).

7. Verbindung nach den Ansprüchen 1 bis 6, ausgewählt aus der folgenden Liste:

2-((4-Ethyl-6,6-dimethyl-2-oxomorpholin-3-yl)-N-(1-methyl-1H-indol-5-yl)acetamid;
N-(4-Chlorbenzyl)-2-(4-ethyl-6,6-dimethyl-2-oxomorpholin-3-yl)acetamid;
N-(2,3-Dihydro-1H-inden-1-yl)-2-(4-ethyl-6,6-dimethyl-2-oxomorpholin-3-yl)acetamid;
N-(Benzo[d]thiazol-5-yl)-2-(4-ethyl-6,6-dimethyl-2-oxomorpholin-3-yl)acetamid;
2-((4-Ethyl-6,6-dimethyl-2-oxomorpholin-3-yl)-N-(1,2,3,4-tetrahydronaphthalin-1-yl)acetamid;
N-Benzyl-2-(4-ethyl-6,6-dimethyl-2-oxomorpholin-3-yl)acetamid;
2-((4-Ethyl-6,6-dimethyl-2-oxomorpholin-3-yl)-N-(pyridin-3-yl)acetamid;
N-(1-Benzylpiperidin-4-yl)-2-(4-ethyl-6,6-dimethyl-2-oxomorpholin-3-yl)acetamid;
2-((4-Ethyl-6,6-dimethyl-2-oxomorpholin-3-yl)-N-(1-phenylethyl)acetamid;
3-((2-(4-Benzylpiperidin-1-yl)-2-oxoethyl)-4-ethyl-6,6-dimethylmorpholin-2-on;
2-((4-Ethyl-6,6-dimethyl-2-oxomorpholin-3-yl)-N-(pyridin-4-yl)acetamid;
2-((4-Ethyl-6,6-dimethyl-2-oxomorpholin-3-yl)-N-(chinazolin-2-yl)acetamid;
2-((4-Ethyl-6,6-dimethyl-2-oxomorpholin-3-yl)-N-phenethylacetamid;
4-Ethyl-6,6-dimethyl-3-(2-oxo-2-(4-phenylpiperazin-1-yl)ethyl)morpholin-2-on;
2-((4-Ethyl-6,6-dimethyl-2-oxomorpholin-3-yl)-N-(pyridin-2-yl)acetamid;
2-((4-Ethyl-6,6-dimethyl-2-oxomorpholin-3-yl)-N-(thiophen-2-ylmethyl)acetamid;
2-((4-Ethyl-6,6-dimethyl-2-oxomorpholin-3-yl)-N-(furan-2-ylmethyl)acetamid;
2-((4-Ethyl-6,6-dimethyl-2-oxomorpholin-3-yl)-N-methyl-N-phenylacetamid;
2-((4-Ethyl-6,6-dimethyl-2-oxomorpholin-3-yl)-N-(pyrimidin-4-yl)acetamid;
N-(3,5-Dimethylisoxazol-4-yl)-2-(4-ethyl-6,6-dimethyl-2-oxomorpholin-3-yl)acetamid;
2-((4-Ethyl-6,6-dimethyl-2-oxomorpholin-3-yl)-N-(1-methyl-1H-pyrazol-5-yl)acetamid;
2-((4-Ethyl-6,6-dimethyl-2-oxomorpholin-3-yl)-N-(3-phenyl-1,2,4-thiadiazol-5-yl)acetamid;
2-((4-Ethyl-6,6-dimethyl-2-oxomorpholin-3-yl)-N-(1-methyl-1H-pyrazol-3-yl)acetamid;
2-((4-Ethyl-6,6-dimethyl-2-oxomorpholin-3-yl)-N-(5-(trifluormethyl)-1,3,4-thiadiazol-2-yl)acetamid;
4-Ethyl-6,6-dimethyl-3-(2-oxo-2-(1H-pyrazol-1-yl)ethyl)morpholin-2-on;
N-(4,5-Dimethylthiazol-2-yl)-2-(4-ethyl-6,6-dimethyl-2-oxomorpholin-3-yl)acetamid;
N,N-Diethyl-2-(4-ethyl-6,6-dimethyl-2-oxomorpholin-3-yl)acetamid;
2-((4-Ethyl-6,6-dimethyl-2-oxomorpholin-3-yl)-N-isobutylacetamid;
N-Cyclohexyl-2-(4-ethyl-6,6-dimethyl-2-oxomorpholin-3-yl)acetamid;
2-((4-Ethyl-6,6-dimethyl-2-oxomorpholin-3-yl)-N-propylacetamid;
N-tert.-Butyl-2-(4-ethyl-6,6-dimethyl-2-oxomorpholin-3-yl)acetamid;
N-(3,3-Dimethylbutyl)-2-(4-ethyl-6,6-dimethyl-2-oxomorpholin-3-yl)acetamid;

2-((4-Ethyl-6,6-dimethyl-2-oxomorpholin-3-yl)-N-(2,2,2-trifluorethyl)acetamid;
N-Cyclopentyl-2-(4-ethyl-6,6-dimethyl-2-oxomorpholin-3-yl)acetamid;
4-Ethyl-6,6-dimethyl-3-(2-oxo-2-(pyrrolidin-1-yl)ethyl)morpholin-2-on;
N-Ethyl-2-(4-ethyl-6,6-dimethyl-2-oxomorpholin-3-yl)acetamid;
N-sec.-Butyl-2-(4-ethyl-6,6-dimethyl-2-oxomorpholin-3-yl)acetamid;
N-(Cyclohexylmethyl)-2-(4-ethyl-6,6-dimethyl-2-oxomorpholin-3-yl)acetamid;
2-((4-Ethyl-6,6-dimethyl-2-oxomorpholin-3-yl)-N,N-dimethylacetamid;
N-(3-Chlorbenzyl)-2-(4-ethyl-6,6-dimethyl-2-oxomorpholin-3-yl)acetamid;
N-(3,4-Dichlorbenzyl)-2-(4-ethyl-6,6-dimethyl-2-oxomorpholin-3-yl)acetamid;
N-(2,6-Dichlorbenzyl)-2-(4-ethyl-6,6-dimethyl-2-oxomorpholin-3-yl)acetamid;
N-(2-Chlorbenzyl)-2-(4-ethyi-6,6-dimethyl-2-oxomorpholin-3-yl)acetamid;
4-Ethyl-6,6-dimethyl-3-(2-(4-methylpiperazin-1-yl)-2-oxoethyl)morpholin-2-on;
N-Cyclopropyl-2-(4-ethyl-6,6-dimethyl-2-oxomorpholin-3-yl)acetamid;
2-((4-Ethyl-6,6-dimethyl-2-oxomorpholin-3-yl)-N-(4-phenoxyphenyl)acetamid;
N-(4-tert.-Butylthiazol-2-yl)-2-(4-ethyl-6,6-dimethyl-2-oxomorpholin-3-yl)acetamid;
2-((4-Ethyl-6,6-dimethyl-2-oxomorpholin-3-yl)-N-(2-methylbenzyl)acetamid;
2-((4-Ethyl-6,6-dimethyl-2-oxomorpholin-3-yl)-N-(3-methylbenzyl)acetamid;
2-((4-Ethyl-6,6-dimethyl-2-oxomorpholin-3-yl)-N-(4-methylbenzyl)acetamid;
2-((4-Ethyl-6,6-dimethyl-2-oxomorpholin-3-yl)-N-(2-(trifluormethyl)benzyl)acetamid;
2-((4-Ethyl-6,6-dimethyl-2-oxomorpholin-3-yl)-N-(3-(trifluormethyl)benzyl)acetamid;
2-((4-Ethyl-6,6-dimethyl-2-oxomorpholin-3-yl)-N-(4-(trifluormethyl)benzyl)acetamid;
2-((4-Ethyl-6,6-dimethyl-2-oxomorpholin-3-yl)-N-(2-methoxybenzyl)acetamid;
2-((4-Ethyl-6,6-dimethyl-2-oxomorpholin-3-yl)-N-(3-methoxybenzyl)acetamid;
2-((4-Ethyl-6,6-dimethyl-2-oxomorpholin-3-yl)-N-(4-methoxybenzyl)acetamid;
N-(Biphenyl-2-ylmethyl)-2-(4-ethyl-6,6-dimethyl-2-oxomorpholin-3-yl)acetamid;
N-(Biphenyl-3-ylmethyl)-2-(4-ethyl-6,6-dimethyl-2-oxomorpholin-3-yl)acetamid;
N-(Biphenyl-4-ylmethyl)-2-(4-ethyl-6,6-dimethyl-2-oxomorpholin-3-yl)acetamid;
2-((4-Ethyl-2-oxo-6-phenylmorpholin-3-yl)-N-(4-isopropylphenyl)acetamid;
2-(((6S)-4-Ethyl-2-oxo-6-phenylmorpholin-3-yl)-N-(4-isopropylphenyl)acetamid;
2-(((6R)-4-Ethyl-2-oxo-6-phenylmorpholin-3-yl)-N-(4-isopropylphenyl)acetamid;
2-(((6S)-4-Ethyl-6-methyl-2-oxomorpholin-3-yl)-N-(4-isopropylphenyl)acetamid;
2-(((6R)-4-Ethyl-6-methyl-2-oxomorpholin-3-yl)-N-(4-isopropylphenyl)acetamid;
2-((4-Ethyl-6-methyl-2-oxo-6-phenylmorpholin-3-yl)-N-(4-isopropylphenyl)acetamid;
2-((4-Ethyl-6-methyl-2-oxo-6-phenylmorpholin-3-yl)-N-(4-isopropylphenyl)acetamid;
2-((4-Ethyl-5,5,6,6-tetramethyl-2-oxomorpholin-3-yl)-N-(4-isopropylphenyl)acetamid;
2-((4-Ethyl-5,6,6-trimethyl-2-oxomorpholin-3-yl)-N-(4-isopropylphenyl)acetamid;
N-(4-Isopropylphenyl)-2-(4,6,6-triethyl-2-oxomorpholin-3-yl)acetamid;
2-((4-Ethyl-6,6-dimethyl-2-oxomorpholin-3-yl)-N-(4-isopropylphenyl)-2-methylpropanamid;
2-((4-Benzyl-6,6-dimethyl-2-oxomorpholin-3-yl)-N-(4-isopropylphenyl)acetamid;
2-((6,6-Dimethyl-2-oxomorpholin-3-yl)-N-(4-isopropylphenyl)acetamid;
N-(4-Isopropylphenyl)-2-(4,6,6-trimethyl-2-oxomorpholin-3-yl)acetamid;
2-((6,6-Dimethyl-2-oxo-4-propylmorpholin-3-yl)-N-(4-isopropylphenyl)acetamid;
2-((4-Cyclopentyl-6,6-dimethyl-2-oxomorpholin-3-yl)-N-(4-isopropylphenyl)acetamid;
2-((4-Isopropyl-6,6-dimethyl-2-oxomorpholin-3-yl)-N-(4-isopropylphenyl)acetamid;
2-((4-Cyclopropyl-6,6-dimethyl-2-oxomorpholin-3-yl)-N-(4-isopropylphenyl)acetamid;
2-((6,6-Dimethyl-2-oxo-4-(2,2,2-trifluorethyl)morpholin-3-yl)-N-(4-isopropylphenyl)acetamid;
2-((4-(2-Fluorethyl)-6,6-dimethyl-2-oxomorpholin-3-yl)-N-(4-isopropylphenyl)acetamid;
2-((4-Acetyl-6,6-dimethyl-2-oxomorpholin-3-yl)-N-(4-isopropylphenyl)acetamid;
2-((6,6-Dimethyl-4-(methylsulfonyl)-2-oxomorpholin-3-yl)-N-(4-isopropylphenyl)acetamid;
2-((6,6-Dimethyl-2-oxo-4-phenylmorpholin-3-yl)-N-(4-isopropylphenyl)acetamid;
2-((4-Ethyl-6,6-dimethyl-2-oxomorpholin-3-yl)-N-(4-isopropylphenyl)acetamid;
4-Ethyl-6,6-dimethyl-3-(2-morpholino-2-oxoethyl)morpholin-2-on.

**8.** Verbindung nach den Ansprüchen 1 bis 7 zur Verwendung als Medikament.

**9.** Verbindung nach Anspruch 8, wobei das Medikament zur Prävention oder Behandlung einer Pilzinfektion bei einem Empfänger verwendet wird.

**10.** Verbindung nach Anspruch 9, wobei die Pilzinfektion durch Candida-Arten, Aspergillus-Arten oder Fusarium-Arten verursacht wird.

**11.** Verbindung nach Anspruch 10, wobei es sich bei der Pilzinfektion um eine systemische Infektion handelt.

**12.** Verbindung nach Anspruch 10, wobei es sich bei der Pilzinfektion um eine topische Infektion handelt.

**13.** Pharmazeutische Zusammensetzung, umfassend eine Verbindung nach den Ansprüchen 1 bis 7 als Wirkstoff in Abmischung mit mindestens einem pharmazeutisch unbedenklichen Träger.

**14.** Pharmazeutische Zusammensetzung nach Anspruch 13 mit antifungaler Wirkung.

**15.** Verfahren zur Herstellung der Verbindung nach den Ansprüchen 1 bis 7, das folgende Schritte umfasst:

- Herstellen eines substituierten Morpholin-2-onderivats durch Kondensation eines geeigneten Aminoalkoholderivats mit Glyoxal oder mit einem geeigneten Essigsäureesterderivat der Formel LG-$CH_2$-COOR (wobei R vorzugsweise für einen Alkyl- oder Benzylrest steht)
- Umsetzen dieses Morpholin-2-onderivats mit einem geeigneten Essigsäureesterderivat der Formel LG-$CH_2$-COOR (wobei R vorzugsweise für einen Alkyl- oder Benzylrest steht) zum Erhalt eines 2-(subst.-2-Oxomorpholin-3-yl)essigsäureesterderivats
- Umwandeln des erhaltenen Esters in ein Carbonsäurederivat durch eine Verseifungsreaktion
- Umwandeln des Säurederivats in ein gewünschtes Amidderivat unter Verwendung von standardmäßigen Peptidkupplungsreagentien
- Herstellen eines 2-(4-benzyl-2-oxomorpholin-3-yl)-N-substituierten Acetamidderivats unter Verwendung des obenaufgeführten Schritts
- Abspalten der N-Benzyl-Schutzgruppe von dem Morpholin-2-onderivat zum Erhalt eines 2-(2-oxomorpholin-3-yl)-N-substituierten Acetamidderivats
- Kondensieren des 2-(2-oxomorpholin-3-yl)-N-substituierten Acetamidderivats mit einem geeigneten Reagens zur Substitution des freien Stickstoffatoms des Morpholin-2-on-Rings und zum Erhalt der gewünschten Verbindungen.

**Revendications**

**1.** Composé possédant une structure selon la formule (A) :

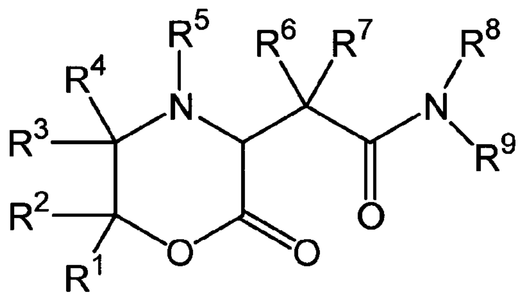

(A)

dans laquelle

- chaque $R^1$ et $R^2$ est choisi indépendamment parmi hydrogène ; alkyle ; alcényle ; alcynyle ; hétéroalkyle ; hétéroalcényle ; hétéroalcynyle ; aryle ; arylalkyle ; arylalcényle ; arylalcynyle ; arylhétéroalkyle ; arylhétéroalcényle ; et arylhétéroalcynyle ;
à condition qu'au moins un parmi $R^1$ et $R^2$ ne soit pas hydrogène ;
- chaque $R^3$ et $R^4$ est choisi indépendamment parmi hydrogène ; alkyle ; alcényle ; alcynyle ; hétéroalkyle ; hétéroalcényle ; et hétéroalcynyle ;
- $R^5$ est choisi indépendamment parmi hydrogène ; alkyle ; alcényle ; alcynyle ; hétéroalkyle ; hétéroalcényle ;

hétéroalcynyle ; aryle ; arylalkyle ; arylalcényle ; arylalcynyle ; $-C(O)R^{10}$ ; et $-S(O)_2R^{10}$ ;
où chacun parmi lesdits alkyle, alcényle, alcynyle, hétéroalkyle, hétéroalcényle, hétéroalcynyle, aryle, arylalkyle, arylalcényle, et arylalcynyle peut être non substitué ou substitué par un ou plusieurs substituants choisis chacun indépendamment parmi alkyle, alcényle, alcynyle, hétéroalkyle, hétéroalcényle, hétéroalcynyle, hydroxyle, =O, halogène, -SH, =S, trifluorométhyle, $-OCF_3$, cyano, nitro,-C(O)OH, ou $-NH_2$ ;
- chaque $R^6$ et $R^7$ est choisi indépendamment parmi hydrogène ; alkyle ; alcényle ; alcynyle ; hétéroalkyle ; hétéroalcényle ; et hétéroalcynyle ;
- chaque $R^8$ et $R^9$ est choisi indépendamment parmi hydrogène ; alkyle ; alcényle ; alcynyle ; hétéroalkyle ; hétéroalcényle ; hétéroalcynyle ; aryle ; arylalkyle ; arylalcényle ; arylalcynyle ; arylhétéroalkyle ; arylhétéroalcényle ; arylhétéroalcynyle ; hétérocycle ; hétérocycle-alkyle ; hétérocycle-alcényle ; hétérocycle-alcynyle ; hétérocycle-hétéroalkyle ; hétérocycle-hétéroalcényle ; et hétérocycle-hétéroalcynyle ; et où $R^8$ et $R^9$ peuvent être pris ensemble afin de former un hétérocycle (de 5, 6 ou 7 chaînons) ;
à condition qu'au moins un parmi $R^8$ et $R^9$ ne soit pas hydrogène ; et
où chacun parmi lesdits alkyle, alcényle, alcynyle, hétéroalkyle, hétéroalcényle, hétéroalcynyle, aryle, arylalkyle, arylalcényle, arylalcynyle, arylhétéroalkyle, arylhétéroalcényle, arylhétéroalcynyle, hétérocycle, hétérocycle-alkyle, hétérocycle-alcényle, hétérocycle-alcynyle, hétérocycle-hétéroalkyle, hétérocycle-hétéroalcényle, et hétérocycle-hétéroalcynyle, peut être non substitué ou substitué par un ou plusieurs $R^{11}$ ;
- chaque $R^{10}$ est choisi indépendamment parmi hydrogène ; alkyle ; alcényle ; alcynyle ; hétéroalkyle ; hétéroalcényle ; hétéroalcynyle ; aryle ; arylalkyle ; arylalcényle ; et arylalcynyle ;
où chacun parmi lesdits alkyle, alcényle, alcynyle, hétéroalkyle, hétéroalcényle, hétéroalcynyle, aryle, arylalkyle, arylalcényle, et arylalcynyle peut être non substitué ou substitué par un ou plusieurs substituants choisis chacun indépendamment parmi alkyle, alcényle, alcynyle, hétéroalkyle, hétéroalcényle, hétéroalcynyle, hydroxyle, =O, halogène, -SH, =S, trifluorométhyle, $-OCF_3$, cyano, nitro,-C(O)OH, ou $-NH_2$ ;
- chaque $R^{11}$ est choisi indépendamment dans le groupe constitué par halogène ; hydroxyle ; sulfhydryle ; $-OZ^2$ ; =O ; $-SZ^2$ ; =S ; $-S(O)Z^2$ ; $-S(O)_2Z^3$ ; $-S(O)_2NZ^4Z^5$ ; trifluorométhyle ; nitro ; $-NZ^4Z^5$ ; $-NZ^4S(O)_2Z^2$ ; $-NZ^4C(O)Z^2$ ; $-NZ^4C(O)NZ^4Z^5$ ; cyano ; $-C(O)Z^3$ ; $-C(O)NZ^4Z^5$ ; -C(O)H ; alkyle ; alcényle ; alcynyle ; hétéroalkyle ; hétéroalcényle ; hétéroalcynyle ; aryle ; hétérocycle ; arylalkyle ; arylalcényle ; arylalcynyle ; arylhétéroalkyle ; arylhétéroalcényle ; arylhétéroalcynyle ; hétérocycle-alkyle ; hétérocycle-alcényle ; hétérocycle-alcynyle ; hétérocycle-hétéroalkyle, hétérocycle-hétéroalcényle ; ou hétérocycle-hétéroalcynyle ;
et où lesdits alkyle, alkyle cyclique, alcényle, alcényle cyclique, alcynyle, alcynyle cyclique, hétéroalkyle, hétéroalcényle, hétéroalcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle, ou hétérocycle-hétéroalcynyle peuvent être non substitués ou substitués par un ou plusieurs substituants choisis dans le groupe constitué par alkyle, alcényle, alcynyle, hétéroalkyle, hétéroalcényle, hétéroalcynyle, hydroxyle, =O, halogène, -SH, =S, trifluorométhyle, $-OCF_3$, cyano, nitro, -C(O)OH, ou $-NH_2$ ;
- chaque $Z^2$ est choisi indépendamment parmi alkyle ; alcényle ; alcynyle ; hétéroalkyle ; hétéroalcényle ; hétéroalcynyle ; aryle ; hétérocycle ; arylalkyle ; arylalcényle ; arylalcynyle ; arylhétéroalkyle ; arylhétéroalcényle ; arylhétéroalcynyle ; hétérocycle-alkyle ; hétérocycle-alcényle ; hétérocycle-alcynyle ; hétérocycle-hétéroalkyle ; hétérocycle-hétéroalcényle ; ou hétérocycle-hétéroalcynyle ;
où lesdits alkyle, alcényle, alcynyle, hétéroalkyle, hétéroalcényle, hétéroalcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle, ou hétérocycle-hétéroalcynyle peuvent être non substitués ou substitués par alkyle, alcényle, alcynyle, hétéroalkyle, hétéroalcényle, hétéroalcynyle, hydroxyle, =O, halogène, -SH, =S, trifluorométhyle, $-OCF_3$, cyano, nitro, -C(O)OH, ou $-NH_2$ ;
- chaque $Z^3$ est choisi indépendamment parmi hydroxyle ; alkyle ; alcényle ; alcynyle ; hétéroalkyle ; hétéroalcényle ; hétéroalcynyle ; aryle ; hétérocycle ; arylalkyle ; arylalcényle ; arylalcynyle ; arylhétéroalkyle ; arylhétéroalcényle ; arylhétéroalcynyle ; hétérocycle-alkyle ; hétérocycle-alcényle ; hétérocycle-alcynyle ; hétérocycle-hétéroalkyle ; hétérocycle-hétéroalcényle ; ou hétérocycle-hétéroalcynyle ;
où lesdits alkyle, alcényle, alcynyle, hétéroalkyle, hétéroalcényle, hétéroalcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle, ou hétérocycle-hétéroalcynyle peuvent être non substitués ou substitués par un ou plusieurs substituants choisis dans le groupe constitué par alkyle, alcényle, alcynyle, hétéroalkyle, hétéroalcényle, hétéroalcynyle, hydroxyle, =O, halogène, -SH, =S, trifluorométhyle, $-OCF_3$, cyano, nitro, -C(O)OH, ou $-NH_2$ ;
- chaque $Z^4$ et $Z^5$ est choisi indépendamment parmi hydrogène ; alkyle ; alcényle ; alcynyle ; hétéroalkyle ; hétéroalcényle ; hétéroalcynyle ; aryle ; hétérocycle ; arylalkyle ; arylalcényle ; arylalcynyle ; arylhétéroalkyle ;

arylhétéroalcényle ; arylhétéroalcynyle ; hétérocycle-alkyle ; hétérocycle-alcényle ; hétérocycle-alcynyle ; hétérocycle-hétéroalkyle ; hétérocycle-hétéroalcényle ; ou hétérocycle-hétéroalcynyle ;
où lesdits alkyle, alcényle, alcynyle, hétéroalkyle, hétéroalcényle, hétéroalcynyle, aryle, hétérocycle, arylalkyle, arylalcényle, arylalcynyle, arylhétéroalkyle, arylhétéroalcényle, arylhétéroalcynyle, hétérocycle-alkyle, hétérocycle-alcényle, hétérocycle-alcynyle, hétérocycle-hétéroalkyle, hétérocycle-hétéroalcényle, ou hétérocycle-hétéroalcynyle peuvent être non substitués ou substitués par un ou plusieurs substituants choisis dans le groupe constitué par alkyle, alcényle, alcynyle, hétéroalkyle, hétéroalcényle, hétéroalcynyle, hydroxyle, =O, halogène, -SH, =S, trifluorométhyle, $-OCF_3$, cyano, nitro, -C(O)OH, ou $-NH_2$ ;
et où $Z^4$ et $Z^5$ peuvent être pris ensemble afin de former un hétérocycle (de 5, 6 ou 7 chaînons) qui peut être non substitué ou substitué par alkyle, alcényle, alcynyle, hétéroalkyle, hétéroalcényle, hétéroalcynyle, hydroxyle, =O, halogène, -SH, =S, trifluorométhyle, $-OCF_3$, cyano, nitro,-C(O)OH, ou $-NH_2$ ;
et les stéréoisomères, les tautomères, les solvats ou les sels pharmaceutiquement acceptables de ceux-ci.

**2.** Composé selon la revendication 1, dans lequel $R^5$ est alkyle.

**3.** Composé selon les revendications 1 et 2, dans lequel $R^8$ est hydrogène.

**4.** Composé selon les revendications 1 à 3, dans lequel $R^3$ et $R^4$ sont hydrogène.

**5.** Composé selon les revendications 1 à 4, dans lequel $R^1$ et $R^2$ sont chacun alkyle.

**6.** Composé selon les revendications 1 à 5, dans lequel le composé possède une structure selon les formules (Fa) à (Fd) :

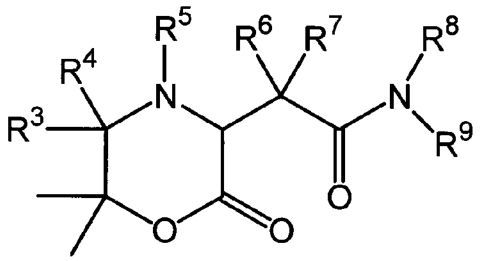

(Fa)

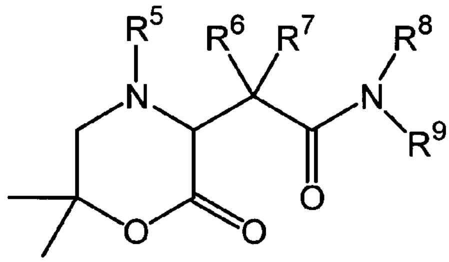

(Fb)

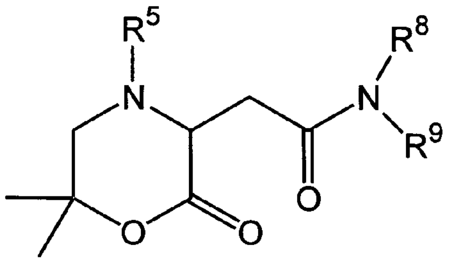

(Fc)

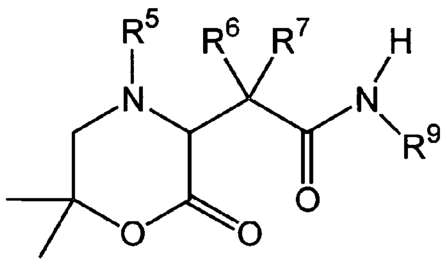

(Fd).

**7.** Composé selon les revendications 1 à 6, choisi dans le groupe constitué par :

le 2-(4-éthyl-6,6-diméthyl-2-oxomorpholin-3-yl)-N-(1-méthyl-1H-indol-5-yl)acétamide ;
le N-(4-chlorobenzyl)-2-(4-éthyl-6,6-diméthyl-2-oxomorpholin-3-yl)acétamide ;
le N-(2,3-dihydro-1H-indén-1-yl)-2-(4-éthyl-6,6-diméthyl-2-oxomorpholin-3-yl)acétamide ;
le N-(benzo[d]thiazol-5-yl)-2-(4-éthyl-6,6-diméthyl-2-oxomorpholin-3-yl)acétamide ;
le 2-(4-éthyl-6,6-diméthyl-2-oxomorpholin-3-yl)-N-(1,2,3,4-tétrahydronaphtalén-1-yl)acétamide ;
le N-benzyl-2-(4-éthyl-6,6-diméthyl-2-oxomorpholin-3-yl)acétamide ;
le 2-(4-éthyl-6,6-diméthyl-2-oxomorpholin-3-yl)-N-(pyridin-3-yl)acétamide ;
le N-(1-benzylpipéridin-4-yl)-2-(4-éthyl-6,6-diméthyl-2-oxomorpholin-3-yl)acétamide ;

le 2-(4-éthyl-6,6-diméthyl-2-oxomorpholin-3-yl)-N-(1-phényléthyl)acétamide ;
la 3-(2-(4-benzylpipéridin-1-yl)-2-oxoéthyl)-4-éthyl-6,6-diméthylmorpholin-2-one ;
le 2-(4-éthyl-6,6-diméthyl-2-oxomorpholin-3-yl)-N-(pyridin-4-yl)acétamide ;
le 2-(4-éthyl-6,6-diméthyl-2-oxomorpholin-3-yl)-N-(quinazolin-2-yl)acétamide ;
le 2-(4-éthyl-6,6-diméthyl-2-oxomorpholin-3-yl)-N-phénéthylacétamide ;
la 4-éthyl-6,6-diméthyl-3-(2-oxo-2-(4-phénylpipérazin-1-yl)éthyl)morpholin-2-one ;
le 2-(4-éthyl-6,6-diméthyl-2-oxomorpholin-3-yl)-N-(pyridin-2-yl)acétamide ;
le 2-(4-éthyl-6,6-diméthyl-2-oxomorpholin-3-yl)-N-(thiophén-2-ylméthyl)acétamide ;
le 2-(4-éthyl-6,6-diméthyl-2-oxomorpholin-3-yl)-N-(furan-2-ylméthyl)acétamide ;
le 2-(4-éthyl-6,6-diméthyl-2-oxomorpholin-3-yl)-N-méthyl-N-phénylacétamide ;
le 2-(4-éthyl-6,6-diméthyl-2-oxomorpholin-3-yl)-N-(pyrimidin-4-yl)acétamide ;
le N-(3,5-diméthylisoxazol-4-yl)-2-(4-éthyl-6,6-diméthyl-2-oxomorpholin-3-yl)acétamide ;
le 2-(4-éthyl-6,6-diméthyl-2-oxomorpholin-3-yl)-N-(1-méthyl-1H-pyrazol-5-yl)acétamide ;
le 2-(4-éthyl-6,6-diméthyl-2-oxomorpholin-3-yl)-N-(3-phényl-1,2,4-thiadiazol-5-yl)acétamide ;
le 2-(4-éthyl-6,6-diméthyl-2-oxomorpholin-3-yl)-N-(1-méthyl-1H-pyrazol-3-yl)acétamide ;
le 2-(4-éthyl-6,6-diméthyl-2-oxomorpholin-3-yl)-N-(5-(trifluorométhyl)-1,3,4-thiadiazol-2-yl)acétamide ;
la 4-éthyl-6,6-diméthyl-3-(2-oxo-2-(1H-pyrazol-1-yl)éthyl)morpholin-2-one ;
le N-(4,5-diméthylthiazol-2-yl)-2-(4-éthyl-6,6-diméthyl-2-oxomorpholin-3-yl)acétamide ;
le N,N-diéthyl-2-(4-éthyl-6,6-diméthyl-2-oxomorpholin-3-yl)acétamide ;
le 2-(4-éthyl-6,6-diméthyl-2-oxomorpholin-3-yl)-N-isobutylacétamide ;
le N-cyclohexyl-2-(4-éthyl-6,6-diméthyl-2-oxomorpholin-3-yl)acétamide ;
le 2-(4-éthyl-6,6-diméthyl-2-oxomorpholin-3-yl)-N-propylacétamide ;
le N-tertio-butyl-2-(4-éthyl-6,6-diméthyl-2-oxomorpholin-3-yl)acétamide ;
le N-(3,3-diméthylbutyl)-2-(4-éthyl-6,6-diméthyl-2-oxomorpholin-3-yl)acétamide ;
le 2-(4-éthyl-6,6-diméthyl-2-oxomorpholin-3-yl)-N-(2,2,2-trifluoroéthyl)acétamide ;
le N-cyclopentyl-2-(4-éthyl-6,6-diméthyl-2-oxomorpholin-3-yl)acétamide ;
la 4-éthyl-6,6-diméthyl-3-(2-oxo-2-(pyrrolidin-1-yl)éthyl)morpholin-2-one ;
le N-éthyl-2-(4-éthyl-6,6-diméthyl-2-oxomorpholin-3-yl)acétamide ;
le N-sec-butyl-2-(4-éthyl-6,6-diméthyl-2-oxomorpholin-3-yl)acétamide ;
le N-(cyclohexylméthyl)-2-(4-éthyl-6,6-diméthyl-2-oxomorpholin-3-yl)acétamide ;
le 2-(4-éthyl-6,6-diméthyl-2-oxomorpholin-3-yl)-N,N-diméthylacétamide ;
le N-(3-chlorobenzyl)-2-(4-éthyl-6,6-diméthyl-2-oxomorpholin-3-yl)acétamide ;
le N-(3,4-dichlorobenzyl)-2-(4-éthyl-6,6-diméthyl-2-oxomorpholin-3-yl)acétamide ;
le N-(2,6-dichlorobenzyl)-2-(4-éthyl-6,6-diméthyl-2-oxomorpholin-3-yl)acétamide ;
le N-(2-chlorobenzyl)-2-(4-éthyl-6,6-diméthyl-2-oxomorpholin-3-yl)acétamide ;
la 4-éthyl-6,6-diméthyl-3-(2-(4-méthylpipérazin-1-yl)-2-oxoéthyl)morpholin-2-one ;
le N-cyclopropyl-2-(4-éthyl-6,6-diméthyl-2-oxomorpholin-3-yl)acétamide ;
le 2-(4-éthyl-6,6-diméthyl-2-oxomorpholin-3-yl)-N-(4-phénoxyphényl)acétamide ;
le N-(4-tertio-butylthiazol-2-yl)-2-(4-éthyl-6,6-diméthyl-2-oxomorpholin-3-yl)acétamide ;
le 2-(4-éthyl-6,6-diméthyl-2-oxomorpholin-3-yl)-N-(2-méthylbenzyl)acétamide ;
le 2-(4-éthyl-6,6-diméthyl-2-oxomorpholin-3-yl)-N-(3-méthylbenzyl)acétamide ;
le 2-(4-éthyl-6,6-diméthyl-2-oxomorpholin-3-yl)-N-(4-méthylbenzyl)acétamide ;
le 2-(4-éthyl-6,6-diméthyl-2-oxomorpholin-3-yl)-N-(2-(trifluorométhyl)benzyl)acétamide ;
le 2-(4-éthyl-6,6-diméthyl-2-oxomorpholin-3-yl)-N-(3-(trifluorométhyl)benzyl)acétamide ;
le 2-(4-éthyl-6,6-diméthyl-2-oxomorpholin-3-yl)-N-(4-(trifluorométhyl)benzyl)acétamide ;
le 2-(4-éthyl-6,6-diméthyl-2-oxomorpholin-3-yl)-N-(2-méthoxybenzyl)acétamide ;
le 2-(4-éthyl-6,6-diméthyl-2-oxomorpholin-3-yl)-N-(3-méthoxybenzyl)acétamide ;
le 2-(4-éthyl-6,6-diméthyl-2-oxomorpholin-3-yl)-N-(4-méthoxybenzyl)acétamide ;
le N-(biphényl-2-ylméthyl)-2-(4-éthyl-6,6-diméthyl-2-oxomorpholin-3-yl)acétamide ;
le N-(biphényl-3-ylméthyl)-2-(4-éthyl-6,6-diméthyl-2-oxomorpholin-3-yl)acétamide ;
le N-(biphényl-4-ylméthyl)-2-(4-éthyl-6,6-diméthyl-2-oxomorpholin-3-yl)acétamide ;
le 2-(4-éthyl-2-oxo-6-phénylmorpholin-3-yl)-N-(4-isopropylphényl)acétamide ;
le 2-((6S)-4-éthyl-2-oxo-6-phénylmorpholin-3-yl)-N-(4-isopropylphényl)acétamide ;
le 2-((6R)-4-éthyl-2-oxo-6-phénylmorpholin-3-yl)-N-(4-isopropylphényl)acétamide ;
le 2-((6S)-4-éthyl-6-méthyl-2-oxomorpholin-3-yl)-N-(4-isopropylphényl)acétamide ;
le 2-((6R)-4-éthyl-6-méthyl-2-oxomorpholin-3-yl)-N-(4-isopropylphényl)acétamide ;
le 2-(4-éthyl-6-méthyl-2-oxo-6-phénylmorpholin-3-yl)-N-(4-isopropylphényl)acétamide ;
le 2-(4-éthyl-6-méthyl-2-oxo-6-phénylmorpholin-3-yl)-N-(4-isopropylphényl)acétamide ;

le 2-(4-éthyl-5,5,6,6-tétraméthyl-2-oxomorpholin-3-yl)-N-(4-isopropylphényl)acétamide ;
le 2-(4-éthyl-5,6,6-triméthyl-2-oxomorpholin-3-yl)-N-(4-isopropylphényl)acétamide ;
le N-(4-isopropylphényl)-2-(4,6,6-triéthyl-2-oxomorpholin-3-yl)acétamide ;
le 2-(4-éthyl-6,6-diméthyl-2-oxomorpholin-3-yl)-N-(4-isopropylphényl)-2-méthylpropanamide ;
le 2-(4-benzyl-6,6-diméthyl-2-oxomorpholin-3-yl)-N-(4-isopropylphényl)acétamide ;
le 2-(6,6-diméthyl-2-oxomorpholin-3-yl)-N-(4-isopropylphényl)acétamide ;
le N-(4-isopropylphényl)-2-(4,6,6-triméthyl-2-oxomorpholin-3-yl)acétamide ;
le 2-(6,6-diméthyl-2-oxo-4-propylmorpholin-3-yl)-N-(4-isopropylphényl)acétamide ;
le 2-(4-cyclopentyl-6,6-diméthyl-2-oxomorpholin-3-yl)-N-(4-isopropylphényl)acétamide ;
le 2-(4-isopropyl-6,6-diméthyl-2-oxomorpholin-3-yl)-N-(4-isopropylphényl)acétamide ;
le 2-(4-cyclopropyl-6,6-diméthyl-2-oxomorpholin-3-yl)-N-(4-isopropylphényl)acétamide ;
le 2-(6,6-diméthyl-2-oxo-4-(2,2,2-trifluoroéthyl)morpholin-3-yl)-N-(4-isopropylphényl)acétamide ;
le 2-(4-(2-fluoroéthyl)-6,6-diméthyl-2-oxomorpholin-3-yl)-N-(4-isopropylphényl)acétamide ;
le 2-(4-acétyl-6,6-diméthyl-2-oxomorpholin-3-yl)-N-(4-isopropylphényl)acétamide ;
le 2-(6,6-diméthyl-4-(méthylsulfonyl)-2-oxomorpholin-3-yl)-N-(4-isopropylphényl)acétamide ;
le 2-(6,6-diméthyl-2-oxo-4-phénylmorpholin-3-yl)-N-(4-isopropylphényl)acétamide ;
le 2-(4-éthyl-6,6-diméthyl-2-oxomorpholin-3-yl)-N-(4-isopropylphényl)acétamide ;
la 4-éthyl-6,6-diméthyl-3-(2-morpholino-2-oxoéthyl)morpholin-2-one.

**8.** Composé selon les revendications 1 à 7, pour une utilisation comme médicament.

**9.** Composé selon la revendication 8, **caractérisée en ce que** le médicament est destiné à la prévention ou au traitement d'une infection fongique chez un sujet.

**10.** Composé selon la revendication 9, **caractérisée en ce que** ladite infection fongique est provoquée par une espèce de *Candida,* une espèce *d'Aspergillus* ou une espèce de *Fusarium.*

**11.** Composé selon la revendication 10, **caractérisée en ce que** ladite infection fongique est une infection systémique.

**12.** Composé selon la revendication 10, **caractérisée en ce que** ladite infection fongique est une infection topique.

**13.** Composition pharmaceutique comprenant un composé selon les revendications 1 à 7, comme ingrédient actif en mélange avec au moins un véhicule pharmaceutiquement acceptable.

**14.** Composition pharmaceutique selon la revendication 13, possédant une activité antifongique.

**15.** Méthode de préparation du composé selon les revendications 1 à 7, comprenant les étapes :

- de préparation d'un dérivé de morpholin-2-one substituée par condensation d'un dérivé d'aminoalcool convenable avec du glyoxal ou avec un dérivé d'acétate convenable de formule LG-$CH_2$-COOR (R étant préférablement un radical alkyle ou benzyle) ;
- de réaction de ce dérivé de morpholin-2-one avec un dérivé d'acétate convenable de formule LG-$CH_2$-COOR (R étant préférablement un radical alkyle ou benzyle) afin d'obtenir un dérivé de 2-(substitué-2-oxomorpholin-3-yl)acétate ;
- de conversion de l'ester obtenu en dérivé d'acide carboxylique via une réaction de saponification ;
- de conversion du dérivé d'acide en un dérivé d'amide désiré en utilisant des réactifs standard de couplage de peptides ;
- de préparation d'un dérivé d'acétamide 2-(4-benzyl-2-oxomorpholin-3-yl)-N-substitué en utilisant l'étape détaillée précédente ;
- d'élimination du groupement protecteur de N-benzyle du dérivé de morpholin-2-one afin d'obtenir un dérivé d'acétamide 2-(2-oxomorpholin-3-yl)-N-substitué ;
- de condensation du dérivé d'acétamide 2-(2-oxomorpholin-3-yl)-N-substitué avec un réactif convenable afin de substituer l'atome d'azote libre du cycle de morpholin-2-one et d'obtenir les composés désirés.

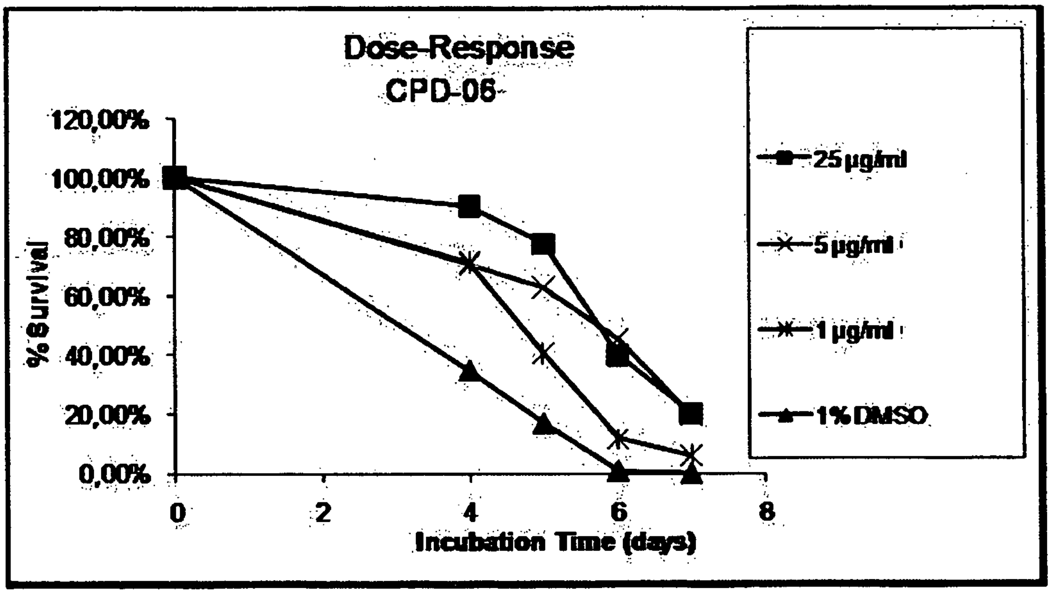


Figure 1

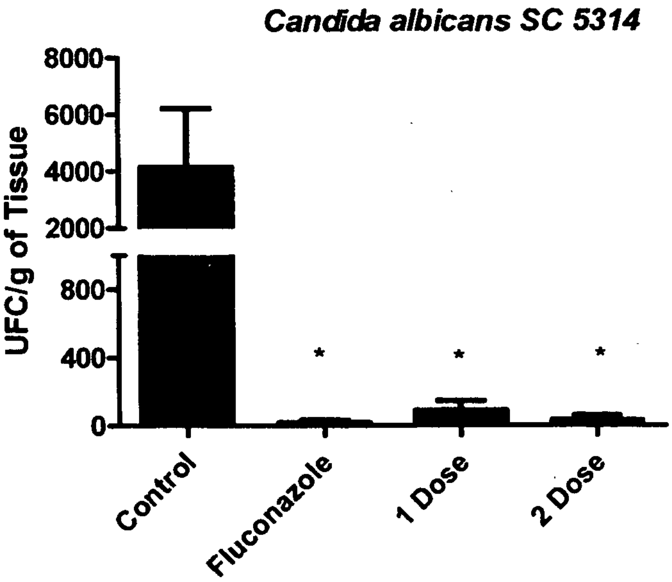


Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4879291 A
- US 4044130 A
- WO 9615111 A, Hoye, T.


### Non-patent literature cited in the description


- **THEODORA W. GREENE.** Protective Groups in Organic Chemistry. John Wiley & Sons, Inc, 1991
- **PAQUETTE, LEO A.** Principles of Modern Heterocyclic Chemistry. 1968
- The Chemistry of Heterocyclic Compounds, A series of Monographs. John Wiley & Sons, 1950, vol. 13, 14, 16, 19, 28
- **KATRITZKY, ALAN R. ; REES, C.W. ; SCRIVEN, E.** Comprehensive Heterocyclic Chemistry. Pergamon Press, 1996
- *J. Am. Chem. Soc.,* 1960, vol. 82, 5566
- **CHOU et al.** *Adv. Enzyme Reg.,* 1984, vol. 22, 27
- **ELION et al.** *J. Biol. Chem.,* 1954, vol. 208, 477-488
- **BABA et al.** *Antimicrob. Agents Chemother.,* 1984, vol. 25, 515-517
- **E. L. ELIEL.** Stereochemistry of Carbon Compounds. McGraw Hill, 1962
- **LOCHMULLER, C. H.** *J. Chromatogr.,* 1975, vol. 113 (3), 283-302
- **ELIEL, E. ; WILEN, S.** Stereochemistry of Organic Compounds. John Wiley & Sons, Inc, 1994, 322
- **JACOB III.** *J. Org. Chem.,* 1982, vol. 47, 4165
- Chiral Liquid Chromatography. Chapman and Hall, 1989
- **OKAMOTO.** Optical resolution of dihydropyridine enantiomers by High-performance liquid chromatography using phenylcarbamates of polysaccharides as a chiral stationary phase. *J. of Chromatogr.,* 1990, vol. 513, 375-378
- Handbook of Pharmaceutical Excipients. 1986
- McCutcheon's Detergents and Emulsifiers Annual. MC Publishing Crop, 1981
- Tensid-Taschenbucw. Hanser Verlag, 1981
- Encyclopaedia of Surfactants. Chemical Publishing Co, 1981
- **BREGER, J. et al.** *PLoS Pathog.,* 2007, vol. 3, e18
- **TAVARES PM et al.** *Antimicrob Agents Chemother.,* December 2008, vol. 52 (12), 4522-5
- **CLEMONS KV ; DA STEVENS.** *J Antimicrob. Chemother.,* 2001, vol. 47, 183-186
- **OKAWA Y et al.** *Biol Pharm Bull,* 2007, vol. 30, 1870-1873